(19) Europäisches Patentamt
European Patent Office
Office européen des brevets

(11) **EP 3 710 557 B1**

(12) **EUROPÄISCHE PATENTSCHRIFT**

(45) Veröffentlichungstag und Bekanntmachung des Hinweises auf die Patenterteilung:
**18.05.2022 Patentblatt 2022/20**

(21) Anmeldenummer: **18799541.0**

(22) Anmeldetag: **12.11.2018**

(51) Internationale Patentklassifikation (IPC):
***C09K 11/06*** *(2006.01)* ***C07D 251/24*** *(2006.01)*
***C07D 209/86*** *(2006.01)* ***H01L 51/00*** *(2006.01)*

(52) Gemeinsame Patentklassifikation (CPC):
**C09K 11/06; C07D 251/24; C07D 333/76; C07D 405/14; C07D 409/14; C07D 487/04; C07D 498/04; C07D 513/04; H01L 51/0054; H01L 51/0067; H01L 51/0072; H01L 51/0073; H01L 51/0074;** H01L 51/006; H01L 51/0085;
(Forts.)

(86) Internationale Anmeldenummer:
**PCT/EP2018/080867**

(87) Internationale Veröffentlichungsnummer:
**WO 2019/096717 (23.05.2019 Gazette 2019/21)**

(54) **ZUSAMMENSETZUNG FÜR ORGANISCHE ELEKTRONISCHE VORRICHTUNGEN**
COMPOSITION FOR ORGANIC ELECTRONIC DEVICES
COMPOSITION POUR DISPOSITIFS ÉLECTRONIQUES ORGANIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.11.2017 EP 17201480**

(43) Veröffentlichungstag der Anmeldung:
**23.09.2020 Patentblatt 2020/39**

(73) Patentinhaber: **Merck Patent GmbH**
**64293 Darmstadt (DE)**

(72) Erfinder:
- **EICKHOFF, Christian**
**68259 Mannheim (DE)**
- **PARHAM, Amir**
**60486 Frankfurt Am Main (DE)**
- **KROEBER, Jonas**
**60311 Frankfurt Am Main (DE)**
- **GROSSMANN, Tobias**
**75387 NEUBUBLACH (DE)**
- **JATSCH, Anja**
**60489 Frankfurt Am Main (DE)**
- **EHRENREICH, Christian**
**64285 Darmstadt (DE)**

(56) Entgegenhaltungen:
**KR-B1- 101 744 248** **US-A1- 2015 340 618**
**US-A1- 2016 248 023**

EP 3 710 557 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

(52) Gemeinsame Patentklassifikation (CPC): (Forts.)
H01L 51/5016; H01L 2251/5384; Y02E 10/549;
Y02P 70/50

## Beschreibung

Die vorliegende Erfindung betrifft eine Zusammensetzung, die einen speziellen bipolaren Host und ein Triphenylen-Derivat umfasst, deren Verwendung in elektronischen Vorrichtungen sowie elektronische Vorrichtungen enthaltend diese Zusammensetzung.

Der Aufbau organischer Elektrolumineszenzvorrichtungen (z.B. OLEDs - organic light emitting diodes oder OLECs - organic light emitting electrochemical cells), in denen organische Halbleiter als funktionelle Materialien eingesetzt werden, ist beispielsweise in US 4539507, US 5151629, EP 0676461 und WO 98/27136 beschrieben. Als emittierende Materialien werden hierbei neben fluoreszierenden Emittern zunehmend metallorganische Komplexe eingesetzt, die Phosphoreszenz statt Fluoreszenz zeigen (M. A. Baldo et al., Appl. Phys. Lett. 1999, 75, 4-6). Aus quantenmechanischen Gründen ist unter Verwendung metallorganischer Verbindungen als Phosphoreszenzemitter eine bis zu vierfach gesteigerte Energie- und Leistungseffizienz möglich. Generell gibt es bei OLEDs, insbesondere auch bei OLEDs die Triplettemission (Phosphoreszenz) zeigen, jedoch immer noch Verbesserungsbedarf, beispielsweise im Hinblick auf Effizienz, Betriebsspannung und Lebensdauer.

Die Eigenschaften organischer elektrolumineszierender Vorrichtungen werden nicht nur durch die eingesetzten Emitter bestimmt. Hier sind insbesondere auch die anderen verwendeten Materialien, wie Host- und Matrixmaterialien, Lochblockiermaterialien, Elektronentransportmaterialien, Lochtransportmaterialien und Elektronen- bzw. Exzitonenblockiermaterialien von besonderer Bedeutung, und davon insbesondere die Host bzw. Matrixmaterialien. Verbesserungen dieser Materialien können zu deutlichen Verbesserungen elektrolumineszierender Vorrichtungen führen.

Hostmaterialien zur Verwendung in organischen elektronischen Vorrichtungen sind dem Fachmann gut bekannt. Im Stand der Technik wird häufig auch der Begriff Matrixmaterial verwendet, wenn ein Hostmaterial für phosphoreszierende Emitter gemeint ist. Diese Verwendung des Begriffs gilt auch für die vorliegende Erfindung. Mittlerweile wurde eine Vielzahl von Hostmaterialien sowohl für fluoreszierende als auch für phosphoreszierende elektronische Vorrichtungen entwickelt.

Gemäß dem Stand der Technik werden unter anderem Ketone (z. B. gemäß WO 2004/093207 oder WO 2010/006680) oder Phosphinoxide (z. B. gemäß WO 2005/003253) als Matrixmaterialien für phosphoreszierende Emitter verwendet. Weitere Matrixmaterialien gemäß dem Stand der Technik repräsentieren Triazine (bspw. WO 2008/056746, EP 0906947, EP 0908787, EP 0906948) sowie Lactame (bspw. WO 2011/116865 oder WO 2011/137951). Weiterhin werden gemäß dem Stand der Technik unter anderem Carbazolderivate (z. B. gemäß WO 2005/039246, US 2005/0069729 oder WO 2014/015931), Indolocarbazolderivate (z. B. gemäß WO 2007/063754 oder WO 2008/056746) oder Indenocarbazolderivate (z. B. gemäß WO 2010/136109 oder WO 2011/000455), insbesondere solche, die mit elektronenarmen Heteroaromaten wie Triazin substituiert sind, als Matrixmaterialien für phosphoreszierende Emitter verwendet. Aus der WO 2011/057706 sind Carbazolderivate bekannt, welche mit zwei Triphenyltriazingruppen substituiert sind. Aus der WO 2011/046182 sind Carbazol-Arylen-TriazinDerivate bekannt, welche am Triazin mit einer Fluorenylgruppe substituiert sind. WO 2009/069442 offenbart Tricyclen, wie Carbazol, Dibenzofuran oder Dibenzothiophen, die hochgradig mit elektronenarmen Heteroaromaten (z.B. Pyridin, Pyrimidin oder Triazin) substituiert sind, als Hostmaterialien. Aus WO 2011/057706, WO 2015/014434 und WO 2015/169412 sind weitere Hostmaterialien bekannt, die unter anderem Triazin-Dibenzofuran-Carbazolderivate und Triazin-Dibenzothiophen-Carbazolderivate umfassen, wobei das Triazin gegebenenfalls mit einem Linker an das Dibenzofuran oder Dibenzothiophen gebunden ist. WO 2009/021126 und US 2015/0340618 beschreiben Terphenylen-Derivate als Hostmaterialien.

Eine weitere Möglichkeit, die Leistungsdaten elektronischer Vorrichtungen, insbesondere von organischen Elektrolumineszenzvorrichtungen, zu verbessern, besteht darin, Kombinationen aus zwei oder mehr Materialien, insbesondere Host-materialien bzw. Matrixmaterialien, zu verwenden.

US 6,392,250 B1 offenbart die Verwendung einer Mischung bestehend aus einem Elektronentransportmaterial, einem Lochtransportmaterial und einem fluoreszierenden Emitter in der Emissionsschicht einer OLED. Mit Hilfe dieser Mischung konnte die Lebensdauer der OLED gegenüber dem Stand der Technik verbessert werden.

US 6,803,720 B1 offenbart die Verwendung einer Mischung enthaltend einen phosphoreszierenden Emitter sowie ein Loch- und ein Elektronentransportmaterial in der Emissionsschicht einer OLED. Dabei sind sowohl das Loch- und das Elektrontransportmaterial kleine organische Moleküle.

In KR 101744248 oder EP3336159 wird eine OLED beschrieben, die eine zweite lichtemittierende Schicht enthält, welche wiederum zwei verschiedene Hostmaterialien 2-2 und 2-1 und einen Emitter enthält, wobei als Hostmaterial 2-2 die Verbindung 3-1 verwendet werden kann.

Gemäß WO 2015/192941 können beispielsweise ein bipolarer Host und ein neutraler Co-Host in einer Mischung verwendet werden, vorzugsweise zusammen mit einem Emitter oder Dotanden. Bipolarer Host und neutraler Co-Host werden so ausgewählt, dass folgende Bedingungen gelten:

$$|HOMO(C)| - \min\{|HOMO(D)|;|HOMO(B)|\} > 0.3$$

$$|HOMO(B)| - |HOMO(D)| < 0.15\ eV$$

$$|LUMO(B)| - |LUMO(C)| > 0.3\ eV$$

$$|LUMO(B)| - |LUMO(D)| > 0,$$

wobei HOMO(C) für die HOMO-Energie des neutralen Co-Hosts steht, HOMO(B) und HOMO(D) entsprechend für die HOMO-Energie des bipolaren Hosts bzw. des Dotanden stehen, LUMO(C), LUMO(B) und LUMO(D) entsprechend für die LUMO-Energie des neutralen Co-Hosts, des bipolaren Hosts bzw. des Dotanden stehen, und die Funktion min{|HOMO(D)|; |HOMO(B)|} den kleineren der beiden Werte |HOMO(D)| und |HOMO(B)| liefert.

Es wird weiterhin die Herstellung der OLED mit Kennzeichnung E21 beschrieben, die in der emittierenden Schicht die Hostmaterialien Verbindung 9a, Verbindung 13e und den gelb phosphoreszierenden Emitter 13j enthält. Die Strukturen der verwendeten Verbindungen sind im Folgenden aufgeführt:

| | | |
|---|---|---|
| 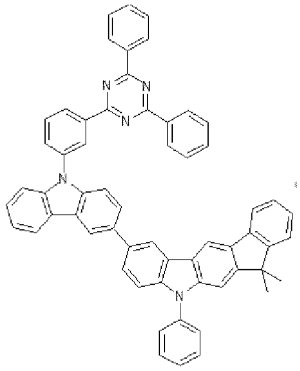 | 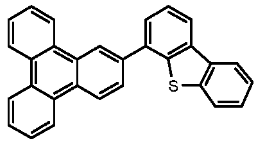 | 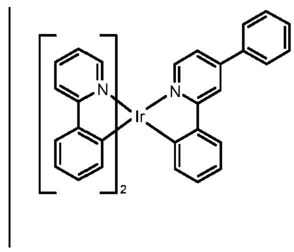 |
| 9a | 13e | 13j. |

Allerdings besteht bei Verwendung der Kombination von bipolaren Verbindungen mit neutralen Co-Hostmaterialien noch Verbesserungsbedarf, insbesondere in Bezug auf die Lebensdauer der organischen elektronischen Vorrichtung, insbesondere bei Verwendung in Kombination mit grün phosphoreszierenden Emittern.

Aufgabe der vorliegenden Erfindung ist daher die Bereitstellung von Materialien, welche sich für den Einsatz in einer organischen elektronischen Vorrichtung, insbesondere in einer organischen Elektrolumineszenzvorrichtung, und insbesondere in einer grün phosphoreszierenden OLED eignen und zu guten Device-Eigenschaften, insbesondere im Hinblick auf eine verbesserte Lebensdauer, führen, sowie die Bereitstellung der entsprechenden elektronischen Vorrichtung.

Es wurde nun gefunden, dass Zusammensetzungen, die bipolare Verbindungen der Formeln (1a) oder (1b) und Triphenylen-Verbindungen der Formel (2) umfassen, diese Aufgabe lösen und die Nachteile aus dem Stand der Technik beseitigen. Derartige Zusammensetzungen führen zu sehr guten Eigenschaften organischer elektronischer Vorrichtungen, insbesondere organischer Elektrolumineszenzvorrichtungen, insbesondere hinsichtlich der Lebensdauer und insbesondere auch bei Anwesenheit einer lichtemittierenden Komponente in der Emissionsschicht bei Konzentrationen zwischen 2 und 15 Gew.-%, insbesondere bei einer grün lichtemittierenden Komponente in der Emissionsschicht.

Ein erster Gegenstand der vorliegenden Erfindung ist daher eine Zusammensetzung umfassend mindestens eine Verbindung der Formel (1a) oder der Formel (1b) gemäß Anspruch 1 und mindestens eine Verbindung der Formel (2),

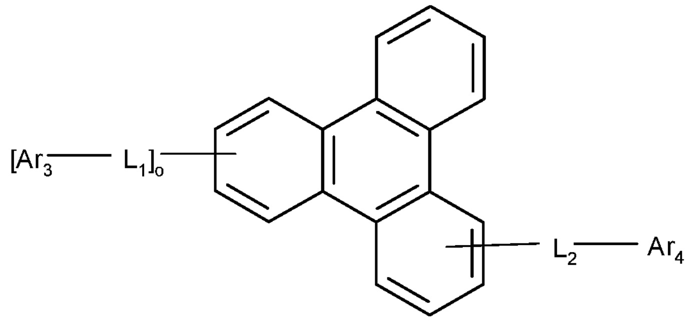

Formel (2)

wobei für die verwendeten Symbole und Indizes gilt:

- Y ist O oder S;
- Ar, $Ar_1$, $Ar_2$ sind jeweils unabhängig voneinander bei jedem Auftreten eine Aryl- oder Heteroarylgruppe mit 5 bis 40 Ringatomen, die mit einem oder mehreren Resten $R^3$ substituiert sein kann oder ein aromatisches oder heteroaromatisches Ringsystem mit 6 bis 40 Ringatomen, das mit einem oder mehreren Resten $R^3$ substituiert sein kann;
- p, q sind jeweils unabhängig voneinander 0, 1, 2, 3 oder 4;
- s, r sind jeweils unabhängig voneinander 0, 1, 2, 3 oder 4;
- R ist bei jedem Auftreten gleich oder verschieden ausgewählt aus der Gruppe bestehend aus D, F, Cl, Br, I, CN, $NO_2$, $N(Ar)_2$, $N(R^2)_2$, C(=O)Ar, C(=O)$R^2$, P(=O)$(Ar)_2$, $P(Ar)_2$, $B(Ar)_2$, $Si(Ar)_3$, $Si(R^2)_3$, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten $R^2$ substituiert sein kann, wobei eine oder mehrere nichtbenachbarte $CH_2$-Gruppen durch $R^2C=CR^2$, $Si(R^2)_2$, C=O, C=S, C=$NR^2$, P(=O)($R^2$), SO, $SO_2$, $NR^2$, O, S oder $CONR^2$ ersetzt sein können und wobei ein oder mehrere H-Atome durch D, F, Cl, Br, I, CN oder $NO_2$ ersetzt sein können, einem aromatischen oder heteroaromatischen Ringsystem mit 5 bis 40 Ringatomen, das jeweils mit einem oder mehreren Resten $R^2$ substituiert sein kann, einer Aryloxy- oder Heteroaryloxygruppe mit 5 bis 40 Ringatomen, die mit einem oder mehreren Resten $R^2$ substituiert sein kann, oder einer Aralkyl- oder Heteroaralkylgruppe mit 5 bis 40 Ringatomen, die mit einem oder mehreren Resten $R^2$ substituiert sein kann; dabei kann maximal ein Substituent R zusammen mit $Ar_1$ ein monocyclisches oder polycyclisches, aliphatisches, aromatisches oder heteroaromatisches Ringsystem bilden, das mit einem oder mehreren Resten $R^2$ substituiert sein kann;
- o ist 0 oder 1;
- $L_1$, $L_2$ sind bei jedem Auftreten gleich oder verschieden eine Einfachbindung oder ein aromatisches oder heteroaromatisches Ringsystem mit 5 bis 30 Ringatomen, das durch einen oder mehrere Reste $R^3$ substituiert sein kann;
- $Ar_3$ und Ar4 sind jeweils unabhängig voneinander ein aromatisches oder heteroaromatisches Ringsystem mit 6 bis 40 Ringatomen, das mit einem oder mehreren Resten $R^1$ substituiert sein kann;
- $R^1$ ist bei jedem Auftreten gleich oder verschieden ausgewählt aus der Gruppe bestehend aus H, D, F, Cl, Br, I, CN, $NO_2$, $N(Ar)_2$, $N(R^2)_2$, C(=O)Ar, C(=O)$R^2$, P(=O)$(Ar)_2$, $P(Ar)_2$, $B(Ar)_2$, $Si(Ar)_3$, $Si(R^2)_3$, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten $R^2$ substituiert sein kann, wobei eine oder mehrere nichtbenachbarte $CH_2$-Gruppen durch $R^2C=CR^2$, $Si(R^2)_2$, C=O, C=S, C=$NR^2$, P(=O)($R^2$), SO, $SO_2$, $NR^2$, O, S oder $CONR^2$ ersetzt sein können und wobei ein oder mehrere H-Atome durch D, F, Cl, Br, I, CN oder $NO_2$ ersetzt sein können, einem aromatischen oder heteroaromatischen Ringsystem mit 5 bis 40 Ringatomen, das jeweils mit einem oder mehreren Resten $R^2$ substituiert sein kann, einer Aryloxy- oder Heteroaryloxygruppe mit 5 bis 40 Ringatomen, die mit einem oder mehreren Resten $R^2$ substituiert sein kann, oder einer Aralkyl- oder Heteroaralkylgruppe mit 5 bis 40 Ringatomen, die mit einem oder mehreren Resten $R^2$ substituiert sein kann; dabei können optional zwei Substituenten $R^1$, die an dasselbe Kohlenstoffatom oder an benachbarte Kohlenstoffatome gebunden sind, ein monocyclisches oder polycyclisches, aliphatisches, aromatisches oder heteroaromatisches Ringsystem bilden, das mit einem oder mehreren Resten $R^2$ substituiert sein kann;
- $R^2$ ist bei jedem Auftreten gleich oder verschieden ausgewählt aus der Gruppe bestehend aus H, D, F, Cl, Br, I, CN, $NO_2$, $N(Ar)_2$, $NH_2$, $N(R^3)_2$, C(=O)Ar, C(=O)H, C(=O)$R^3$, P(=O)$(Ar)_2$, einer geradkettigen Alkyl-,

Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten $R^3$ substituiert sein kann, wobei eine oder mehrere nicht-benachbarte $CH_2$-Gruppen durch HC=CH, $R^3C=CR^3$, C≡C, $Si(R^3)_2$, $Ge(R^3)_2$, $Sn(R^3)_2$, C=O, C=S, C=Se, $C=NR^3$, $P(=O)(R^3)$, SO, $SO_2$, NH, $NR^3$, O, S, CONH oder $CONR^3$ ersetzt sein können und wobei ein oder mehrere H-Atome durch D, F, Cl, Br, I, CN oder $NO_2$ ersetzt sein können, einem aromatischen oder heteroaromatischen Ringsystem mit 5 bis 60 Ringatomen, das jeweils mit einem oder mehreren Resten $R^3$ substituiert sein kann, einer Aryloxy- oder Heteroaryloxygruppe mit 5 bis 60 Ringatomen, die mit einem oder mehreren Resten $R^3$ substituiert sein kann, oder einer Kombination dieser Systeme, wobei optional zwei oder mehr benachbarte Substituenten $R^2$ ein monocyclisches oder polycyclisches, aliphatisches, aromatisches oder heteroaromatisches Ringsystem bilden können, das mit einem oder mehreren Resten $R^3$ substituiert sein kann;

$R^3$ ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann; dabei können zwei oder mehr benachbarte Substituenten $R^3$ miteinander ein mono- oder polycyclisches, aliphatisches Ringsystem bilden.

Substituent der Formel (1-1) bedeutet

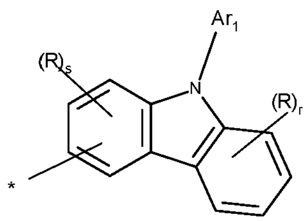

(Formel 1-1)

Weitere Gegenstände der Erfindung umfassen Formulierungen, die derartige Zusammensetzungen enthalten, die Verwendung dieser Zusammensetzungen in einer organischen elektronischen Vorrichtung, organische elektronische Vorrichtungen, bevorzugt Elektrolumineszenzvorrichtungen, die derartige Zusammensetzungen enthalten und dabei die Zusammensetzung bevorzugt in einer Schicht enthalten, sowie Verfahren zur Herstellung derartiger Vorrichtungen. Die entsprechenden bevorzugten Ausführungsformen, wie nachfolgend beschrieben, sind ebenfalls Gegenstand der vorliegenden Erfindung. Die überraschenden und vorteilhaften Effekte werden durch spezifische Selektion bekannter Materialien erreicht, insbesondere, was die Auswahl der bipolaren Materialien der Formel (1a) oder der Formel (1b) betrifft.

Die Schicht, die die Zusammensetzung umfassend mindestens eine Verbindung der Formel (1a) oder der Formel (1b) und mindestens eine Verbindung der Formel (2), wie zuvor beschrieben oder nachfolgend bevorzugt beschrieben, enthält, ist insbesondere eine emittierende Schicht (EML), eine Elektronentransportschicht (ETL), eine Elektroneninjektionsschicht (EIL) und/oder eine Lochblockierschicht (HBL).

Wenn es sich um eine emittierende Schicht handelt, dann ist es bevorzugt eine phosphoreszierende Schicht, die dadurch gekennzeichnet ist, dass sie zusätzlich zu der Zusammensetzung umfassend die Matrixmaterialien der Formeln (1a) oder (1b) und Formel (2), wie zuvor beschrieben, einen phosphoreszierenden Emitter enthält, insbesondere bevorzugt einen grün phosphoreszierenden Emitter.

Benachbarte Kohlenstoffatome im Sinne der vorliegenden Erfindung sind Kohlenstoffatome, die direkt miteinander verknüpft sind.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht.

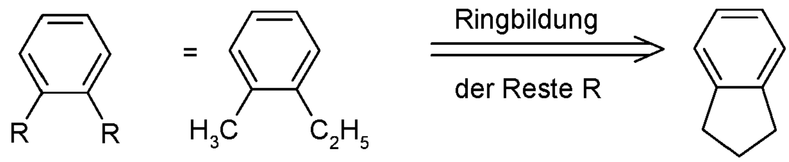


Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

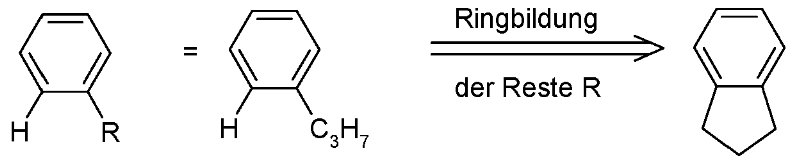


Eine Arylgruppe im Sinne dieser Erfindung enthält 6 bis 40 aromatische Ringatome, bevorzugt C-Atome. Eine Heteroarylgruppe im Sinne dieser Erfindung enthält 5 bis 40 aromatische Ringatome, wobei die Ringatome C-Atome und mindestens ein Heteroatom umfassen, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Phenyl, abgeleitet von Benzol, bzw. ein einfacher heteroaromatischer Cyclus, beispielsweise abgeleitet von Pyridin, Pyrimidin oder Thiophen, oder eine kondensierte Aryl- oder Heteroarylgruppe, beispielsweise Naphthalin, Anthracen, Phenanthren, Chinolin oder Isochinolin, verstanden.

Ein aromatisches Ringsystem im Sinne dieser Erfindung enthält 6 bis 40 C-Atome im Ringsystem und kann durch einen oder mehrere Reste $R^3$ substituiert sein, wobei $R^3$ eine nachfolgend beschriebene Bedeutung hat. Ein aromatisches Ringsystem umfasst auch Arylgruppen, wie zuvor beschrieben.

Ein heteroaromatisches Ringsystem im Sinne dieser Erfindung enthält 5 bis 40 Ringatome und mindestens ein Heteroatom und kann durch einen oder mehrere Reste $R^3$ substituiert sein, wobei $R^3$ eine nachfolgend beschriebene Bedeutung hat. Ein bevorzugtes heteroaromatisches Ringsystem hat 10 bis 40 Ringatome und mindestens ein Heteroatom und kann durch einen oder mehrere Reste $R^3$ substituiert sein, wobei $R^3$ eine nachfolgend beschriebene Bedeutung hat. Ein heteroaromatisches Ringsystem umfasst auch Heteroarylgruppen, wie zuvor beschrieben. Die Heteroatome im heteroaromatischen Ringsystem sind bevorzugt ausgewählt aus N, O und/oder S.

Unter einem aromatischen oder heteroaromatischen Ringsystem im Sinne dieser Erfindung wird ein System verstanden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische bzw. heteroaromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sind Systeme, in denen zwei oder mehrere Aryl- oder Heteroarylgruppen direkt aneinander gebunden sind, wie z. B. Biphenyl, Terphenyl, Quaterphenyl oder Bipyridin, ebenfalls von der Definition des aromatischen bzw. heteroaromatischen Ringsystems umfasst.

Unter einem aromatischen oder heteroaromatischen Ringsystem mit 5 - 40 Ringatomen, welches noch jeweils mit den genannten Resten $R^3$ substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Unter einer cyclischen Alkyl-, Alkoxy- oder Thioalkylgruppe im Sinne dieser Erfindung wird eine monocyclische, eine bicyclische oder eine polycyclische Gruppe verstanden.

Im Rahmen der vorliegenden Erfindung werden unter einer $C_1$- bis $C_{20}$-Alkylgruppe, in der auch einzelne H-Atome oder $CH_2$-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyln-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyln-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden.

Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden.

Unter einer Alkinylgruppe werden beispielsweise Ethinyl, Propinyl, Butinyl, Pentinyl, Hexinyl, Heptinyl oder Octinyl verstanden.

Unter einer $C_1$- bis $C_{20}$-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einer $C_1$- bis $C_{20}$-Thioalkylgruppe werden beispielsweise S-Alkylgruppen verstanden, beispielsweise Thiomethyl, 1-Thioethyl, 1-Thio-i-propyl, 1-Thio-n-propyl, 1-Thio-i-butyl, 1-Thio-n-butyl oder 1-Thio-t-butyl.

Eine Aryloxy- oder Heteroaryloxygruppe mit 5 bis 40 Ringatomen bedeutet O-Aryl oder O-Heteroaryl und bedeutet, dass die Aryl- bzw. Heteroarylgruppe über ein Sauerstoffatom gebunden wird.

Eine Aralkyl- oder Heteroaralkylgruppe mit 5 bis 40 Ringatomen bedeutet, dass eine Alkylgruppe, wie zuvor beschrieben, mit einer Arylgruppe bzw. Heteroarylgruppe substituiert ist.

Ein phosphoreszierender Emitter im Sinne der vorliegenden Erfindung ist eine Verbindung, die Lumineszenz aus einem angeregten Zustand mit höherer Spinmultiplizität zeigt, also einem Spinzustand > 1, insbesondere aus einem angeregten Triplettzustand. Im Sinne dieser Anmeldung sollen alle lumineszierenden Komplexe mit Übergangsmetallen oder Lanthaniden als phosphoreszierende Emitter angesehen werden. Eine genauere Definition erfolgt weiter unten.

Wenn die Zusammensetzung umfassend mindestens eine Verbindung der Formeln (1a) oder (1b), wie zuvor beschrieben oder nachfolgend bevorzugt beschrieben, und mindestens eine Verbindung der Formel (2), wie zuvor beschrieben oder nachfolgend beschrieben, als Matrixmaterial für einen phosphoreszierenden Emitter eingesetzt wird, ist es bevorzugt, wenn deren Triplettenergie nicht wesentlich kleiner als die Triplettenergie des phosphoreszierenden Emitters ist. Dabei gilt bevorzugt für das Triplettniveau $T_1$(Emitter) - $T_1$(Matrix) $\leq$ 0.2 eV, besonders bevorzugt $\leq$ 0.15 eV, ganz besonders bevorzugt $\leq$ 0.1 eV. Dabei ist $T_1$(Matrix) das Triplettniveau des Matrixmaterials in der Emissionsschicht, wobei diese Bedingung für jedes der beiden Matrixmaterialien gilt, und $T_1$(Emitter) ist das Triplettniveau des phosphoreszierenden Emitters. Enthält die Emissionsschicht mehr als zwei Matrixmaterialien, so gilt die oben genannte Beziehung bevorzugt auch für jedes weitere Matrixmaterial. Im Folgenden wird der Ausdruck Dotand synonym zu Emitter benutzt.

Besonders vorteilhaft hinsichtlich der Leistungsdaten und Lebensdauer elektronischer Vorrichtungen ist, wenn die Komponenten der Zusammensetzung die folgenden Bedingungen erfüllen

$$|HOMO(C)| - \min\{|HOMO(D)|;|HOMO(B)|\} > 0.3$$

$$|HOMO(B)| - |HOMO(D)| < 0.15\ eV$$

$$|LUMO(B)| - |LUMO(C)| > 0.3\ eV$$

$$|LUMO(B)| - |LUMO(D)| > 0,$$

wobei HOMO(C) für die HOMO-Energie des neutralen Co-Hosts steht, HOMO(B) und HOMO(D) entsprechend für die HOMO-Energie des bipolaren Hosts bzw. des Dotanden stehen, LUMO(C), LUMO(B) und LUMO(D) entsprechend für die LUMO-Energie des neutralen Co-Hosts, des bipolaren Hosts bzw. des Dotanden stehen, und die Funktion min{|HOMO(D)|; |HOMO(B)|} den kleineren der beiden Werte |HOMO(D)| und |HOMO(B)| liefert und wobei |HOMO| bzw. |LUMO| für den absoluten Betrag des jeweiligen Wertes steht.

Die angegebenen Energiewerte beziehen sich dabei auf isolierte Verbindungen, und werden, wie im Folgenden dargelegt, ermittelt.

Die HOMO (highest occupied molecular orbital) und LUMO (lowest unoccupied molecular orbital) Energien sowie das Triplettniveau der Materialien werden über quantenchemische Rechnungen bestimmt. Hierzu wird vorliegend das Programm¬paket "Gaussian09, Revision D.01" (Gaussian Inc.) verwendet. Es können aber auch andere Programmpakete verwendet werden, solange darin dieselben Methoden implementiert wurden. Zur Berech¬nung organischer Substanzen ohne Metalle (mit Methode "org." bezeichnet) wird zuerst eine Geometrie¬optimierung mit der semi-empirischen Methode AM1 (Gaussian-Einga¬be¬zeile"# AM1 opt") mit der Ladung (Charge) 0 und der Multiplizität (Multiplicity) 1 durchgeführt. Im Anschluss erfolgt auf Grundlage der optimierten Geo¬me¬trie eine Energie-rechnung (single point) für den elektronischen Grundzustand und das Triplett-Niveau. Hierbei wird die TDDFT-Methode (Time Dependent Density Functional Theory) B3PW91 mit dem Basissatz 6-31 G(d) (Gaussian-Eingabezeile "# B3PW91/6-31G(d) td=(50-50,nstates=4)") verwendet (Ladung 0, Multiplizität 1). Für metallorganische Verbindungen (mit Methode "M-org." bezeichnet) wird die Geometrie mit der Methode Hartree-Fock und dem Basissatz LanL2MB (Gaussian-Eingabezeile "# HF/LanL2MB opt") optimiert (Ladung 0, Multiplizität 1). Die Energierechnung erfolgt analog zu den organischen Substanzen, wie oben beschrieben, mit dem Unterschied, dass für das Metallatom der Basissatz "LanL2DZ" und für die Liganden der Basissatz "6-31 G(d)" verwendet wird (Gaussian-Eingabezeile "#B3PW91/gen pseudo=lanl2 td=(50-50,nstates=4)"). Aus der Energie-rechnung erhält man das HOMO als das letzte mit zwei Elektronen besetze Orbital (Alpha occ. eigenvalues) und LUMO als das erste unbesetzte Orbital (Alpha virt. eigenvalues) in Hartree-Einheiten (HEh bzw. LEh). Daraus wird der anhand von Cyclo¬voltammetriemessungen kalibrierte HOMO- und LUMO-Wert in Elektronen¬volt wie folgt bestimmt:

$$LUMO(eV) = (1.0658*LEh*27.212)-0.5049$$

$$HOMO(eV) = (0.8308*HEh*27.212)-1.1180$$

Diese Werte sind im Sinne dieser Anmeldung als HOMO bzw. als LUMO der Materialien anzusehen.

Das Triplett-Niveau T1 eines Materials ist definiert als die relative Anregungs¬energie (in eV) des Triplettzustands mit der niedrigsten Energie, der sich aus der quanten¬chemischen Energierechnung ergibt.

Die oben genannten Bedingungen sowie das Verfahren zur Bestimmung der einzelnen Energiewerte gestatten dem Fachmann auf diesem Gebiet auf einfache Weise, die geeigneten Verbindungen aus dem Stand der Technik zu identifizieren. Die Berechnung von Orbitalenergien stellt für den Fachmann eine Routinetätigkeit dar, die er mit Hilfe des oben genannten Verfahrens in kurzer Zeit durchführen kann.

Dem Fachmann ist bekannt, dass ein bipolarer Host ein solcher ist, der in der verwendeten Mischung im verwendeten Bauteil signifikant sowohl zum Elektronen- als auch zum Lochtransport beiträgt. Dem Fachmann ist weiterhin bekannt, dass dies dadurch zu erreichen ist, dass ein Material ausgewählt wird, (a) in das aufgrund seiner Energieniveaulagen im Vergleich zu den Energieniveaulagen weiterer in derselben Mischung verwendeter Materialien signifikant sowohl Elektronen als auch Löcher injiziert werden und (b) in dem der Transport nicht aufgrund extrem niedriger Elektronen- oder Lochmobilität (kleiner als 10-8 $cm^2/(Vs)$) unterdrückt ist. Die Messung von Elektronen- und Lochmobilitäten werden routinemäßig vom Fachmann mittels Standardverfahren durchgeführt.

Bipolare Hosts der Formeln (1a) oder (1b):

In einer Ausführungsform der Erfindung werden Verbindungen der Formeln (1a)oder (1b) ausgewählt, in denen Y ausgewählt ist aus O oder S und der Substituent

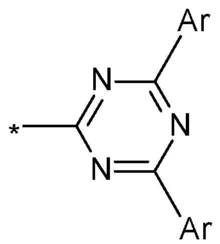

in Position 1, 2, 3 oder 4 des Dibenzofurans oder Dibenzothiophens gebunden ist, wobei X, Ar, $Ar_1$, $Ar_2$, R, n, p, q, r und s eine zuvor angegebene Bedeutung haben oder eine nachfolgend angegebene Bedeutung haben und * die Verknüpfungsstelle mit dem Dibenzofuran bzw. Dibenzothiophen kennzeichnet.

Bevorzugt steht Y für O.

Verbindungen der Formel (1a) sind wie folgt-dargestellt,

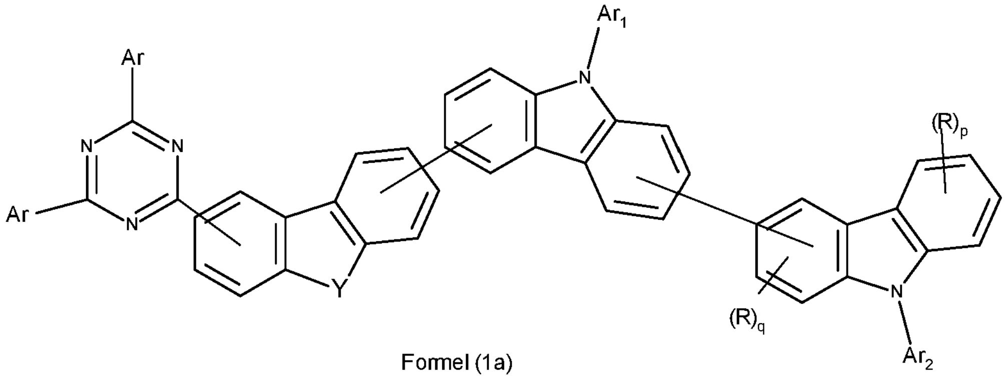

Formel (1a) ,

wobei Y, Ar, $Ar_1$, $Ar_2$, R, p und q eine zuvor angegebene Bedeutung haben oder eine nachfolgend angegebene Bedeutung haben.

Verb indungen der Formel (1b) sind wie folgt dargestellt,

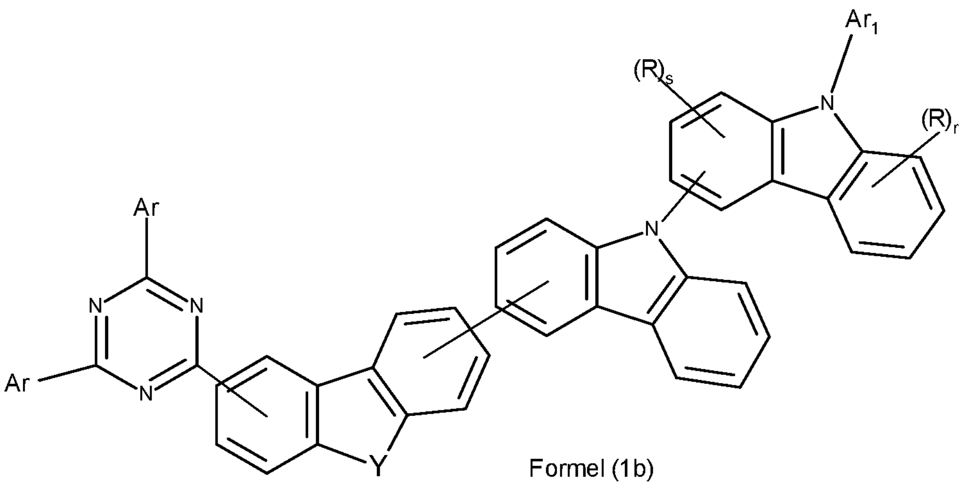

Formel (1b)

wobei Y, Ar, $Ar_1$, $Ar_2$, R, p und q eine zuvor angegebene Bedeutung ,haben oder eine nachfolgend angegebene Bedeutung haben.

Bevorzugt wird mindestens eine Verbindung der Formel (1a) für die Zusammensetzung ausgewählt, mit Substituenten, die zuvor beschrieben oder nachfolgend bevorzugt beschrieben werden.

Bevorzugt wird mindestens eine Verbindung der Formel (1b) für die Zusammensetzung ausgewählt, mit Substituenten, die zuvor beschrieben oder nachfolgend bevorzugt beschrieben werden.

In einer Ausführungsform der Erfindung werden Verbindungen der Formel (1a) oder (1b) ausgewählt, in denen Y ausgewählt ist aus O oder S und der Substituent

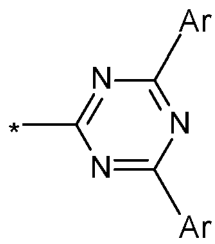

in Position 1, 2, 3 oder 4 des Dibenzofurans oder Dibenzothiophens gebunden ist und die Carbazoleinheit in Position 6, 7, 8 oder 9 des Dibenzofurans oder Dibenzothiophens gebunden ist, wobei Y, X, Ar, $Ar_1$, $Ar_2$, R, p, q, r und s eine zuvor angegebene Bedeutung haben oder eine nachfolgend angegebene Bedeutung haben und * die Verknüpfungsstelle mit dem Dibenzofuran bzw. Dibenzothiophen kennzeichnet.

Es ist weiter bevorzugt, wenn die Carbazoleinheit in Position 7 oder 8 des Dibenzofurans oder Dibenzothiophens gebunden ist.

Besonders bevorzugt wird demzufolge eine Verbindung der Formel (1a) oder (1b) für die Zusammensetzung ausgewählt, in denen die Carbazoleinheit in Position 7 oder 8 des Dibenzofurans oder Dibenzothiophens gebunden ist.

In dieser Ausführungsform werden Verbindungen der Formeln (1c), (1d), (1e) oder (1f) bevorzugt für die Zusammensetzung ausgewählt,

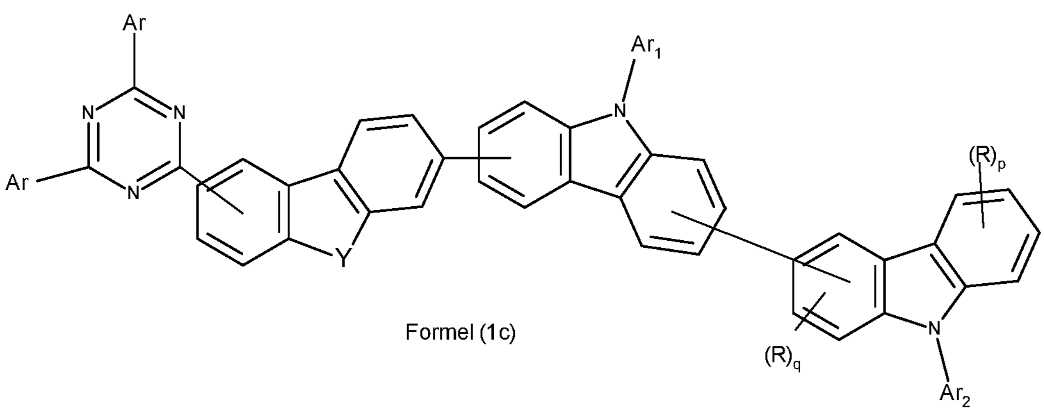

Formel (1c)

,

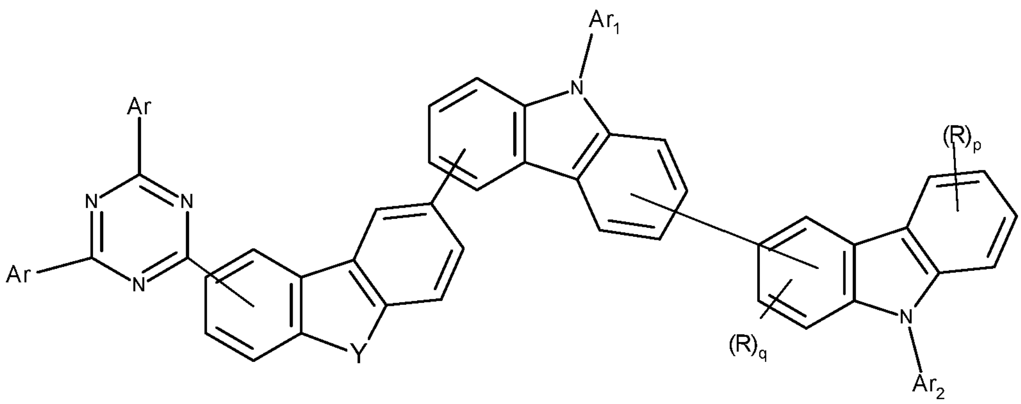

Formel (1d)

,

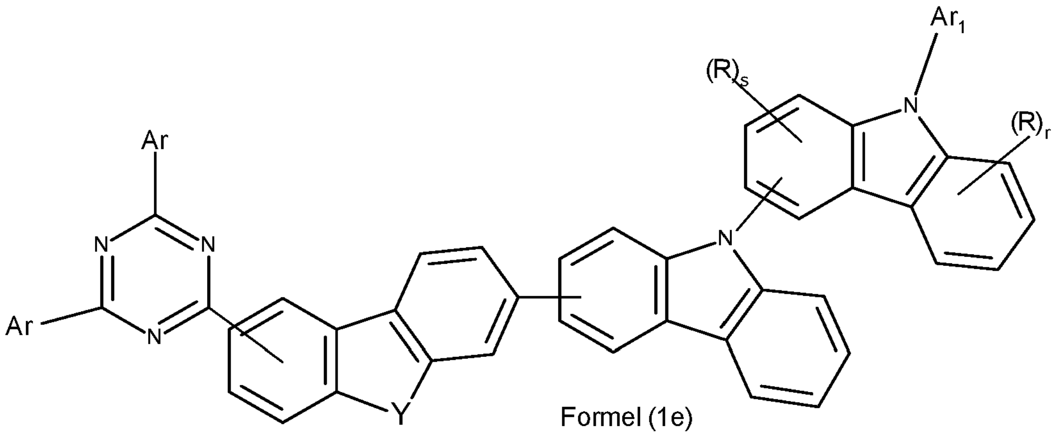

Formel (1e)

,

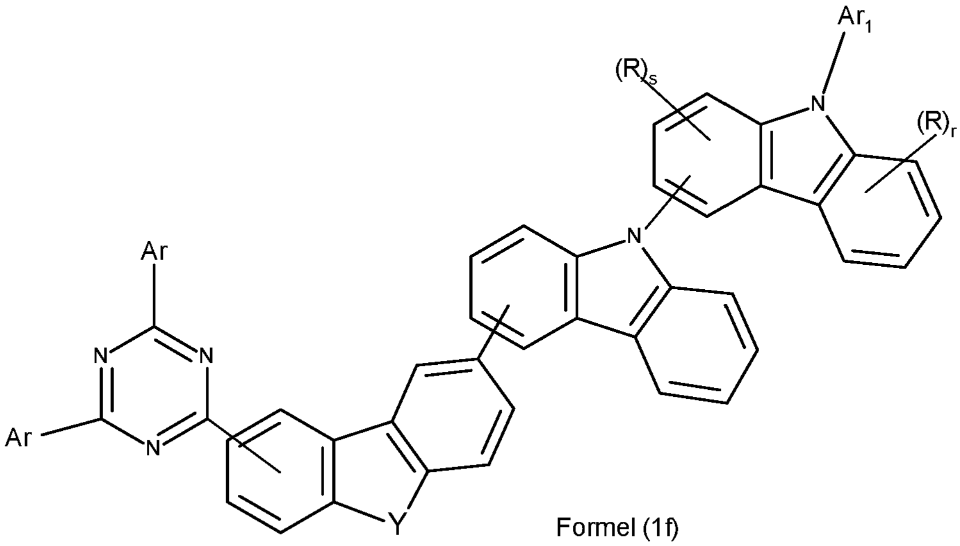

Formel (1f)

,

wobei Y, Ar, $Ar_1$, $Ar_2$, R, p, q, r und s eine zuvor angegebene Bedeutung haben oder eine nachfolgend angegebene Bedeutung haben.

Es ist weiter bevorzugt, wenn die Dibenzofuran- oder Dibenzothiopheneinheit in Position 3 des Carbazols verknüpft ist.

Besonders bevorzugt wird demzufolge eine Verbindung der Formeln (1a), (1b), (1c), (1d),(1e) oder (1f) für die Zusammensetzung ausgewählt, in denen die Dibenzofuran- bzw. Dibenzothiopheneinheit in Position 3 des Carbazols gebunden ist.

In dieser Ausführungsform werden Verbindungen der Formeln (1g), (1h), (1i) oder (1j) bevorzugt für die Zusammensetzung ausgewählt,

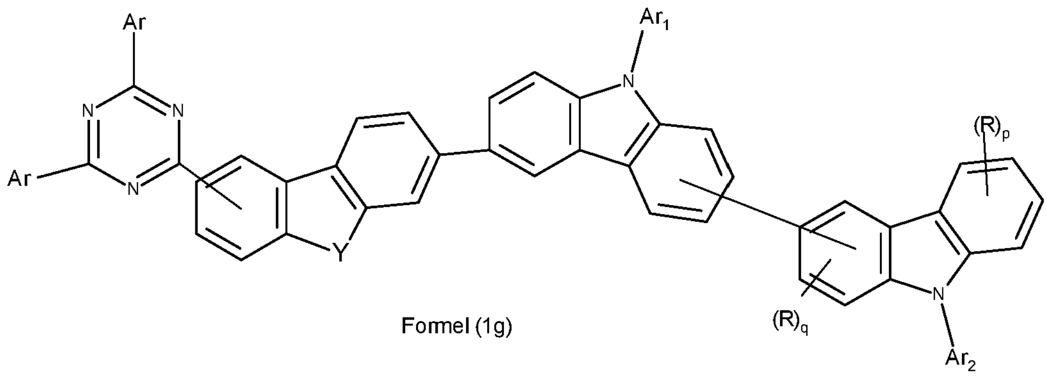

Formel (1g)

,

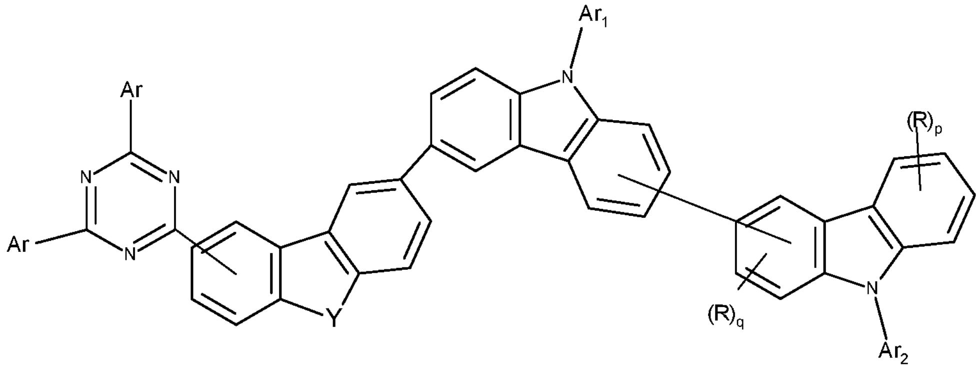

Formel (1h)

,

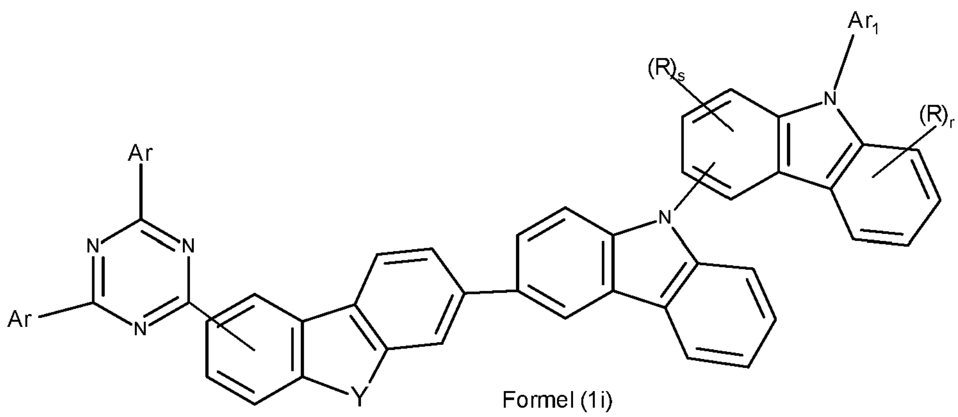

Formel (1i)

,

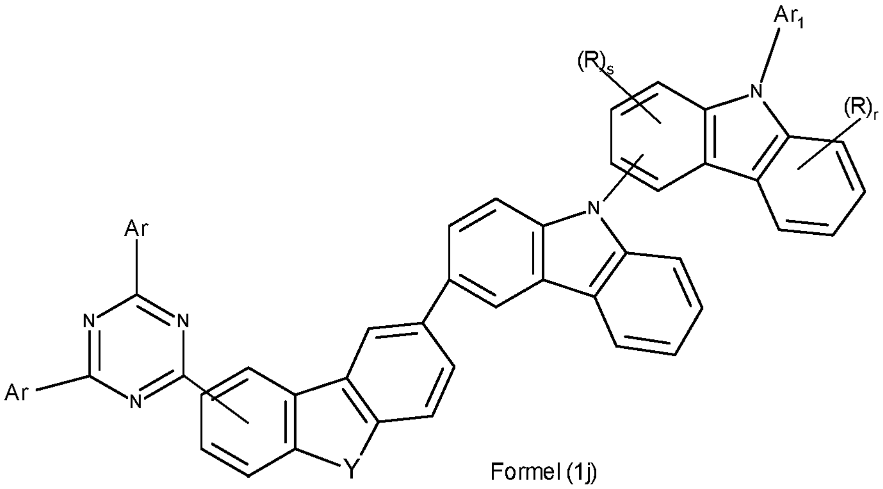

Formel (1j)

, wobei Y, Ar, $Ar_1$, $Ar_2$, R, p, q, r und s eine zuvor angegebene Bedeutung haben oder eine nachfolgend angegebene Bedeutung haben.

In Verbindungen der Formeln (1a), (1b), (1c), (1 d), (1 e), (1f), (1g), (1h), (1i) oder (1j) oder bevorzugt beschriebenen Verbindungen der Formeln (1a), (1b), (1c), (1d), (1 e), (1f), (1g), (1h),(1i) oder (1j) steht Ar jeweils unabhängig voneinander bevorzugt für eine Arylgruppe mit 6 bis 40 C-Atomen, wie zuvor beschrieben oder bevorzugt beschrieben, die mit einem oder mehreren Resten $R^3$ substituiert sein kann. Besonders bevorzugt steht mindestens ein Ar für Phenyl und der andere aromatische Substituent Ar steht für eine Arylgruppe mit 6 bis 40 C-Atomen, die mit einem oder mehreren Resten $R^3$ substituiert sein kann. Besonders bevorzugt stehen beide Gruppen Ar für Phenyl.

In Verbindungen der Formeln (1a), (1b), (1c), (1d), (1e), (1f), (1g), (1h), (1i) oder (1j) oder bevorzugt beschriebenen Verbindungen der Formeln (1a), (1b), (1c), (1d),(1e), (1f), (1g), (1h),(1i) oder (1j) stehen $Ar_1$ und $Ar_2$ jeweils unabhängig voneinander bevorzugt für eine Arylgruppe mit 6 bis 40 C-Atomen, wie zuvor beschrieben oder bevorzugt beschrieben, die mit einem oder mehreren Resten $R^3$ substituiert sein kann. Besonders bevorzugt stehen $Ar_1$ und $Ar_2$ jeweils unabhängig voneinander für Phenyl oder Naphthyl, das jeweils mit einem oder mehreren Resten $R^3$ substituiert sein kann.

Wenn in Verbindungen der Formeln (1a), (1b), (1c), (1d),(1e), (1f), (1g), (1h), (1i) oder (1j) oder bevorzugt beschriebenen Verbindungen der Formeln (1a), (1b), (1c), (1d), (1e), (1f), (1g), (1h),(1i) oder (1j) $Ar_1$ und $Ar_2$, wie zuvor beschrieben oder bevorzugt beschrieben, eine Arylgruppe bedeutet, die mit einem oder mehreren Resten $R^3$ substituiert ist, so wird der Substituent $R^3$ bei jedem Auftreten gleich oder verschieden bevorzugt ausgewählt aus der Gruppe bestehend aus D, F, einer geradkettigen oder verzweigten Alkylgruppe mit 1 bis 20 C-Atomen oder einem aromatischen oder heteroaromatischen Ringsystem mit 5 bis 40 Ringatomen. Das heteroaromatische Ringsystem mit 5 bis 40 Ringatomen ist für diese/diesen Substituenten $R^3$ bevorzugt abgeleitet von Dibenzofuran oder Dibenzothiophen. Das aromatische Ringsystem mit 6 bis 40 Ringatomen ist für diese/diesen Substituenten $R^3$ bevorzugt Phenyl, Biphenyl oder Terphenyl, besonders bevorzugt Phenyl. Die geradkettige oder verzweigte Alkylgruppe ist für diese/diesen Substituenten $R^3$ bevorzugt Methyl, Ethyl, n-Propyl oder n-Butyl, besonders bevorzugt Methyl.

In Verbindungen der Formeln (1a), (1b), (1c), (1 d), (1 e), (1f), (1g), (1h), (1i) oder (1j) oder bevorzugt beschriebenen Verbindungen der Formeln (1a), (1b), (1c), (1d),(1e), (1f), (1g), (1h),(1i) oder (1j) sind p, q, r und s jeweils unabhängig voneinander bevorzugt 0 oder 1; besonders bevorzugt sind p und q 0.

In Verbindungen der Formeln (1a), (1c), (1d), (1g) oder (1h) oder bevorzugt beschriebenen Verbindungen der Formeln (1a), (1c), (1d), (1g) oder (1h) sind p und q jeweils unabhängig voneinander bevorzugt 0 oder 1, wobei im Fall p = 1 und q = 0 oder q = 1 und p =0, R bevorzugt mit dem Substituenten $Ar_2$ ein monocyclisches oder polycyclisches, aliphatisches, aromatisches oder heteroaromatisches Ringsystem bildet, das mit einem oder mehreren Resten $R^2$ substituiert sein kann. Bevorzugt ist das sich bildende Ringsystem unsubstituiert.

Bevorzugte Beispiele für die Bildung eines derartigen Ringsystems sind die Substituenten der Formeln (1-2), (1-3), (1-4), (1-5) und (1-6), bevorzugt der Substituenten der Formeln (1-4), (1-5) und (1-6);

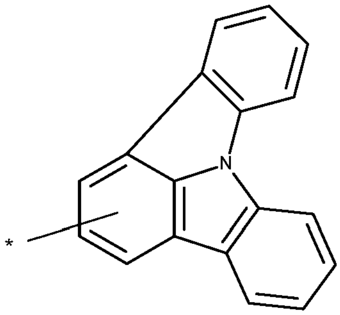

Formel (1-2) ,

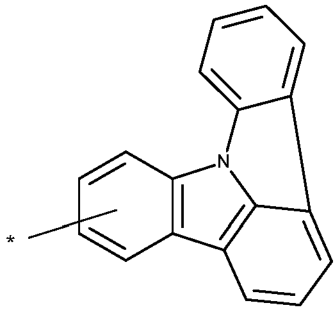

Formel (1-3) ,

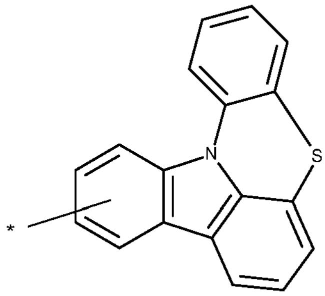

Formel (1-4)

,

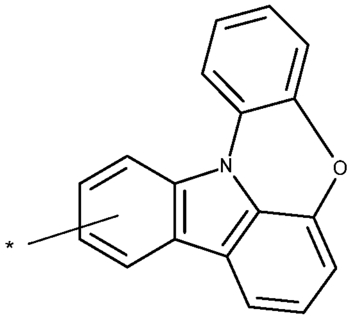

Formel (1-5) ,

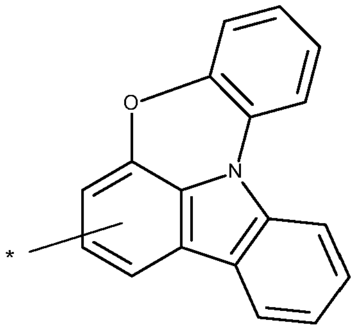

Formel (1-6) ,

wobei die Anknüpfungsstelle dieses Substituenten mit * markiert ist und in Position 5, 6, 7 oder 8 der Carbazoleinheit möglich ist.

In Verbindungen der Formeln (1b), (1e), (1f), (1i) oder (1j) oder bevorzugt beschriebenen Verbindungen der Formeln (1b), (1e), (1f), (1i) oder (1j) sind r und s jeweils unabhängig voneinander bevorzugt 0 oder 1, wobei im Fall r = 1 und s = 0 oder s = 1 und r = 0, R bevorzugt mit dem Substituenten $Ar_1$ ein monocyclisches oder polycyclisches, aliphatisches, aromatisches oder heteroaromatisches Ringsystem bildet, das mit einem oder mehreren Resten $R^2$ substituiert sein kann. Bevorzugt ist das sich bildende Ringsystem unsubstituiert.

Bevorzugte Beispiele für die Bildung eines derartigen Ringsystems im Substituenten der Formel (1-1) sind die Substituenten der Formeln (1-2), (1-3), (1-4), (1-5) und (1-6), bevorzugt der Formeln (1-2) und (1-3);

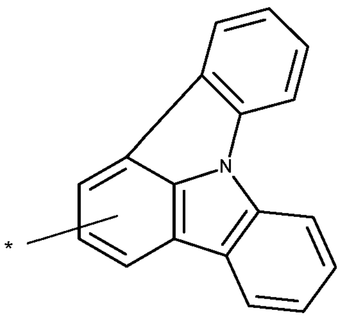

Formel (1-2) ,

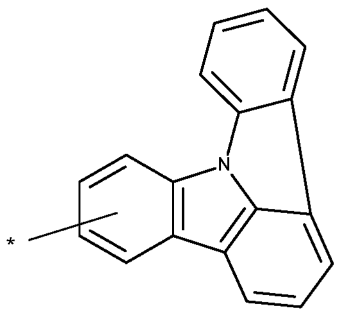

Formel (1-3) ,

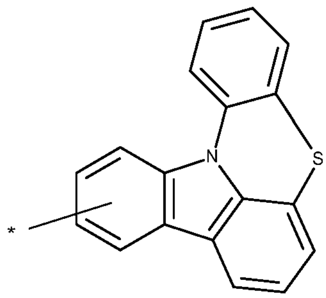

Formel (1-4) ,

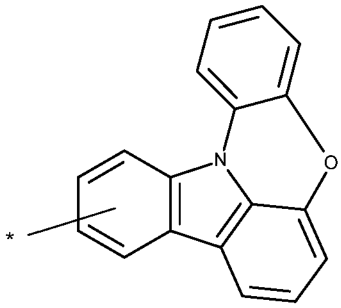

Formel (1-5) ,

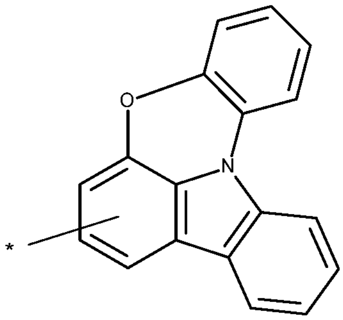

Formel (1-6) .

Wenn in Verbindungen der Formeln (1a), (1b), (1c), (1 d), (1 e), (1f), (1g), (1h), (1i) oder (1j) oder bevorzugt beschriebenen Verbindungen der Formeln (1a), (1b), (1c), (1d), (1e), (1f), (1g), (1h), (1i) oder (1j) p, q, r und s 1 bedeuten, so wird der Substituent R bei jedem Auftreten gleich oder verschieden bevorzugt ausgewählt aus der Gruppe bestehend aus D, F, einer Alkylgruppe mit 1 bis 20 C-Atomen oder einem aromatischen oder heteroaromatischen Ringsystem mit 5 bis 40 Ringatomen. Das heteroaromatische Ringsystem mit 5 bis 40 Ringatomen ist für diesen Substituenten R bevorzugt abgeleitet von Dibenzofuran oder Dibenzothiophen. Das aromatische Ringsystem mit 6 bis 40 Ringatomen ist für diesen Substituenten R bevorzugt Phenyl, Biphenyl oder Terphenyl, besonders bevorzugt Phenyl. Die Alkylgruppe mit 1 bis 20 C-Atomen ist für diesen Substituenten R bevorzugt eine lineare oder verzweigte Alkylgruppe mit 1 bis 4 C-Atomen, besonders bevorzugt Methyl, Ethyl, n-Propyl oder n-Butyl, ganz besonders bevorzugt Methyl.

Beispiele für geeignete Verbindungen der Formeln (1a), (1b), (1c), (1d), (1e), (1f), (1g), (1h), (1i) oder (1j), die erfindungsgemäß ausgewählt werden, sind die nachstehend genannten Strukturen der Tabelle 1.

Tabelle 1:

| | | |
|---|---|---|
| 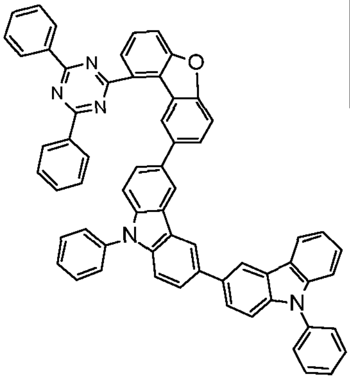 1 | 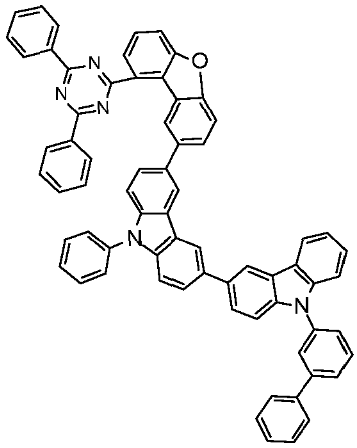 2 | 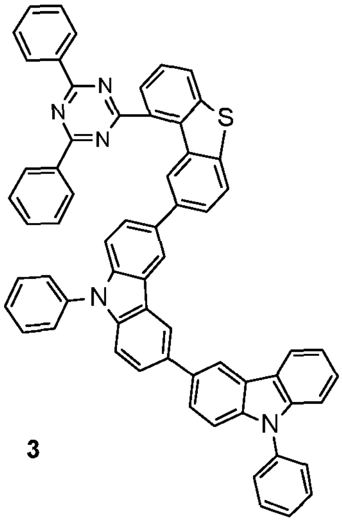 3 |
| 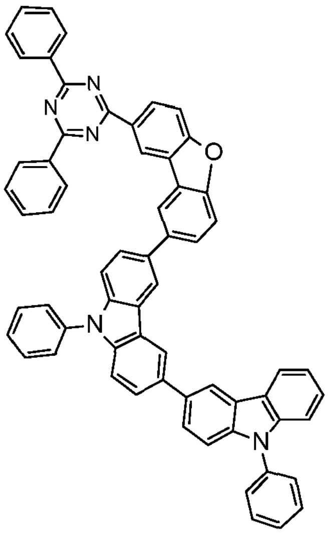 4 | 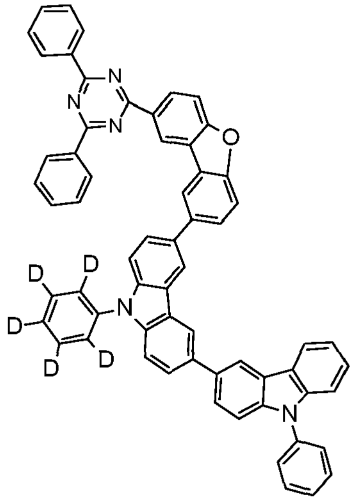 | 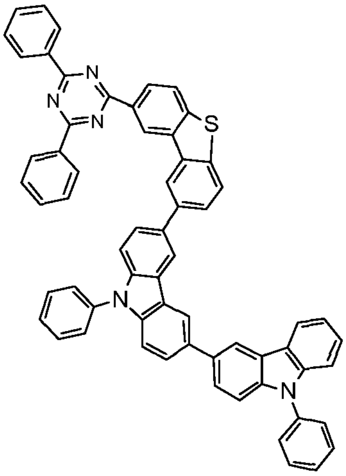 |
| 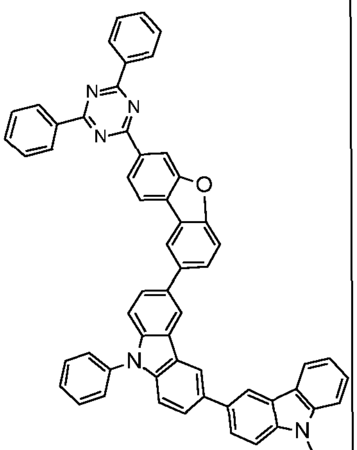 | 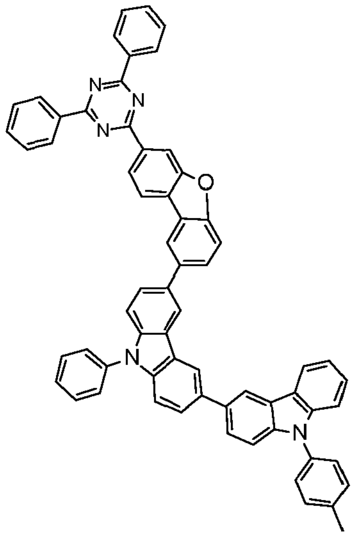 | 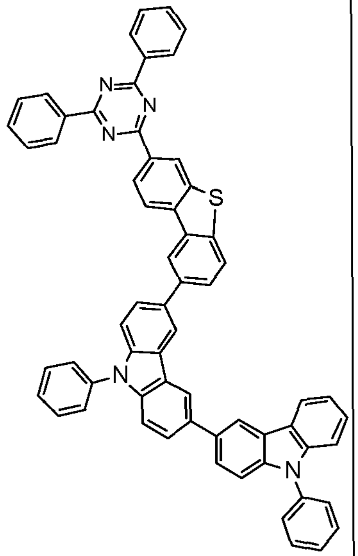 |

| | | |
|---|---|---|
| 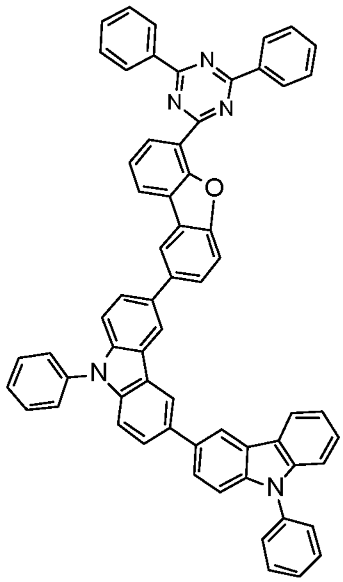<br>**5** | 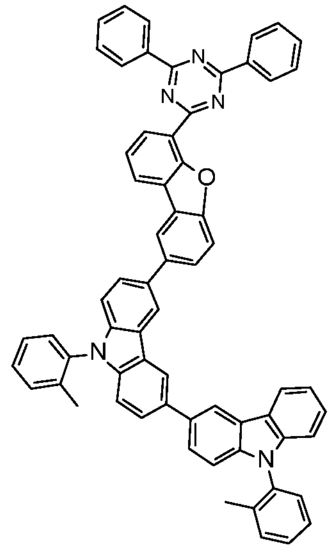 | 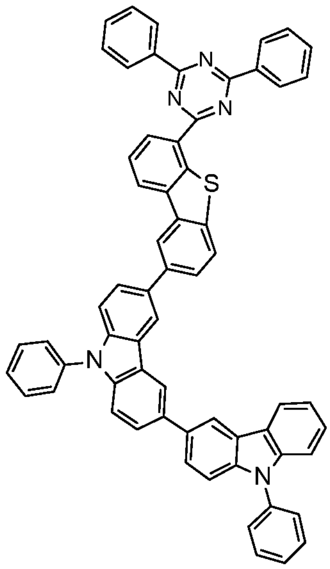 |
| 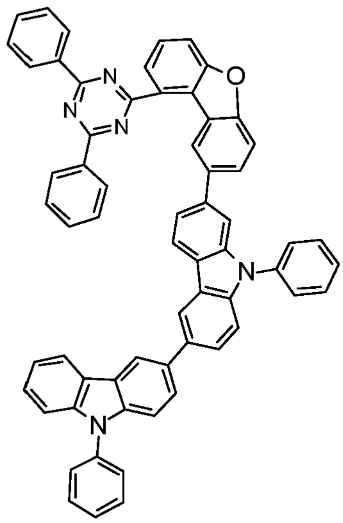<br>**6** | 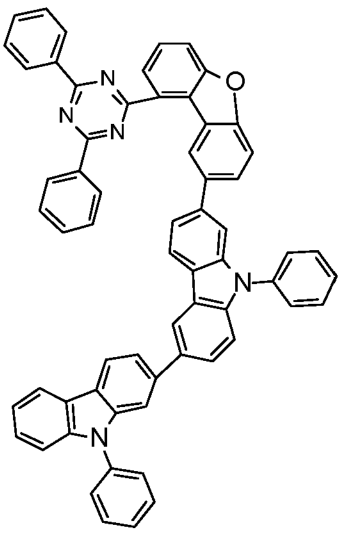 | 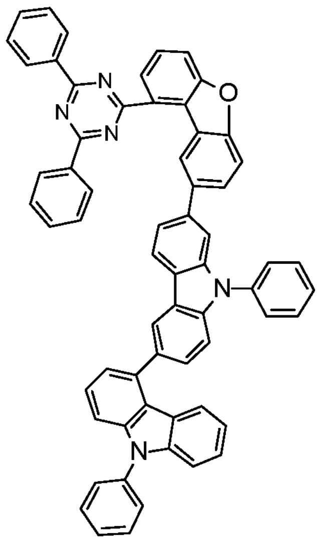 |

| | | |
|---|---|---|
| 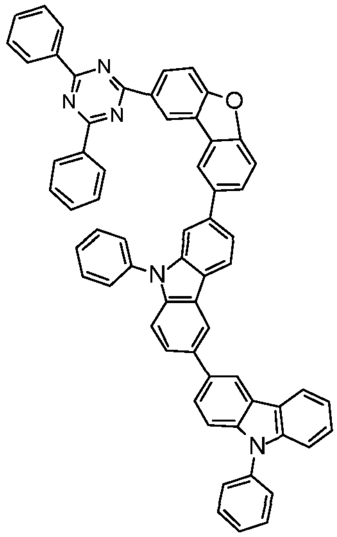 | 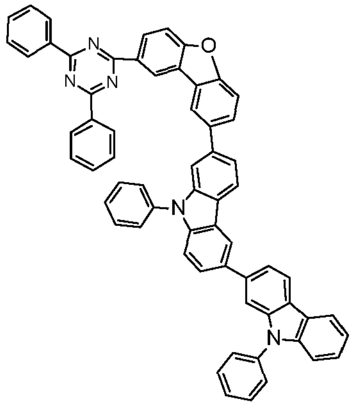 | 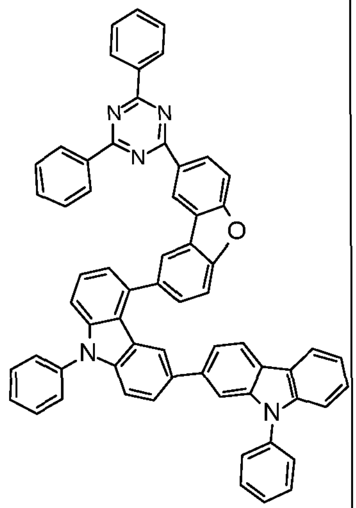 |
| 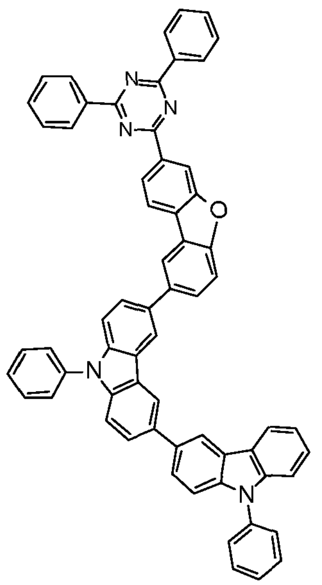 | 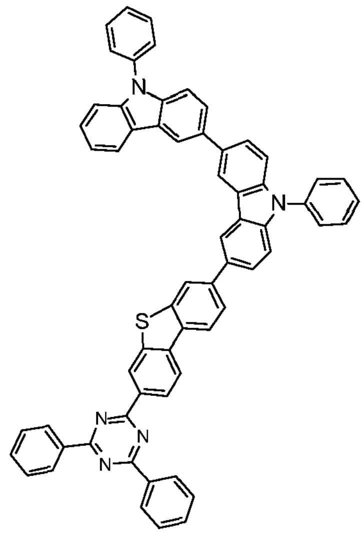<br>7 | 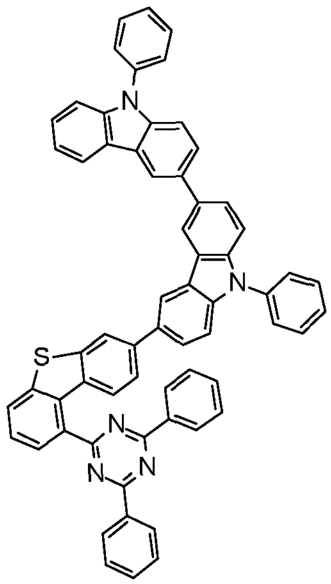 |
| 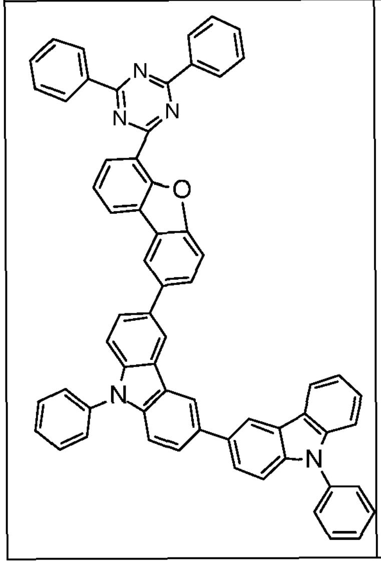 | 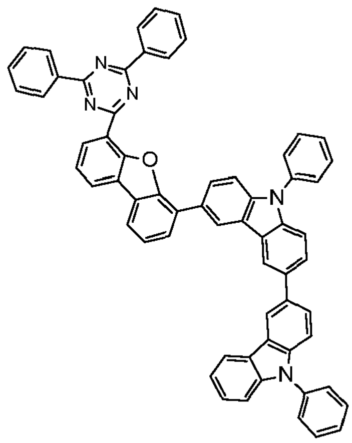 | 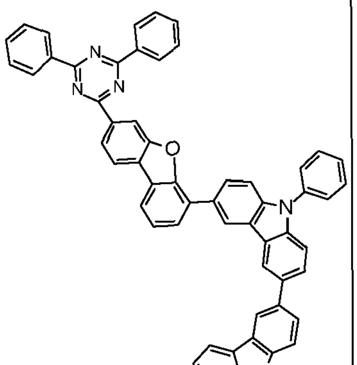 |

| | | |
|---|---|---|
| 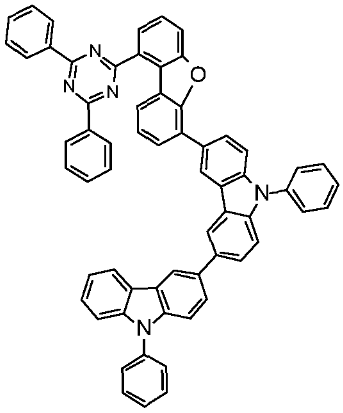 | 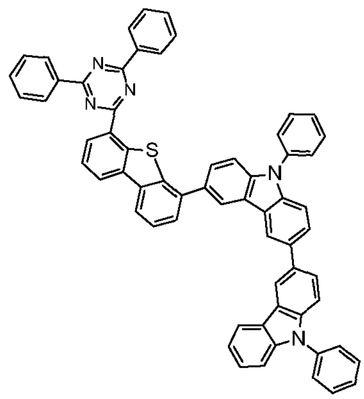 | 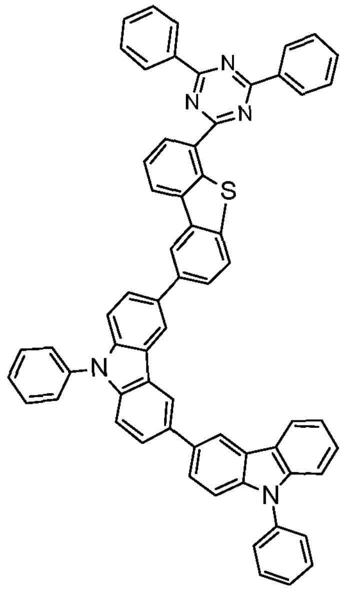 |
| 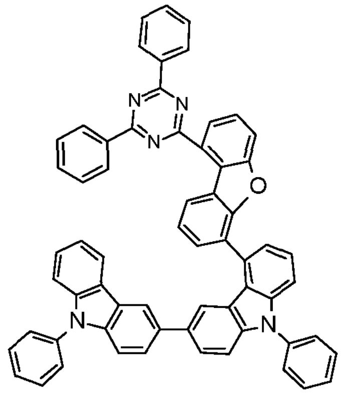 | 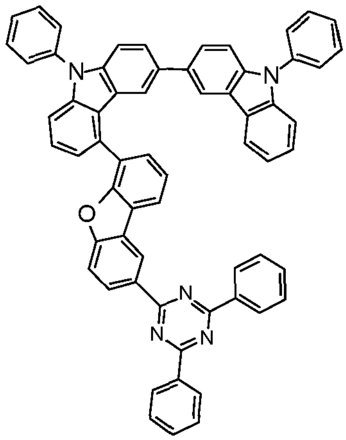 | 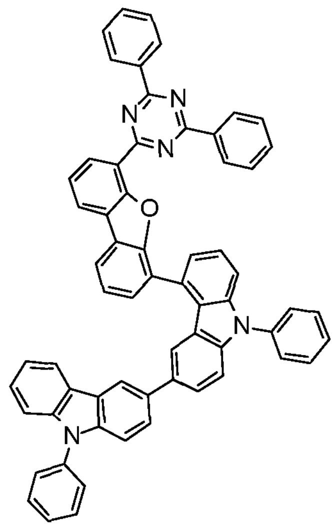 |
| 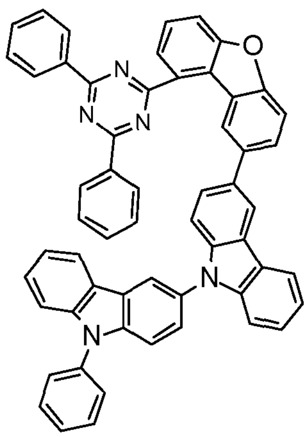 8 | 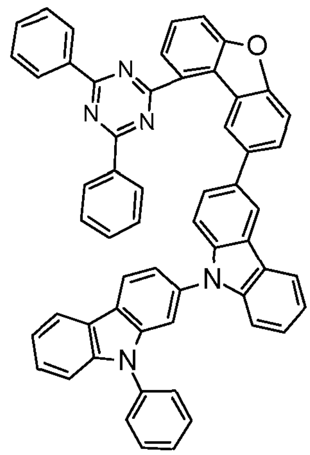 | 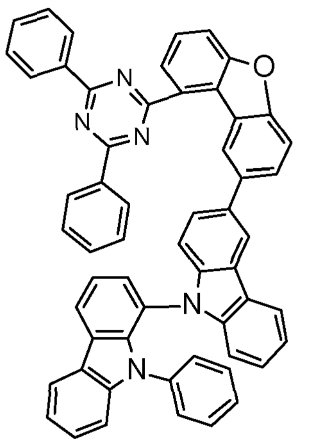 |

| | | |
|---|---|---|
| 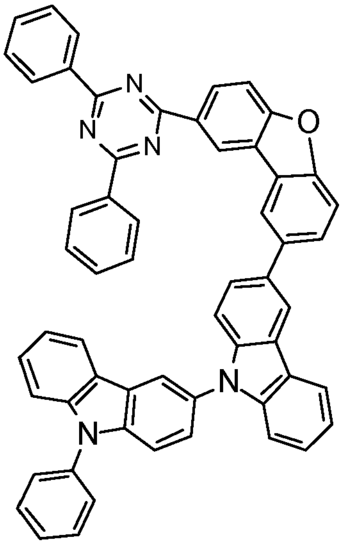 | 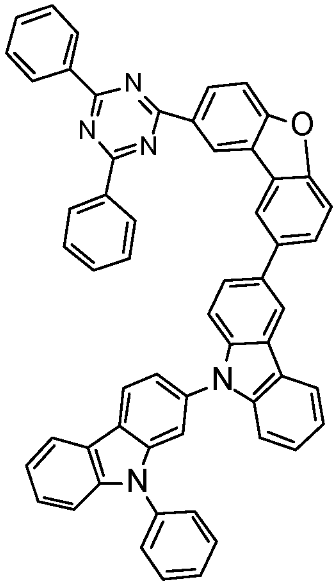 | 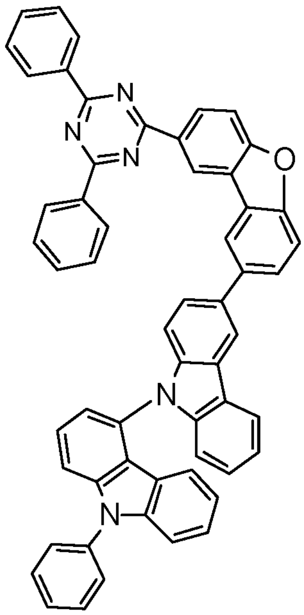 |
| 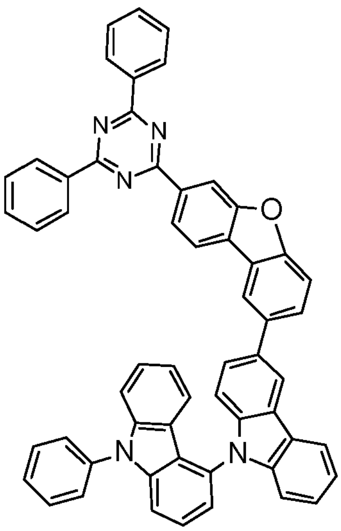 | 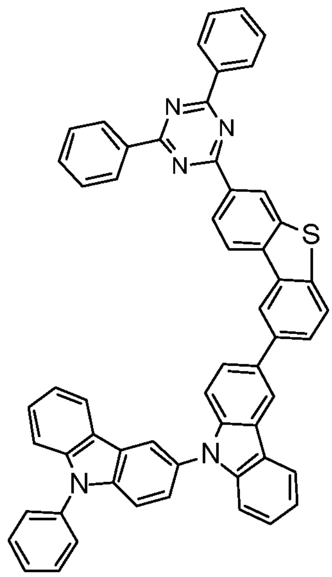 | 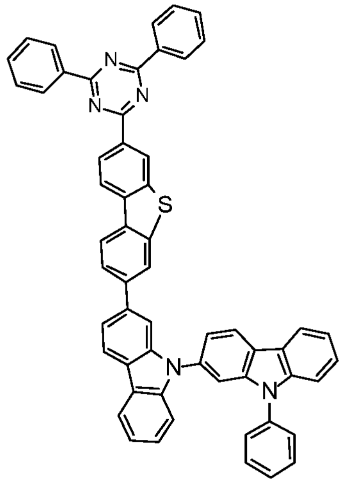 |

| | | |
|---|---|---|
| 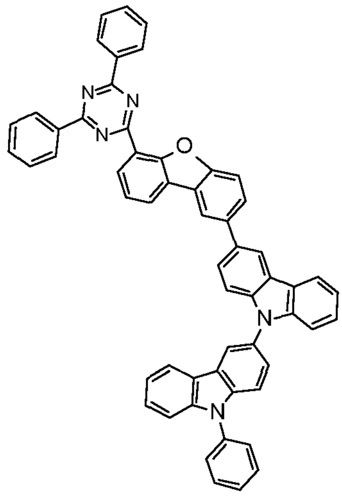 | 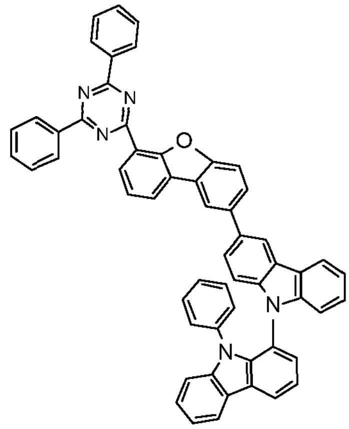 | 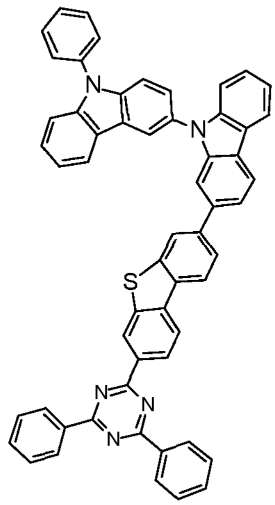 |
| 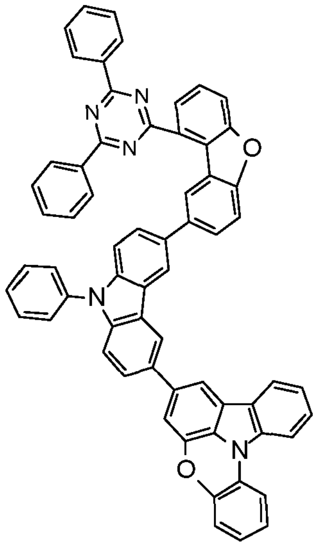 | 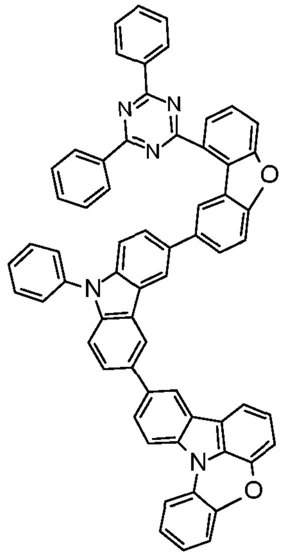 | 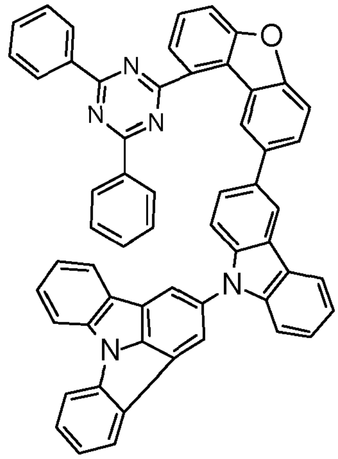 |
| 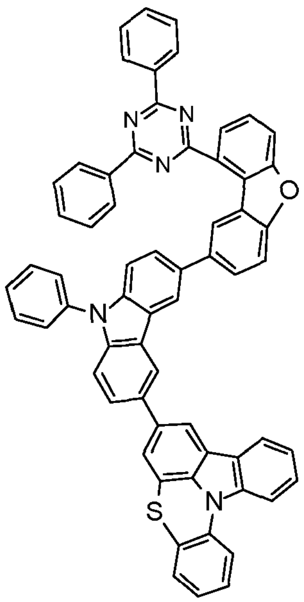 | 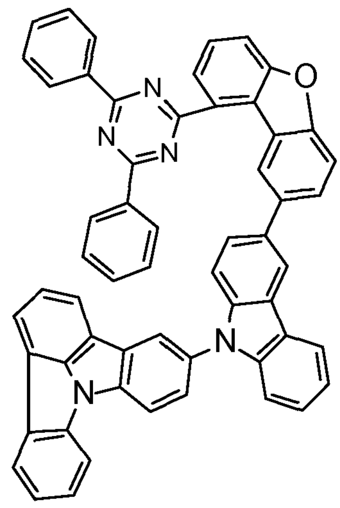 | 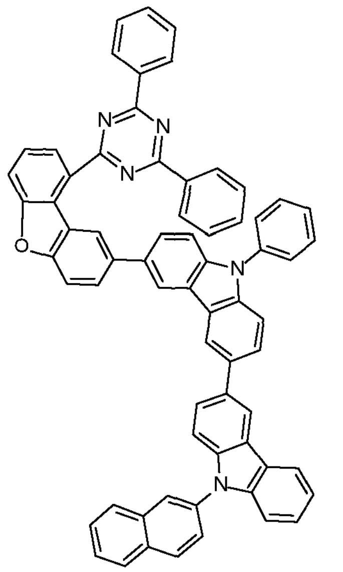 |

Besonders bevorzugte Beispiele für Verbindungen der Formeln (1a), (1b), (1c), (1d), (1e), (1f), (1g), (1h), (1i)

oder (1j), die erfindungsgemäß ausgewählt werden, sind die vorstehend genannten Verbindungen 1 bis 8 der Tabelle 1.

Die Herstellung der Verbindungen der Formeln (1a), (1b), (1c), (1d), (1e), (1f), (1g), (1h), (1i) oder (1j) sowie der Verbindungen 1 bis 8 ist dem Fachmann bekannt. Die erfindungsgemäßen Verbindungen können nach dem Fachmann bekannten Syntheseschritten, wie z. B. Bromierung, Suzuki-Kupplung, Ullmann-Kupplung, Hartwig-Buchwald-Kupplung, etc., dargestellt werden. Ein geeignetes Syntheseverfahren ist allgemein im folgenden Schema 1 dargestellt.

Die Herstellung der Verbindungen der Formeln (1a), (1c), (1d), (1g) oder (1h) kann nach folgendem Schema 1 erfolgen, wobei Y, R, p, q, Ar, $Ar_1$ und $Ar_2$ eine der zuvor angegebenen Bedeutungen haben.

Schema 1:

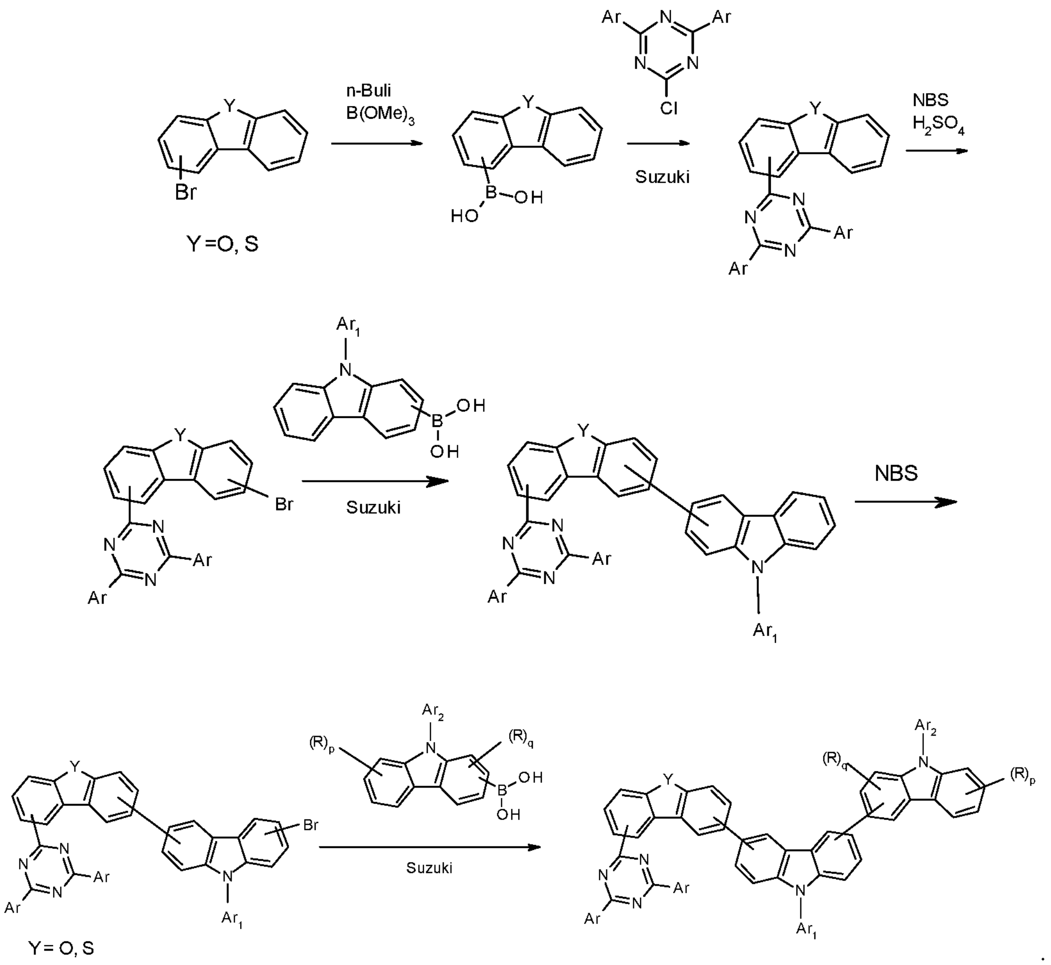


Die Herstellung der Verbindungen der Formeln (1b), (1e), (1f), (1i) oder (1j) kann nach folgendem Schema 2 erfolgen, wobei Y, R, r, s, Ar und $Ar_1$ eine der zuvor angegebenen Bedeutungen haben.

Schema 2:

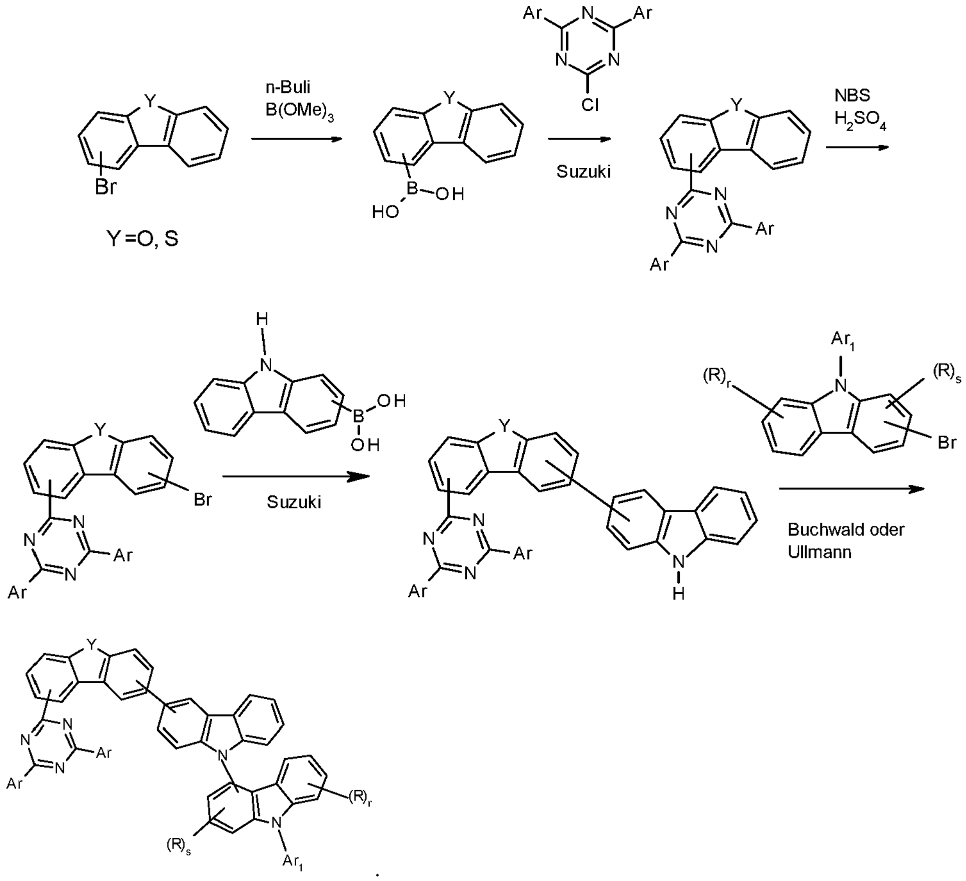


Triphenylen-Derivate der Formel (2):
In einer Ausführungsform der Erfindung werden Verbindungen der Formel (2) ausgewählt, wie zuvor beschrieben, die mit Verbindungen der Formeln (1a), (1b), (1c), (1d), (1e), (1f), (1g), (1h), (1i) und (1j), wie zuvor beschrieben oder bevorzugt beschrieben, oder mit den Verbindungen der Tabelle 1, insbesondere den Verbindungen 1 bis 8, in der Zusammensetzung verwendet werden.

$Ar_4$ steht in Verbindungen der Formel (2) bevorzugt für ein heteroaromatisches Ringsystem mit 6 bis 40 Ringatomen, das mit einem oder mehreren Resten $R^1$ substituiert sein kann, wobei $R^1$ jeweils unabhängig voneinander eine zuvor angegebene oder nachfolgend bevorzugt angegebene Bedeutung hat.

Verbindungen der Formel (2), in denen $Ar_4$ jeweils ein besonders bevorzugtes heteroaromatisches Ringsystem bedeutet, werden durch die Formeln (2a), (2b) und (2c) dargestellt,

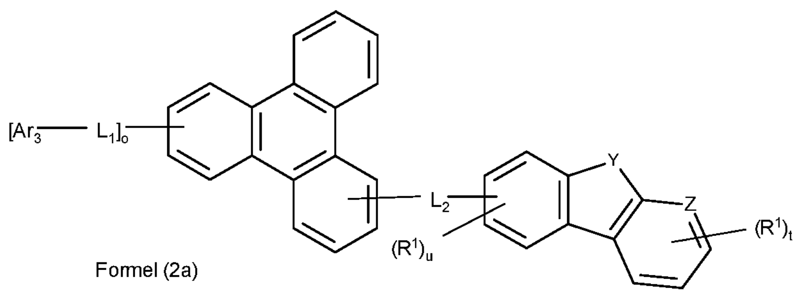

Formel (2a)

,

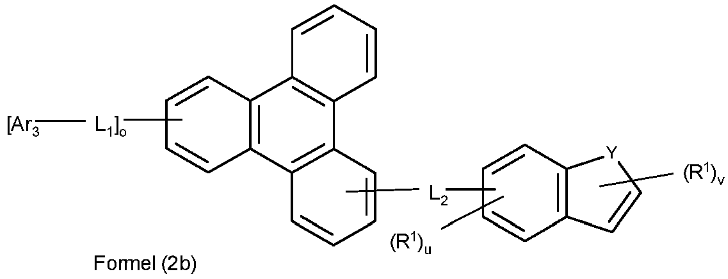

Formel (2b) ,

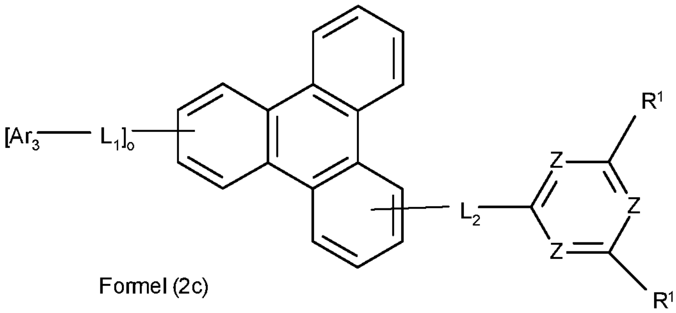

Formel (2c)

wobei die verwendeten Symbole und Indizes $Ar_3$, $L_1$ und $L_2$ eine zuvor genannte oder eine nachfolgend genannte bevorzugte Bedeutung haben und

Z jeweils unabhängig voneinander bei jedem Auftreten N oder $CR^1$ bedeutet, wobei $R^1$ eine zuvor genannte oder nachfolgend bevorzugt genannte Bedeutung hat,
Y in Formel (2a) oder (2b) O oder S bedeutet,
t und u jeweils unabhängig voneinander 0, 1, 2 oder 3 bedeuten und v jeweils unabhängig voneinander 0, 1 oder 2 bedeutet.

Ein weiterer Gegenstand der Erfindung ist demzufolge eine Zusammensetzung, wie zuvor beschrieben, wobei die Verbindung der Formel (2) der Verbindung der Formel (2a), (2b) oder (2c) entspricht.
In Verbindungen der Formeln (2a) und (2b) ist Y bevorzugt S.
In Verbindungen der Formel (2a) ist Z bevorzugt $CR^1$, wobei $R^1$ eine zuvor genannte oder nachfolgend bevorzugt genannte Bedeutung hat.
In Verbindungen der Formel (2c) steht Z mindestens ein Mal bevorzugt für N oder mindestens zwei Mal bevorzugt für N oder drei Mal für N. Besonders bevorzugt ist Z jeweils N.
In Verbindungen der Formel (2a) steht t bevorzugt für 0, 1 oder 2.
In Verbindungen der Formel (2a) steht u bevorzugt für 0 oder 1.
In Verbindungen der Formel (2b) steht v bevorzugt für 2.
In Verbindungen der Formel (2b) steht u bevorzugt für 0.
Die Linker $L_1$ und $L_2$ sind bei jedem Auftreten in den Formeln (2), (2a), (2b) oder (2c) gleich oder verschieden eine Einfachbindung oder ein aromatisches oder heteroaromatisches Ringsystem mit 5 bis 30 Ringatomen, das durch einen oder mehrere Reste $R^3$ substituiert sein kann.
In einer bevorzugten Ausführungsform der Verbindungen der Formeln (2), (2a), (2b) oder (2c) sind $L_1$ und $L_2$ jeweils unabhängig voneinander eine Einfachbindung oder ein aromatisches oder heteroaromatisches Ringsystem ausgewählt aus L-1 bis L-16,

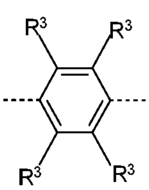
L-1

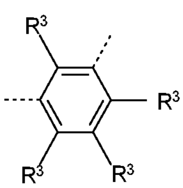
L-2

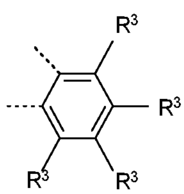
L-3

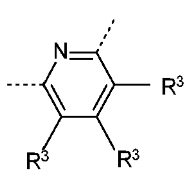
L-4

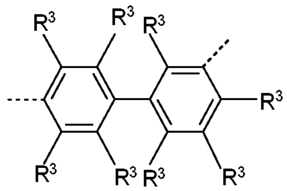
L-5

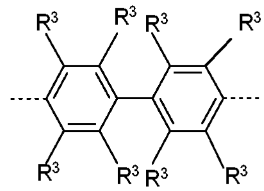
L-6

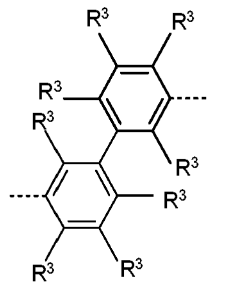
L-7

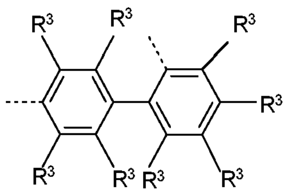
L-8

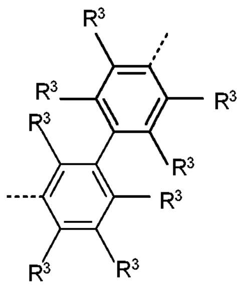
L-9

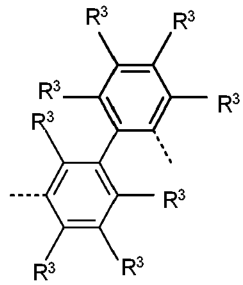
L-10

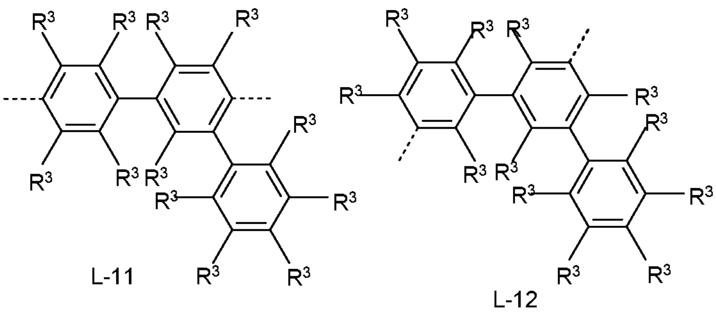
L-11

L-12

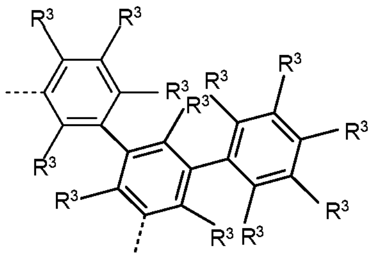
L-13

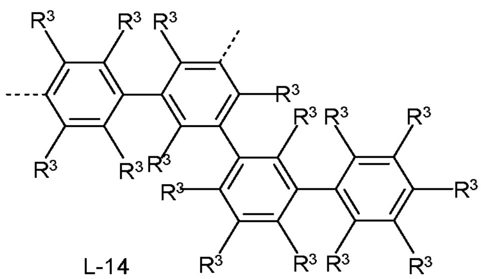
L-14

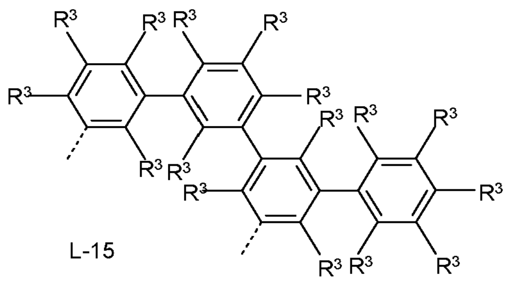
L-15

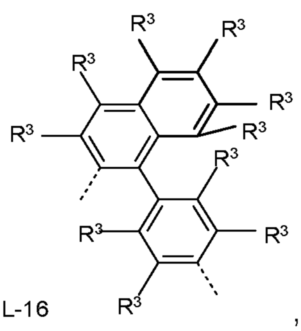
L-16

,

wobei $R^3$ bei jedem Auftreten gleich oder verschieden eine Bedeutung hat, wie zuvor beschrieben oder nachfolgend

bevorzugt beschrieben und die gestrichelte Bindung die Bindung an das entsprechende C-Atom darstellt.

In einer bevorzugten Ausführungsform der Verbindungen der Formeln (2), (2a), (2b) oder (2c) ist $L_1$ bei jedem Auftreten bevorzugt eine Einfachbindung.

In einer bevorzugten Ausführungsform der Verbindungen der Formeln (2), (2a), (2b) oder (2c) ist $L_2$ bei jedem Auftreten bevorzugt eine Einfachbindung oder ein Linker ausgewählt aus L-2, L-3, L-4, L-5, L-7 oder L-13.

$R^3$ in den Linkern L-1 bis L-16 ist bei jedem Auftreten unabhängig voneinander bevorzugt H oder Phenyl, besonders bevorzugt H.

Ein weiterer Gegenstand der Erfindung ist demzufolge eine Zusammensetzung, wie zuvor beschrieben, wobei die Verbindung der Formel (2) der Verbindung der Formel (2a), (2b) oder (2c) entspricht und $L_1$ bei jedem Auftreten eine Einfachbindung bedeutet.

In Verbindungen der Formeln (2), (2a), (2b) oder (2c) ist o 0 oder 1, bevorzugt 0.

$Ar_3$ bedeutet bei jedem Auftreten ein aromatisches oder heteroaromatisches Ringsystem mit 6 bis 40 Ringatomen, das mit einem oder mehreren Resten $R^1$ substituiert sein kann, wobei $R^1$ bei jedem Auftreten gleich oder verschieden eine Bedeutung hat, wie zuvor beschrieben oder nachfolgend bevorzugt beschrieben.

In Verbindungen der Formeln (2), (2a), (2b) oder (2c) oder bevorzugt beschriebenen Verbindungen der Formeln (2), (2a), (2b) oder (2c), wird $Ar_3$ bei Auftreten bevorzugt aus den aromatischen oder heteroaromatischen Ringsystemen Ar-1 bis Ar-8 ausgewählt,

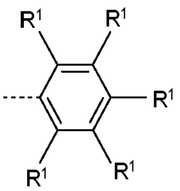
Ar-1

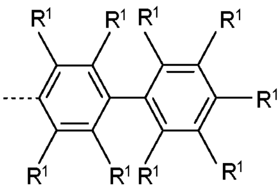
Ar-2

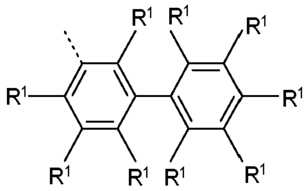
Ar-3

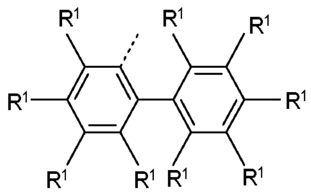
Ar-4

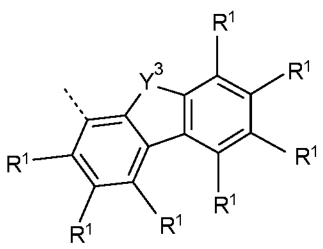
Ar-5

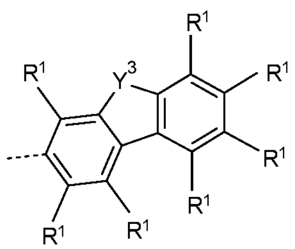
Ar-6

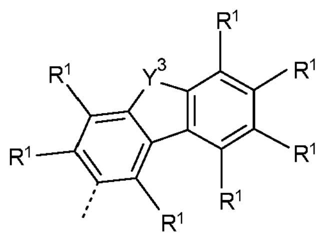
Ar-7

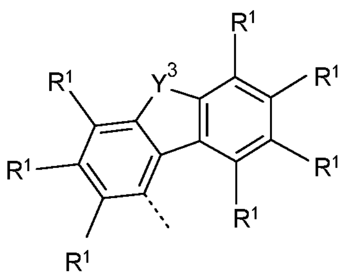
Ar-8 ,

wobei $Y^3$ bei jedem Auftreten gleich oder verschieden O, $NR^{\#}$, S oder $C(R^{\#})_2$ bedeutet, wobei der Rest $R^{\#}$, der an N gebunden ist, ungleich H ist und $R^1$ die zuvor genannte oder eine nachfolgend bevorzugte Bedeutung hat und die gestrichelte Bindung die Bindung an $L_1$ oder das Triphenylen darstellt.

Der Rest $R^{\#}$ ist bei jedem Auftreten gleich oder verschieden H, D, F, Cl, Br, I, CN, $NO_2$, $N(Ar)_2$, $N(R^2)_2$, C(=O)Ar, $C(=O)R^2$, $P(=O)(Ar)_2$, $P(Ar)_2$, $B(Ar)_2$, $Si(Ar)_3$, $Si(R^2)_3$, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine

Alkenylgruppe mit 2 bis 20 C-Atomen, die jeweils mit einem oder mehreren Resten $R^2$ substituiert sein kann, wobei eine oder mehrere nicht-benachbarte $CH_2$-Gruppen durch $R^2C=CR^2$, $Si(R^2)_2$, C=O, C=S, $C=NR^2$, $P(=O)(R^2)$, SO, $SO_2$, $NR^2$, O, S oder $CONR^2$ ersetzt sein können und wobei ein oder mehrere H-Atome durch D, F, Cl, Br, I, CN oder $NO_2$ ersetzt sein können, ein aromatisches oder heteroaromatisches Ringsystem mit 5 bis 40 Ringatomen, das jeweils mit einem oder mehreren Resten $R^2$ substituiert sein kann, eine Aryloxy- oder Heteroaryloxygruppe mit 5 bis 40 Ringatomen, die mit einem oder mehreren Resten $R^2$ substituiert sein kann, oder eine Aralkyl- oder Heteroaralkylgruppe mit 5 bis 40 Ringatomen, die mit einem oder mehreren Resten $R^2$ substituiert sein kann; dabei können optional zwei Substituenten $R^\#$, die an dasselbe Kohlenstoffatom oder an benachbarte Kohlenstoffatome gebunden sind, ein monocyclisches oder polycyclisches, aliphatisches, aromatisches oder heteroaromatisches Ringsystem bilden, das mit einem oder mehreren Resten $R^2$ substituiert sein kann.

$Y^3$ ist bevorzugt O, S oder $C(CH_3)_2$. $Y^3$ ist ganz besonders bevorzugt S.

In den Strukturen Ar-1 bis Ar-8 ist der Substituent $R^1$ bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann; dabei können zwei oder mehr benachbarte Substituenten $R^1$ miteinander ein mono- oder polycyclisches, aliphatisches Ringsystem bilden. In den Strukturen Ar-1 bis Ar-8 ist der Substituent $R^1$ bevorzugt bei jedem Auftreten gleich oder verschieden ausgewählt aus der Gruppe bestehend aus H, F, CN, einem aliphatischen Kohlenwasserstoffrest mit 1 bis 10 C-Atomen oder einem aromatischen oder heteroaromatischen Ringsystem mit 5 bis 30 Ringatomen. In den Strukturen Ar-1 bis Ar-8 ist der Substituent $R^1$ bevorzugt bei jedem Auftreten H.

In Verbindungen der Formeln (2), (2a), (2b) oder (2c) oder bevorzugt beschriebenen Verbindungen der Formeln (2), (2a), (2b) oder (2c) wird $Ar_3$ bei seinem Auftreten besonders bevorzugt aus den aromatischen oder heteroaromatischen Ringsystemen Ar-1, Ar-5, Ar-6, Ar-7 oder Ar-8 ausgewählt, wobei die Substituenten $R^1$ und $Y^3$ eine zuvor genannte oder als bevorzugt beschriebene Bedeutung haben.

Wenn in Verbindungen der Formeln (2), (2a) oder (2b) t, u oder v größer 0 sind, so wird der Substituent $R^1$ bei jedem Auftreten gleich oder verschieden bevorzugt ausgewählt aus der Gruppe bestehend aus D, F, einer Alkylgruppe mit 1 bis 40 C-Atomen oder einem aromatischen oder heteroaromatischen Ringsystem mit 5 bis 40 Ringatomen, das mit einem oder mehreren Resten $R^2$ substituiert sein kann. Das aromatische oder heteroaromatische Ringsystem mit 5 bis 40 Ringatomen in diesem $R^1$ ist bevorzugt abgeleitet von Benzol, Dibenzofuran, Dibenzothiophen, 9-Phenyl-Carbazol, Biphenyl, Triphenylen und Triazin, das mit einem oder mehreren Resten $R^2$ substituiert sein kann. Besonders bevorzugte Substituenten $R^1$ in $[R^1]t$, wenn t größer 0 ist, sind jeweils unabhängig voneinander Phenyl, Triphenylen und Triazin, die mit einem oder mehreren Substituenten $R^2$ substituiert sein können. $R^2$ ist bei seinem Auftreten in diesem Fall jeweils unabhängig voneinander bevorzugt Phenyl oder Triphenylen.

Besonders bevorzugte Substituenten $R^1$ in $[R^1]_u$, wenn u größer 0 ist, sind jeweils unabhängig voneinander Phenyl, das mit einem oder mehreren Substituenten $R^2$ substituiert sein kann.

Besonders bevorzugte Substituenten $R^1$ in $[R^1]_v$, wenn v größer 0 ist, sind jeweils unabhängig voneinander Phenyl, das mit einem oder mehreren Substituenten $R^2$ substituiert sein kann.

In Verbindungen der Formel (2c) sind die Substituenten $R^1$ bevorzugt ausgewählt aus Phenyl, Biphenyl, Dibenzothiophen, Pyrimidin oder Pyridin, die mit einem oder mehreren Substituenten $R^2$ substituiert sein können, wobei zwei Substituenten $R^1$ zusammen auch einen aromatischen Ring bilden können, der mit einem oder mehreren Substituenten $R^2$ substituiert sein kann. $R^2$ ist bei seinem Auftreten in diesem Fall jeweils unabhängig voneinander bevorzugt Phenyl.

In Verbindungen der Formel (2a) ist es ebenfalls bevorzugt, wenn zwei Substituenten $R^1$, die bevorzugt an benachbarten Kohlenstoffatomen gebunden sind, ein monocyclisches oder polycyclisches, aliphatisches, aromatisches oder heteroaromatisches Ringsystem bilden, bevorzugt ein aromatisches oder heteroaromatisches Ringsystem.

Der Substituent $R^2$ ist bei jedem Auftreten gleich oder verschieden bevorzugt ausgewählt aus der Gruppe bestehend aus D, F, Cl, Br, I, CN, $NO_2$, $N(Ar)_2$, $NH_2$, $N(R^3)_2$, C(=O)Ar, C(=O)H, $C(=O)R^3$, $P(=O)(Ar)_2$, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten $R^3$ substituiert sein kann, einem aromatischen oder heteroaromatischen Ringsystem mit 5 bis 60 Ringatomen, das jeweils mit einem oder mehreren Resten $R^3$ substituiert sein kann, einer Aryloxy- oder Heteroaryloxygruppe mit 5 bis 60 Ringatomen, die mit einem oder mehreren Resten $R^3$ substituiert sein kann. Der Substituent $R^2$ ist bei seinem Auftreten besonders bevorzugt ein aromatisches oder heteroaromatisches Ringsystem, wie zuvor beschrieben, bevorzugt ausgewählt und abgeleitet aus der Gruppe Carbazol, 9-Phenyl-carbazol, Dibenzofuran, Dibenzothiophen, Fluoren, Terphenyl oder Spirobifluoren.

Im Fall der Substitution eines der Substituenten $R^2$, wie zuvor beschrieben, mit einem Substituenten $R^3$, gelten die Bedeutungen von $R^3$ wie zuvor beschrieben bzw. bevorzugt beschrieben.

$R^3$ ist vorzugsweise bei jedem Auftreten gleich oder verschieden H oder ein aromatisches oder heteroaromatisches Ringsystem mit 5 bis 30 Ringatomen. $R^3$ ist besonders bevorzugt H oder Phenyl.

Beispiele für geeignete Verbindungen der Formeln (2), (2a), (2b) oder (2c), die erfindungsgemäß ausgewählt werden, sind die nachstehend genannten Strukturen der Tabelle 2.

Tabelle 2:

| | | |
|---|---|---|
| 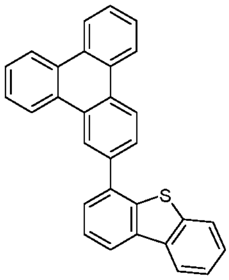 **9** | 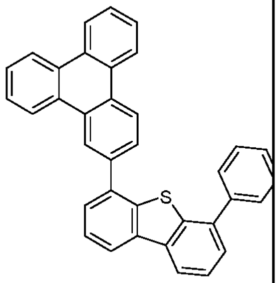 **10** | 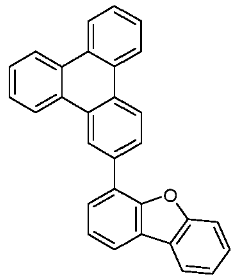 |
| 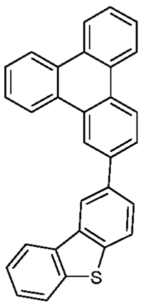 | 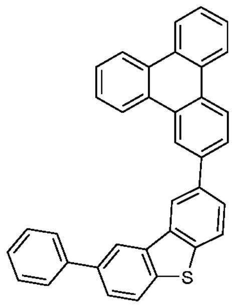 | 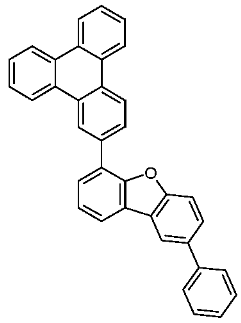 |
| 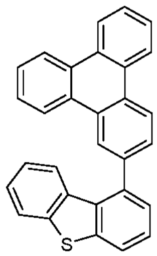 | 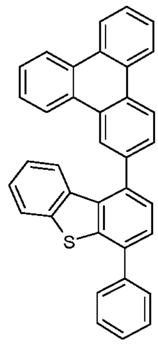 | 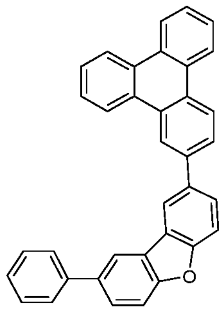 |
| 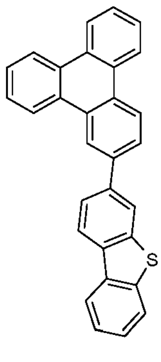 | 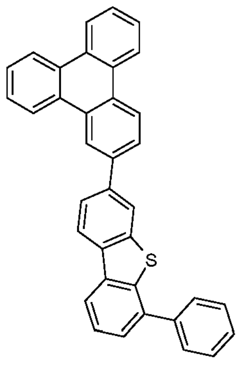 | 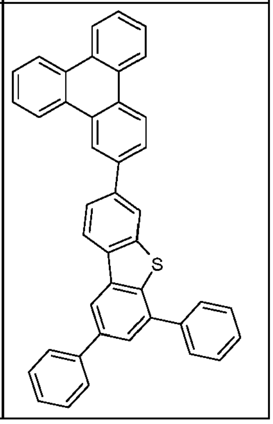 |

| | | |
|---|---|---|
| 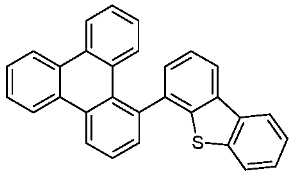 | 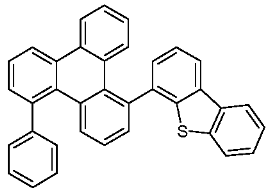 | 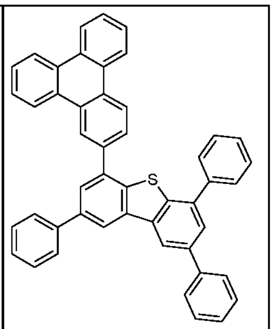 |
| 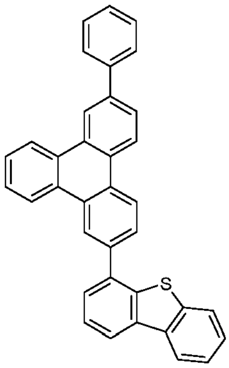 | 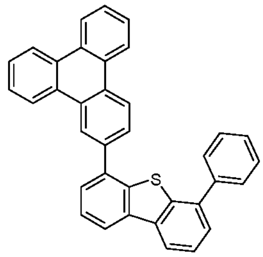 | 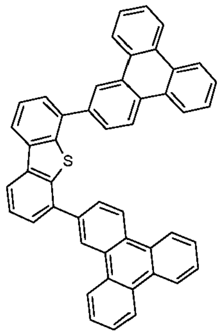 |
| 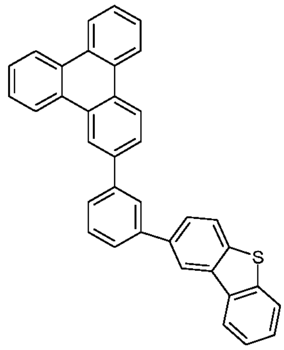 11 | 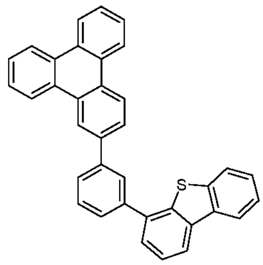 12 | 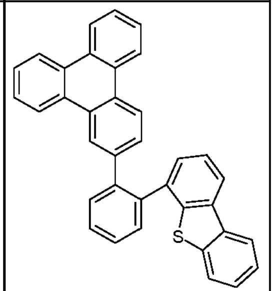 |
| 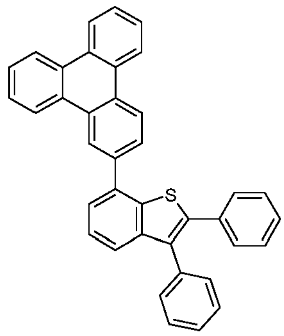 | 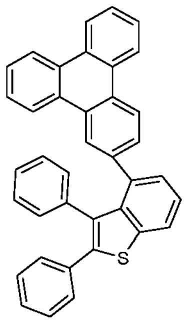 | 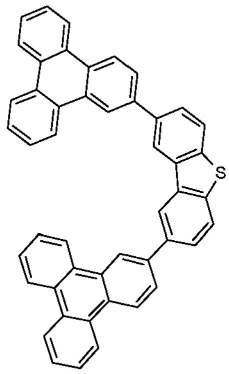 |

| | | |
|---|---|---|
| 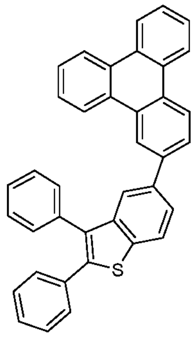 | 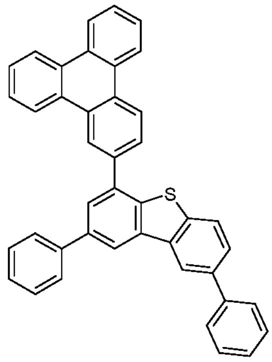 | 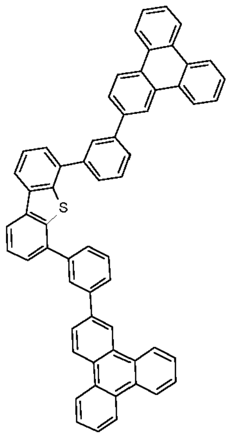 |
| 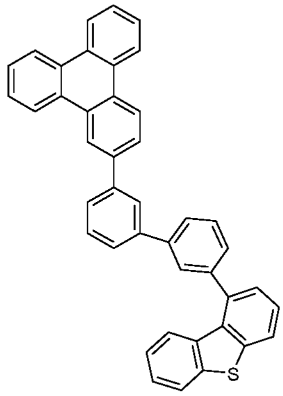 | 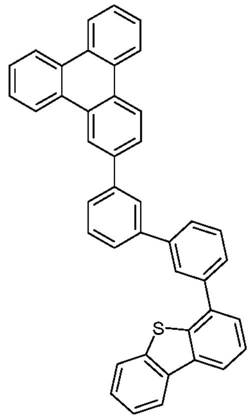<br>**13** | 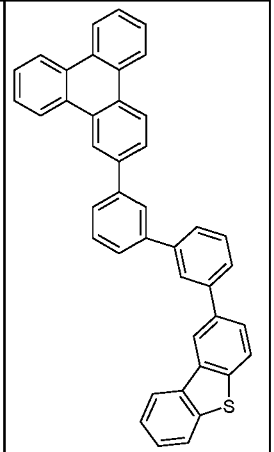 |
| 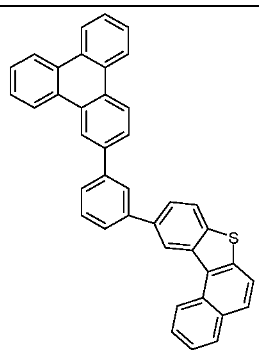 | 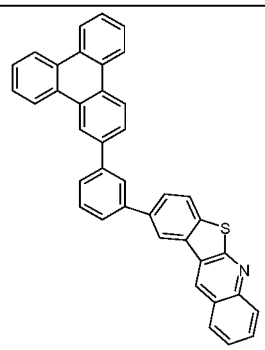 | 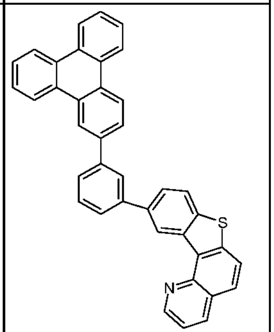 |
| 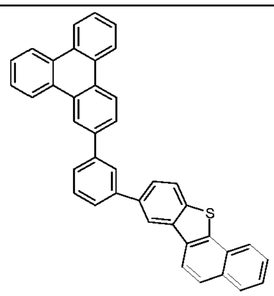 | 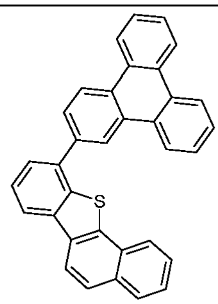 | 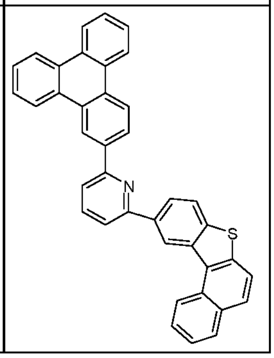 |

| | | |
|---|---|---|
| 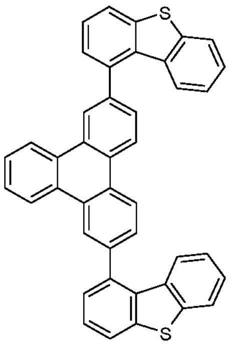 | 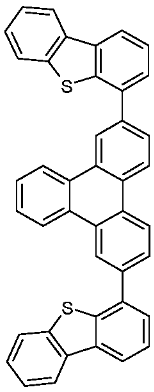<br>14 | 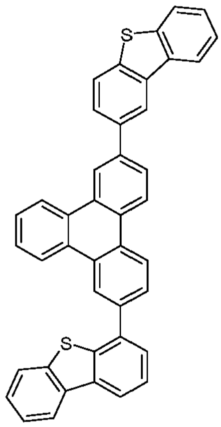 |
| 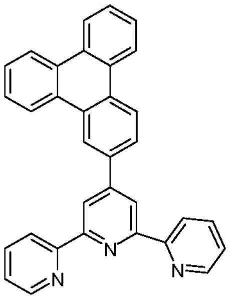 | 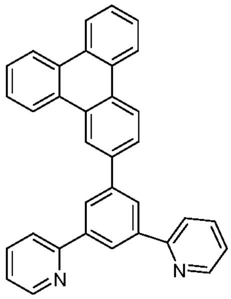 | 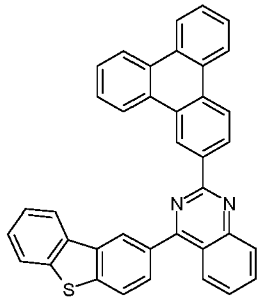 |
| 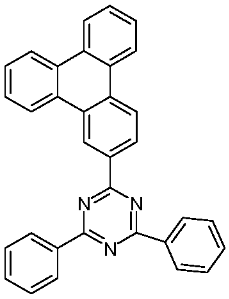<br>15 | 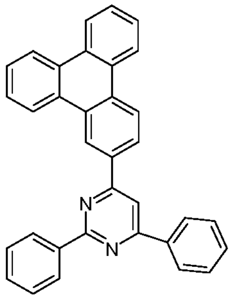 | 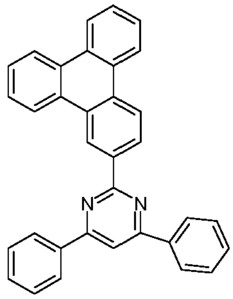 |
| 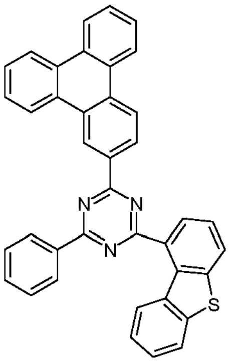 | 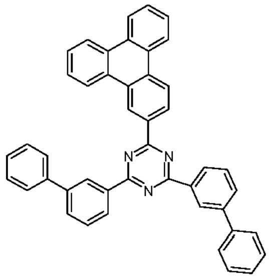 | 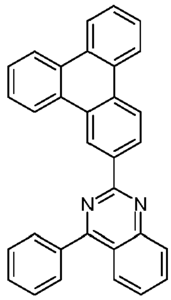 |

| | | |
|---|---|---|
| 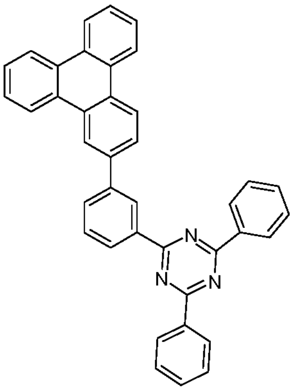 16 | 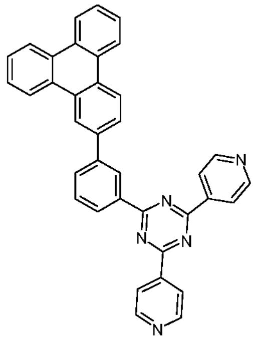 | 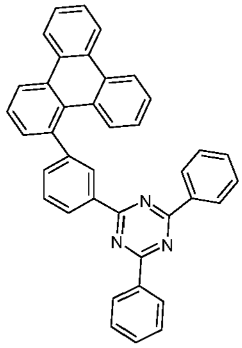 |
| 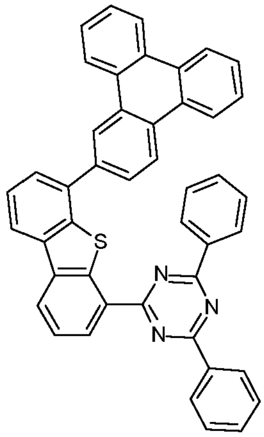 | 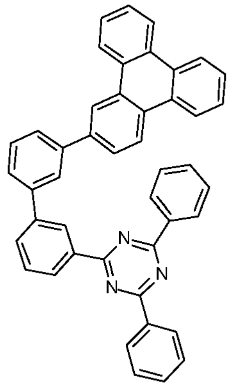 17 | 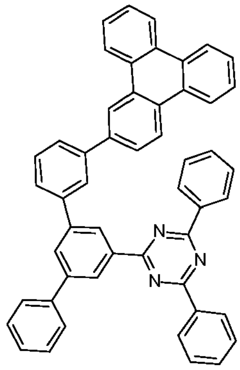 |
| 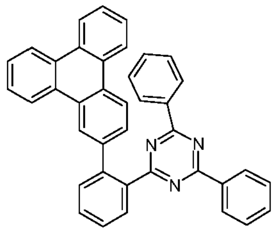 | 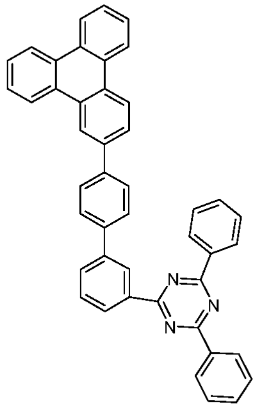 | 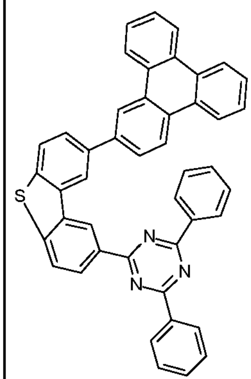 |

| | | |
|---|---|---|
| 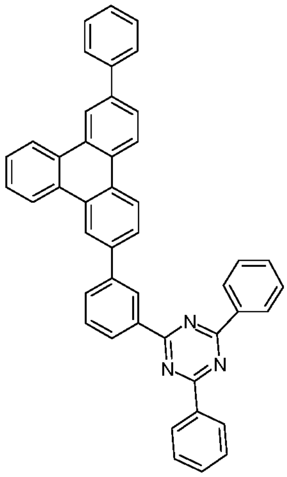 | 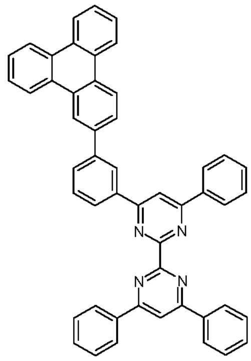 | 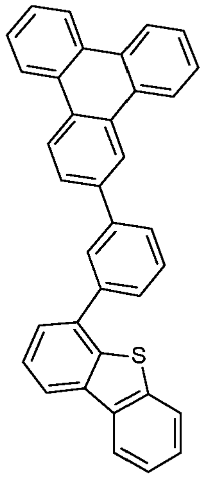 |
| 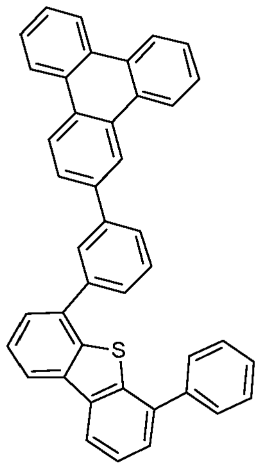 | 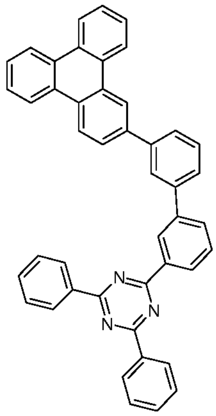 | 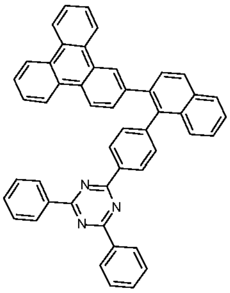 |

Besonders geeignete Beispiele für Verbindungen der Formeln (2), (2a), (2b) oder (2c), die erfindungsgemäß ausgewählt werden, sind die Verbindungen **9** bis **17,** wie zuvor beschrieben.

Die Herstellung der Verbindungen der Formel (2) oder der bevorzugten Verbindungen der Formeln (2a), (2b) und (2c) sowie der Verbindungen der Tabelle 2 ist dem Fachmann bekannt. Die Verbindungen können nach dem Fachmann bekannten Syntheseschritten, wie z. B. Bromierung, Suzuki-Kupplung, dargestellt werden. Die Herstellung der Triphenylen-Derivate ist beispielsweise auch in WO 2009/021126 oder US 2015340618 beschrieben.

Die Herstellung der Verbindungen der Formel (2) oder der bevorzugten Verbindungen der Formel (2a) kann beispielsweise nach Schema 3 erfolgen. Die Reaktionsbedingungen sind dem Fachmann bekannt. Die Bromierung des Triphenylens ist beispielsweise in US20060280965 beschrieben. Die Suzuki-Kupplung ist beispielsweise in WO 2009/021126 beschrieben.

Schema 3:

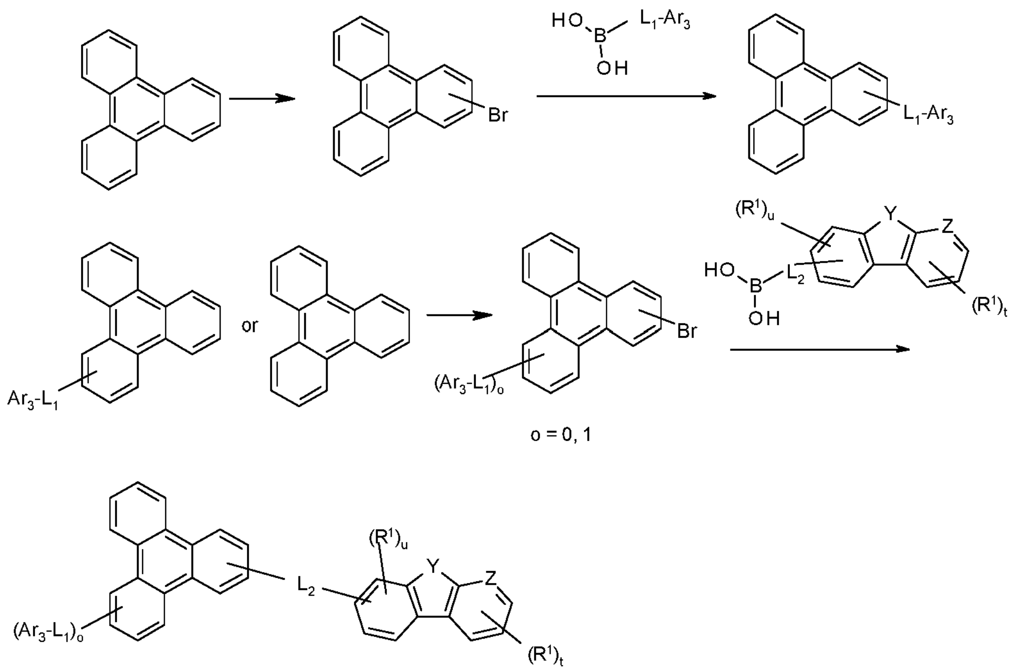


Die Herstellung der Verbindungen der Formel (2) oder der bevorzugten Verbindungen der Formel (2b) kann beispielsweise nach Schema 4 erfolgen. Die Reaktionsbedingungen sind dem Fachmann bekannt. Die Bromierung des Triphenylens ist beispielsweise in US20060280965 beschrieben. Die Suzuki-Kupplung ist beispielsweise in WO 2009/021126 beschrieben.

Schema 4:

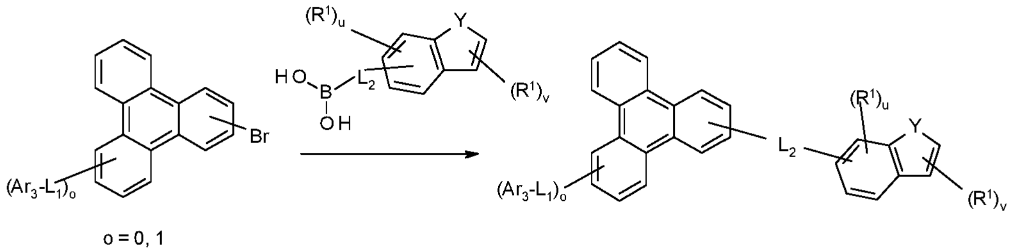


Die Herstellung der Verbindungen der Formel (2) oder der bevorzugten Verbindungen der Formel (2c) kann beispielsweise nach Schema 5 erfolgen. Die Reaktionsbedingungen sind dem Fachmann bekannt. Die Bromierung des Triphenylens ist beispielsweise in US20060280965 beschrieben. Die Suzuki-Kupplung ist beispielsweise in WO 2009/021126 beschrieben.

Schema 5:

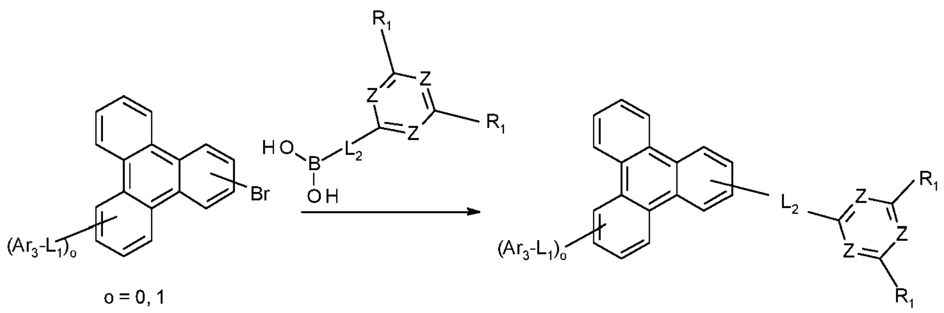

Alle in Schema 3 bis 5 verwendeten Indizes und Symbole haben die gleiche Bedeutung, wie zuvor beschrieben oder als bevorzugt beschrieben. Weitere Details zu den Synthesen bzw. weitere Literaturzitate sind im Ausführungsteil beschrieben.

Die vorstehend genannten Hostmaterialien der Formeln (1a) bis (1j) sowie deren bevorzugt beschriebene Ausführungsformen oder die Verbindungen der Tabelle 1 können erfindungsgemäß beliebig mit den genannten Hostmaterialien der Formeln (2), (2a), (2b) und (2c) sowie deren bevorzugt beschriebenen Ausführungsformen oder den Verbindungen der Tabelle 2 kombiniert werden.

Besonders bevorzugte Mischungen der Hostmaterialien der Formeln (1a) oder (1b) mit den Hostmaterialien der Formel (2) für die erfindungsgemäße Zusammensetzung erhält man durch Kombination der Verbindungen **1** bis **8** der Tabelle 1 mit den Verbindungen der Tabelle 2.

Ganz besonders bevorzugte Mischungen M1 bis M72 der Hostmaterialien der Formeln (1a) oder (1b) mit den Hostmaterialien der Formel (2) erhält man durch Kombination der Verbindungen **1** bis **8,** beschrieben in der Tabelle 1, mit den Verbindungen **9** bis **17,** beschrieben in der Tabelle 2, wie im Folgenden in Tabelle 3 gezeigt.

Tabelle 3:

| | | | | | |
|---|---|---|---|---|---|
| M1 | **1** | **9** | M2 | **1** | **10** |
| M3 | **1** | **11** | M4 | **1** | **12** |
| M5 | **1** | **13** | M6 | **1** | **14** |
| M7 | **1** | **15** | M8 | **1** | **16** |
| M9 | **1** | **17** | M10 | **2** | **9** |
| M11 | **2** | **10** | M12 | **2** | **11** |
| M13 | **2** | **12** | M14 | **2** | **13** |
| M15 | **2** | **14** | M16 | **2** | **15** |
| M17 | **2** | **16** | M18 | **2** | **17** |
| M19 | **3** | **9** | M20 | **3** | **10** |
| M21 | **3** | **11** | M22 | **3** | **12** |
| M23 | **3** | **13** | M24 | **3** | **14** |
| M25 | **3** | **15** | M26 | **3** | **16** |
| M27 | **3** | **17** | M28 | **4** | **9** |
| M29 | **4** | **10** | M30 | **4** | **11** |
| M31 | **4** | **12** | M32 | **4** | **13** |
| M33 | **4** | **14** | M34 | **4** | **15** |
| M35 | **4** | **16** | M36 | **4** | **17** |
| M37 | **5** | **9** | M38 | **5** | **10** |
| M39 | **5** | **11** | M40 | **5** | **12** |
| M41 | **5** | **13** | M42 | **5** | **14** |
| M43 | **5** | **15** | M44 | **5** | **16** |
| M45 | **5** | **17** | M46 | **6** | **9** |
| M47 | **6** | **10** | M48 | **6** | **11** |
| M49 | **6** | **12** | M50 | **6** | **13** |
| M51 | **6** | **14** | M52 | **6** | **15** |
| M53 | **6** | **16** | M54 | **6** | **17** |
| M55 | **7** | **9** | M56 | **7** | **10** |
| M57 | **7** | **11** | M58 | **7** | **12** |

(fortgesetzt)

| | | | | | |
|---|---|---|---|---|---|
| M59 | **7** | **13** | M60 | **7** | **14** |
| M61 | **7** | **15** | M62 | **7** | **16** |
| M63 | **7** | **17** | M64 | **8** | **9** |
| M65 | **8** | **10** | M66 | **8** | **11** |
| M67 | **8** | **12** | M68 | **8** | **13** |
| M69 | **8** | **14** | M70 | **8** | **15** |
| M71 | **8** | **16** | M72 | **8** | **17.** |

Die Konzentration des bipolaren Hosts der Formeln (1a) oder (1b), wie zuvor beschrieben oder bevorzugt beschrieben, in der erfindungsgemäßen Zusammensetzung liegt im Bereich von 5 Gew.-% bis 90 Gew.-%, bevorzugt im Bereich von 10 Gew.-% bis 85 Gew.-%, mehr bevorzugt im Bereich von 20 Gew.-% bis 85 Gew.-%, noch mehr bevorzugt im Bereich von 30 Gew.-% bis 80 Gew.-%, ganz besonders bevorzugt im Bereich von 20 Gew.-% bis 60 Gew.-% und am meisten bevorzugt im Bereich von 30 Gew.-% bis 50 Gew.-%, bezogen auf die gesamte Zusammensetzung.

Die Konzentration des Triphenylenderivats der Formel (2), wie zuvor beschrieben oder als bevorzugt beschrieben, in der Zusammensetzung liegt im Bereich von 10 Gew.-% bis 95 Gew.-%, bevorzugt im Bereich von 15 Gew.-% bis 90 Gew.-%, mehr bevorzugt im Bereich von 15 Gew.-% bis 80 Gew.-%, noch mehr bevorzugt im Bereich von 20 Gew.-% bis 70 Gew.-%, ganz besonders bevorzugt im Bereich von 40 Gew.-% bis 80 Gew.-% und am meisten bevorzugt im Bereich von 50 Gew.-% bis 70 Gew.-%, bezogen auf die gesamte Zusammensetzung.

Die erfindungsgemäße Zusammensetzung kann in einer weiteren bevorzugten Ausführungsform neben mindestens einer Verbindung der Formeln (1a) oder (1b), wie zuvor beschrieben oder als bevorzugt beschrieben, als bipolaren Host, und mindestens einer Verbindung der Formel (2), wie zuvor beschrieben oder als bevorzugt beschrieben, als neutraler Co-Host, noch weitere Verbindungen, insbesondere organische funktionelle Materialien, enthalten. Die Zusammensetzung bildet in dieser Ausführungsform bevorzugt eine organische Schicht in einer elektronischen Vorrichtung, wie nachfolgend beschrieben.

Die vorliegende Erfindung betrifft daher auch eine Zusammensetzung, die neben den vorstehend genannten Materialien der Formeln (1a) oder (1b) und (2) mindestens noch eine weitere Verbindung enthält, die ausgewählt ist aus der Gruppe bestehend aus Lochinjektionsmaterialien, Lochtransportmaterialien, Lochblockier-materialien, *wide-band-gap*-Materialien, fluoreszierenden Emittern, phosphoreszierenden Emittern, Hostmaterialien, Elektronenblockiermaterialien, Elektronentransportmaterialien, Elektroneninjektionsmaterialien, n-Dotanden und p-Dotanden. Es bereitet dem Fachmann dabei keinerlei Schwierigkeiten, diese aus einer Vielzahl ihm bekannter Materialien auszuwählen.

Unter n-Dotanden werden hierin Reduktionsmittel, d.h. Elektronendonatoren verstanden. Bevorzugte Beispiele für n-Dotanden sind $W(hpp)_4$ und weitere elektronenreiche Metallkomplexe gemäß WO 2005/086251 A2, P=N-Verbindungen (z.B. WO 2012/175535 A1, WO 2012/175219 A1), Naphthylencarbodiimide (z.B. WO 2012/168358 A1), Fluorene (z.B. WO 2012/031735 A1), Radikale und Diradikale (z.B. EP 1837926 A1, WO 2007/107306 A1), Pyridine (z.B. EP 2452946 A1, EP 2463927 A1), N-heterocyclische Verbindungen (z.B. WO 2009/000237 A1) und Acridine sowie Phenazine (z.B. US 2007/145355 A1).

Unter p-Dotanden werden hierin Oxidationsmittel, d.h. Elektronenakzeptoren verstanden. Bevorzugte Beispiele für p-Dotanden sind $F_4$-TCNQ, $F_6$-TNAP, NDP-2 (Fa. Novaled), NDP-9 (Fa. Novaled), Chinone (z.B. EP 1538684 A1, WO 2006/081780 A1, WO 2009/003455 A1, WO 2010/097433 A1), Radialene (z.B. EP 1988587 A1, US 2010/102709 A1, EP 2180029 A1, WO 2011/131185 A1, WO 2011134458 A1, US 2012/223296 A1), S-haltige Übergangsmetallkomplexe (z.B. WO 2007/134873 A1, WO 2008/061517 A2, WO 2008/061518 A2, DE 102008051737 A1, WO 2009/089821 A1, US 2010/096600 A1), Bisimidazole (z.B. WO 2008/138580 A1), Phthalocyanine (z.B. WO 2008/058525 A2), Bora-Tetraazapentalene (z.B. WO 2007/115540 A1) Fullerene (z.B. DE 102010046040 A1) und Hauptgruppenhalogenide (z.B. WO 2008/128519 A2).

Unter wide-band-gap-Material wird hierin ein Material im Sinne der Offenbarung von US 7,294,849 verstanden, das durch eine Bandlücke von mindestens 3.5 eV charakterisiert ist, wobei unter Bandlücke der Abstand zwischen HOMO und LUMO-Energie eines Materials verstanden wird.

Es ist bevorzugt, wenn die erfindungsgemäße Zusammensetzung umfassend einen bipolaren Host der Formel (1a) oder (1b) und einen neutralen Co-Host der Formel (2) zusätzlich wenigstens eine lichtemittierende Verbindung bzw. einen Emitter enthält, wobei phosphoreszierende Emitter besonders bevorzugt sind.

Vom Begriff phosphoreszierende Emitter sind typischerweise Verbindungen umfasst, bei denen die Lichtemission durch einen spin-verbotenen Übergang aus einem angeregten Zustand mit höherer Spinmultiplizität, also einem

Spinzustand > 1, erfolgt, beispielsweise durch einen Übergang aus einem Triplett-Zustand oder einem Zustand mit einer noch höheren Spinquantenzahl, beispielsweise einem Quintett-Zustand. Bevorzugt wird hierbei ein Übergang aus einem Triplett-Zustand verstanden.

Als phosphoreszierende Emitter (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten. Im Sinne der vorliegenden Erfindung werden alle lumineszierenden Verbindungen, die die oben genannten Metalle enthalten, als phosphoreszierende Emitter angesehen.

Generell eignen sich alle phosphoreszierenden Komplexe, wie sie gemäß Stand der Technik für phosphoreszierende OLEDs verwendet werden und wie sie dem Fachmann auf dem Gebiet der organischen Elektrolumineszenzvorrichtungen bekannt sind.

Beispiele der oben beschriebenen Emitter können den Anmeldungen WO 2016/015815, WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2015/036074, WO 2015/104045, WO 2015/117718, WO 2016/015815, WO 2016/124304, WO 2017/032439, WO 2015/036074, WO 2015/117718 und WO 2016/015815 entnommen werden.

Bevorzugte Beispiele von phosphoreszierenden Emittern sind in der folgenden Tabelle 4 aufgeführt.

Tabelle 4:

| | | |
|---|---|---|
| 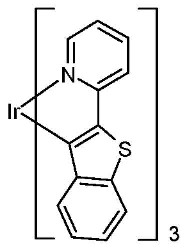 | 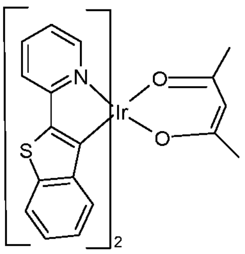 | 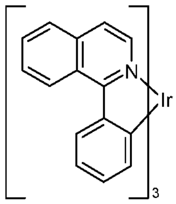 |

| | | |
|---|---|---|
| 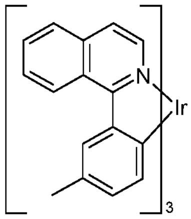 | 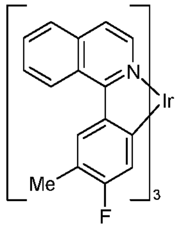 | 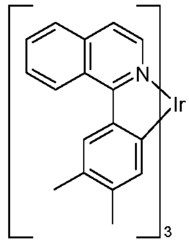 |
| 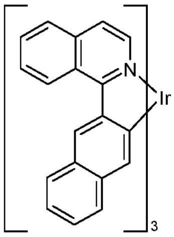 | 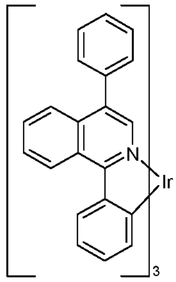 | 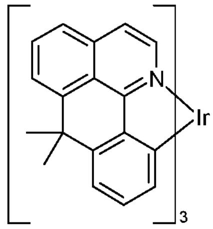 |
| 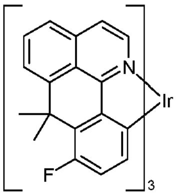 | 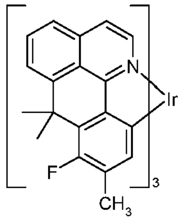 | 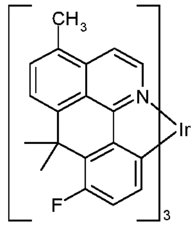 |
| 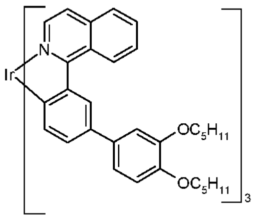 | 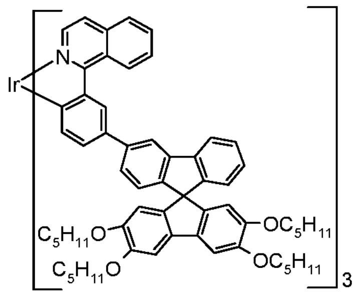 | 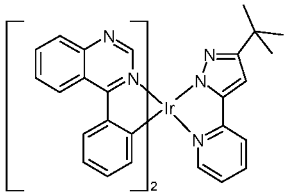 |
| 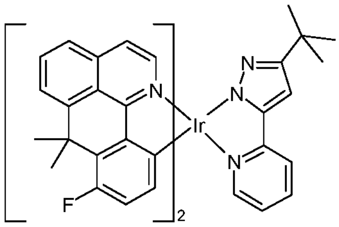 | 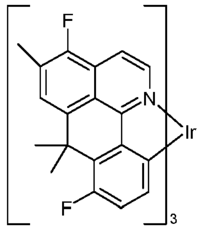 | 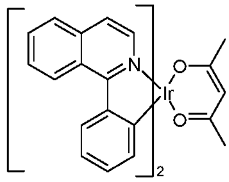 |
| 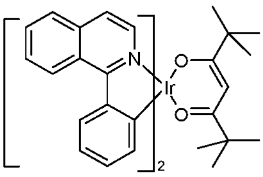 | 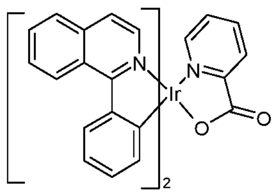 | 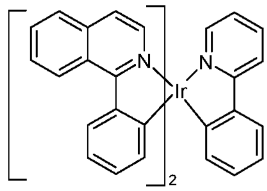 |

| | | |
|---|---|---|
| 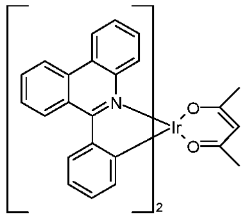 | 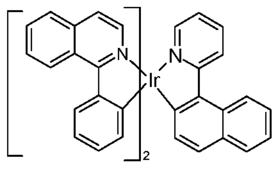 | 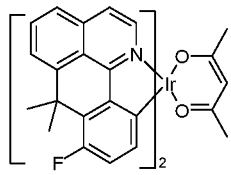 |
| 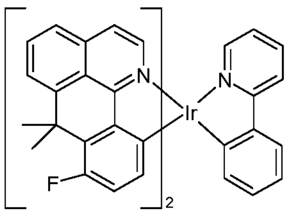 | 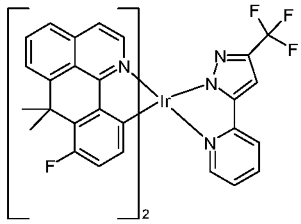 | 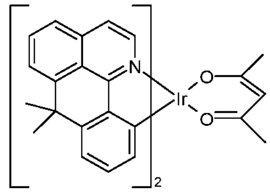 |
| 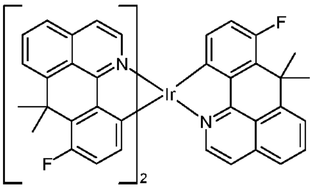 | 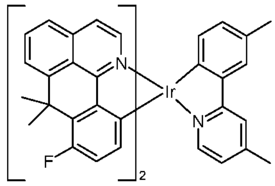 | 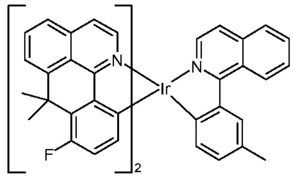 |
| 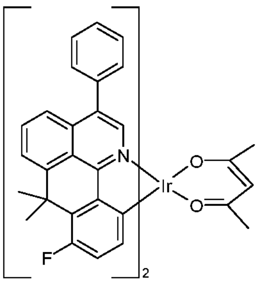 | 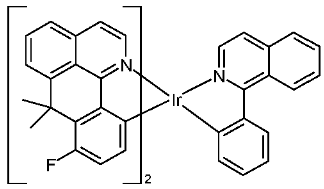 | 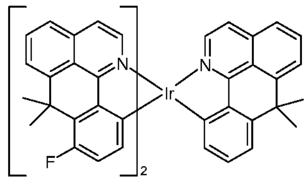 |
| 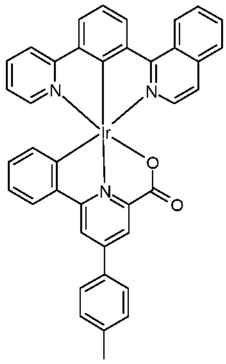 | 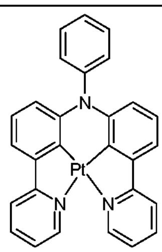 | 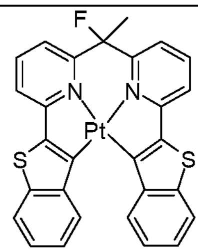 |
| 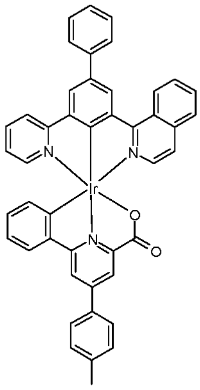 | 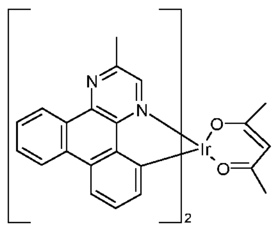 | 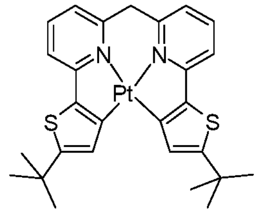 |

| | | |
|---|---|---|
| 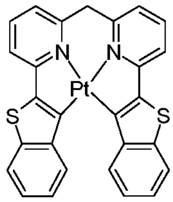 | 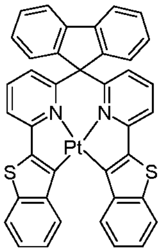 | 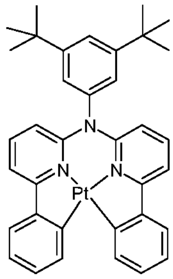 |
| 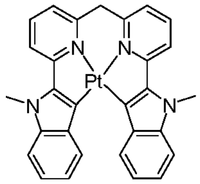 | 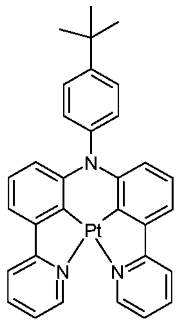 | 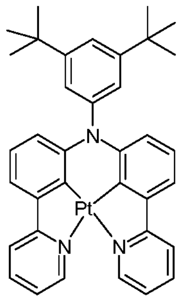 |
| 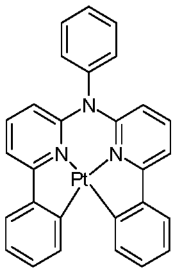 | 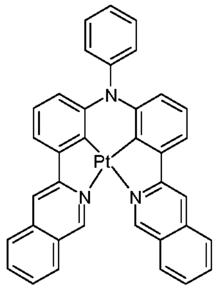 | |
| 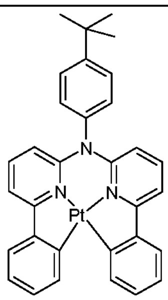 | 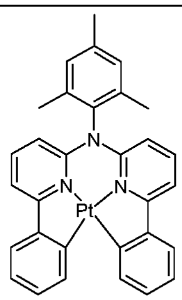 | 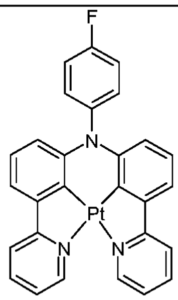 |
| 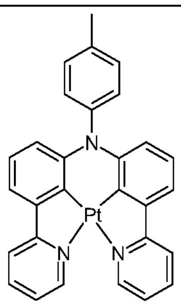 | 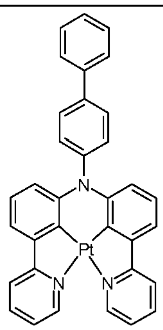 | 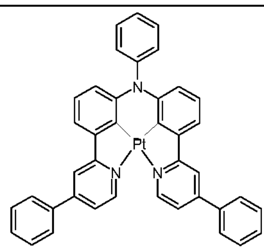 |

| | | |
|---|---|---|
| 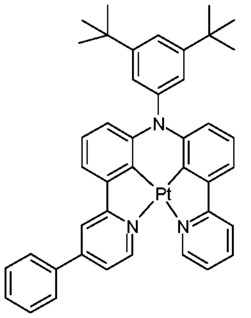 | 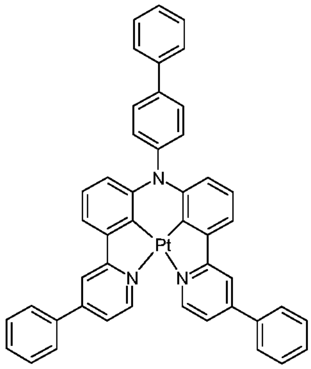 | 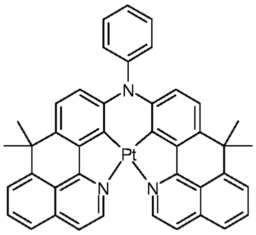 |
| 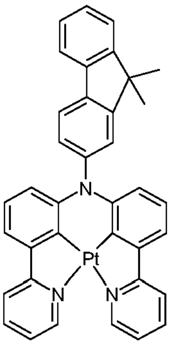 | 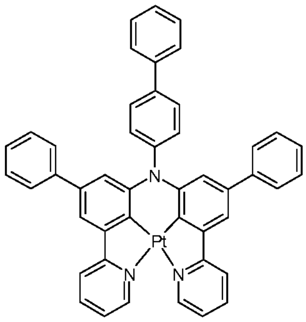 | 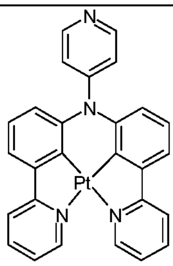 |
| 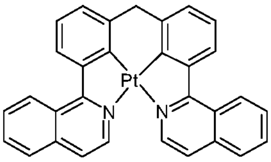 | 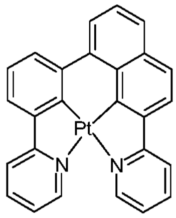 | 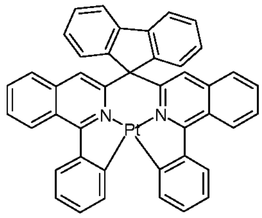 |
| 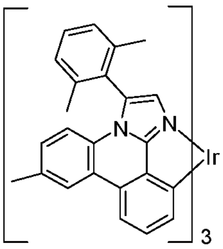 | 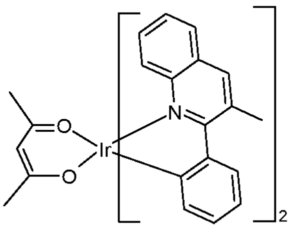 | 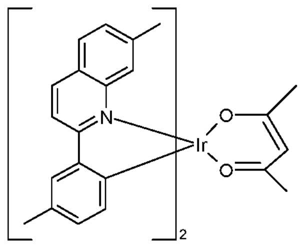 |
| 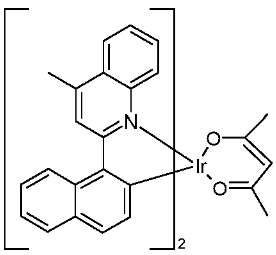 | 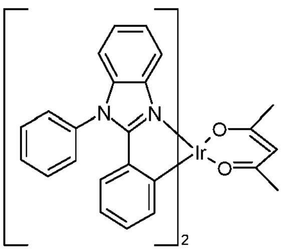 | 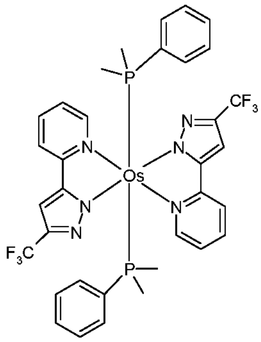 |

| | | |
|---|---|---|
| 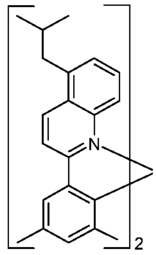 | 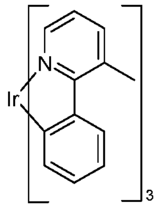 | 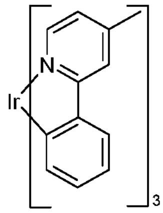 |
| 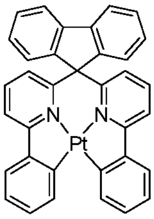 | 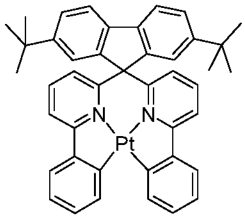 | 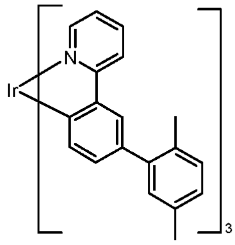 |
| 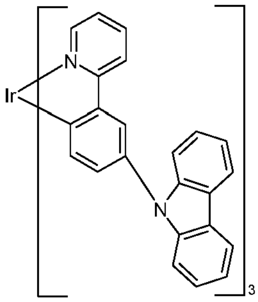 | 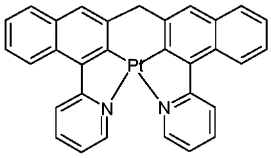 | 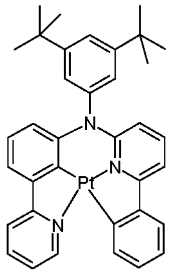 |
| 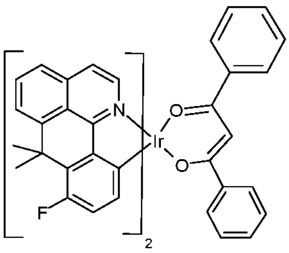 | 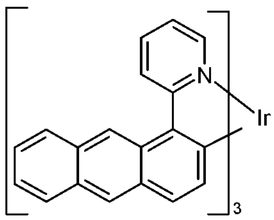 | 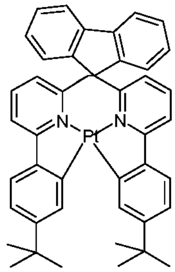 |
| 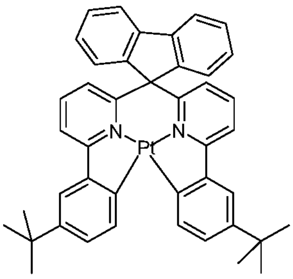 | 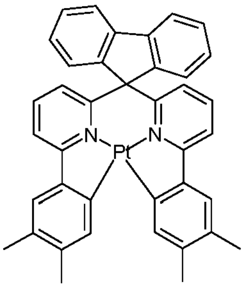 | 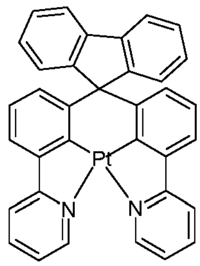 |
| 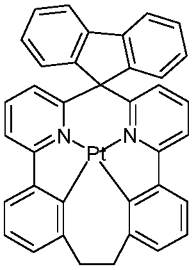 | 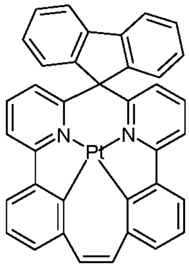 | 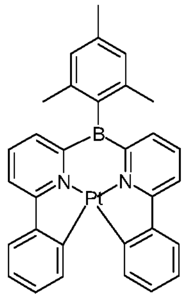 |

| | | |
|---|---|---|
| 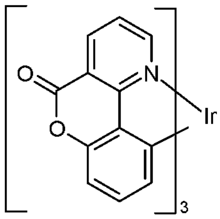 | 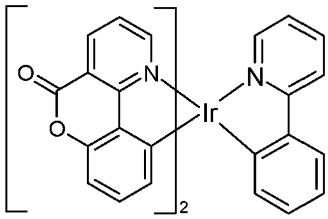 | 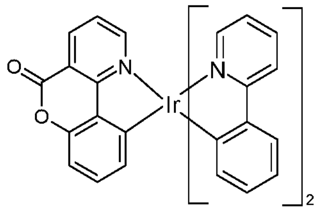 |
| 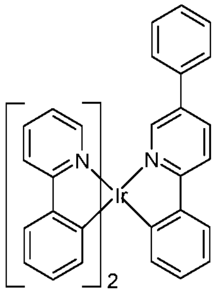 | 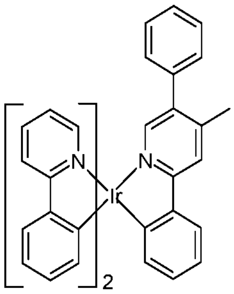 TEG1 | 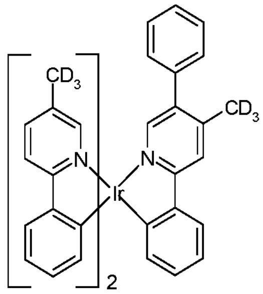 |
| 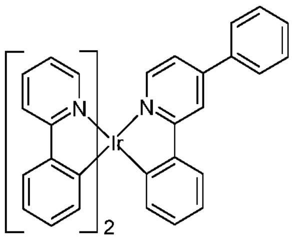 | 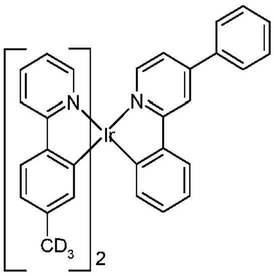 | 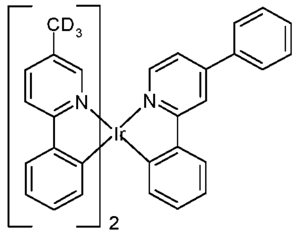 |
| 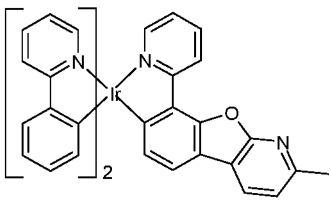 | 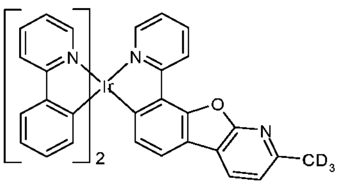 | 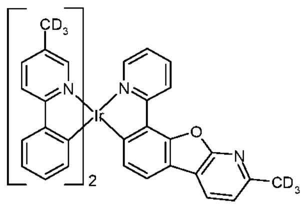 |
| 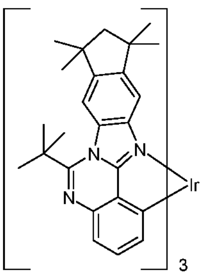 | 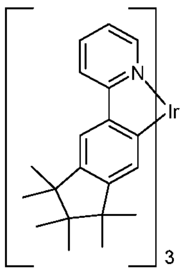 | 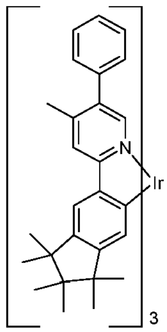 |
| 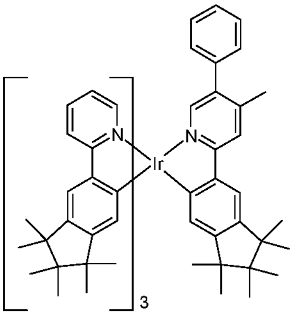 | 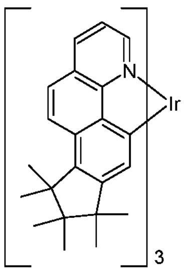 | 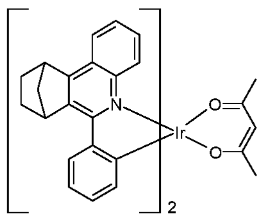 |

Bevorzugte Beispiele von phosphoreszierenden polypodalen Emittern sind in der folgenden Tabelle 5 aufgeführt.

Tabelle 5:

| | | | |
|---|---|---|---|
| CAS-1269508-30-6 | CAS-1989601-68-4 | CAS-1989602-19-8 | CAS-1989602-70-1 |
| CAS-1215692-34-4 | CAS-1989601-69-5 | CAS-1989602-20-1 | CAS-1989602-71-2 |
| CAS-1370364-40-1 | CAS-1989601-70-8 | CAS-1989602-21-2 | CAS-1989602-72-3 |
| CAS-1370364-42-3 | CAS-1989601-71-9 | CAS-1989602-22-3 | CAS-1989602-73-4 |
| CAS-1989600-74-9 | CAS-1989601-72-0 | CAS-1989602-23-4 | CAS-1989602-74-5 |
| CAS-1989600-75-0 | CAS-1989601-73-1 | CAS-1989602-24-5 | CAS-1989602-75-6 |
| CAS-1989600-77-2 | CAS-1989601-74-2 | CAS-1989602-25-6 | CAS-1989602-76-7 |
| CAS-1989600-78-3 | CAS-1989601-75-3 | CAS-1989602-26-7 | CAS-1989602-77-8 |
| CAS-1989600-79-4 | CAS-1989601-76-4 | CAS-1989602-27-8 | CAS-1989602-78-9 |
| CAS-1989600-82-9 | CAS-1989601-77-5 | CAS-1989602-28-9 | CAS-1989602-79-0 |
| CAS-1989600-83-0 | CAS-1989601-78-6 | CAS-1989602-29-0 | CAS-1989602-80-3 |
| CAS-1989600-84-1 | CAS-1989601-79-7 | CAS-1989602-30-3 | CAS-1989602-82-5 |
| CAS-1989600-85-2 | CAS-1989601-80-0 | CAS-1989602-31-4 | CAS-1989602-84-7 |
| CAS-1989600-86-3 | CAS-1989601-81-1 | CAS-1989602-32-5 | CAS-1989602-85-8 |
| CAS-1989600-87-4 | CAS-1989601-82-2 | CAS-1989602-33-6 | CAS-1989602-86-9 |
| CAS-1989600-88-5 | CAS-1989601-83-3 | CAS-1989602-34-7 | CAS-1989602-87-0 |
| CAS-1989600-89-6 | CAS-1989601-84-4 | CAS-1989602-35-8 | CAS-1989602-88-1 |
| CAS-1989601-11-7 | CAS-1989601-85-5 | CAS-1989602-36-9 | CAS-1989604-00-3 |
| CAS-1989601-23-1 | CAS-1989601-86-6 | CAS-1989602-37-0 | CAS-1989604-01-4 |
| CAS-1989601-26-4 | CAS-1989601-87-7 | CAS-1989602-38-1 | CAS-1989604-02-5 |
| CAS-1989601-28-6 | CAS-1989601-88-8 | CAS-1989602-39-2 | CAS-1989604-03-6 |
| CAS-1989601-29-7 | CAS-1989601-89-9 | CAS-1989602-40-5 | CAS-1989604-04-7 |
| CAS-1989601-33-3 | CAS-1989601-90-2 | CAS-1989602-41-6 | CAS-1989604-05-8 |
| CAS-1989601-40-2 | CAS-1989601-91-3 | CAS-1989602-42-7 | CAS-1989604-06-9 |
| CAS-1989601-41-3 | CAS-1989601-92-4 | CAS-1989602-43-8 | CAS-1989604-07-0 |
| CAS-1989601-42-4 | CAS-1989601-93-5 | CAS-1989602-44-9 | CAS-1989604-08-1 |
| CAS-1989601-43-5 | CAS-1989601-94-6 | CAS-1989602-45-0 | CAS-1989604-09-2 |
| CAS-1989601-44-6 | CAS-1989601-95-7 | CAS-1989602-46-1 | CAS-1989604-10-5 |
| CAS-1989601-45-7 | CAS-1989601-96-8 | CAS-1989602-47-2 | CAS-1989604-11-6 |
| CAS-1989601-46-8 | CAS-1989601-97-9 | CAS-1989602-48-3 | CAS-1989604-13-8 |
| CAS-1989601-47-9 | CAS-1989601-98-0 | CAS-1989602-49-4 | CAS-1989604-14-9 |
| CAS-1989601-48-0 | CAS-1989601-99-1 | CAS-1989602-50-7 | CAS-1989604-15-0 |
| CAS-1989601-49-1 | CAS-1989602-00-7 | CAS-1989602-51-8 | CAS-1989604-16-1 |
| CAS-1989601-50-4 | CAS-1989602-01-8 | CAS-1989602-52-9 | CAS-1989604-17-2 |
| CAS-1989601-51-5 | CAS-1989602-02-9 | CAS-1989602-53-0 | CAS-1989604-18-3 |
| CAS-1989601-52-6 | CAS-1989602-03-0 | CAS-1989602-54-1 | CAS-1989604-19-4 |
| CAS-1989601-53-7 | CAS-1989602-04-1 | CAS-1989602-55-2 | CAS-1989604-20-7 |
| CAS-1989601-54-8 | CAS-1989602-05-2 | CAS-1989602-56-3 | CAS-1989604-21-8 |

(fortgesetzt)

| | | | |
|---|---|---|---|
| CAS-1989601-55-9 | CAS-1989602-06-3 | CAS-1989602-57-4 | CAS-1989604-22-9 |
| CAS-1989601-56-0 | CAS-1989602-07-4 | CAS-1989602-58-5 | CAS-1989604-23-0 |
| CAS-1989601-57-1 | CAS-1989602-08-5 | CAS-1989602-59-6 | CAS-1989604-24-1 |
| CAS-1989601-58-2 | CAS-1989602-09-6 | CAS-1989602-60-9 | CAS-1989604-25-2 |
| CAS-1989601-59-3 | CAS-1989602-10-9 | CAS-1989602-61-0 | CAS-1989604-26-3 |
| CAS-1989601-60-6 | CAS-1989602-11-0 | CAS-1989602-62-1 | CAS-1989604-27-4 |
| CAS-1989601-61-7 | CAS-1989602-12-1 | CAS-1989602-63-2 | CAS-1989604-28-5 |
| CAS-1989601-62-8 | CAS-1989602-13-2 | CAS-1989602-64-3 | CAS-1989604-29-6 |
| CAS-1989601-63-9 | CAS-1989602-14-3 | CAS-1989602-65-4 | CAS-1989604-30-9 |
| CAS-1989601-64-0 | CAS-1989602-15-4 | CAS-1989602-66-5 | CAS-1989604-31-0 |
| CAS-1989601-65-1 | CAS-1989602-16-5 | CAS-1989602-67-6 | CAS-1989604-32-1 |
| CAS-1989601-66-2 | CAS-1989602-17-6 | CAS-1989602-68-7 | CAS-1989604-33-2 |
| CAS-1989601-67-3 | CAS-1989602-18-7 | CAS-1989602-69-8 | CAS-1989604-34-3 |
| CAS-1989604-35-4 | CAS-1989604-88-7 | CAS-1989605-52-8 | CAS-1989606-07-6 |
| CAS-1989604-36-5 | CAS-1989604-89-8 | CAS-1989605-53-9 | CAS-1989606-08-7 |
| CAS-1989604-37-6 | CAS-1989604-90-1 | CAS-1989605-54-0 | CAS-1989606-09-8 |
| CAS-1989604-38-7 | CAS-1989604-92-3 | CAS-1989605-55-1 | CAS-1989606-10-1 |
| CAS-1989604-39-8 | CAS-1989604-93-4 | CAS-1989605-56-2 | CAS-1989606-11-2 |
| CAS-1989604-40-1 | CAS-1989604-94-5 | CAS-1989605-57-3 | CAS-1989606-12-3 |
| CAS-1989604-41-2 | CAS-1989604-95-6 | CAS-1989605-58-4 | CAS-1989606-13-4 |
| CAS-1989604-42-3 | CAS-1989604-96-7 | CAS-1989605-59-5 | CAS-1989606-14-5 |
| CAS-1989604-43-4 | CAS-1989604-97-8 | CAS-1989605-61-9 | CAS-1989606-15-6 |
| CAS-1989604-45-6 | CAS-1989605-09-5 | CAS-1989605-62-0 | CAS-1989606-16-7 |
| CAS-1989604-46-7 | CAS-1989605-10-8 | CAS-1989605-63-1 | CAS-1989606-17-8 |
| CAS-1989604-47-8 | CAS-1989605-11-9 | CAS-1989605-64-2 | CAS-1989606-18-9 |
| CAS-1989604-48-9 | CAS-1989605-13-1 | CAS-1989605-65-3 | CAS-1989606-19-0 |
| CAS-1989604-49-0 | CAS-1989605-14-2 | CAS-1989605-66-4 | CAS-1989606-20-3 |
| CAS-1989604-50-3 | CAS-1989605-15-3 | CAS-1989605-67-5 | CAS-1989606-21-4 |
| CAS-1989604-52-5 | CAS-1989605-16-4 | CAS-1989605-68-6 | CAS-1989606-22-5 |
| CAS-1989604-53-6 | CAS-1989605-17-5 | CAS-1989605-69-7 | CAS-1989606-23-6 |
| CAS-1989604-54-7 | CAS-1989605-18-6 | CAS-1989605-70-0 | CAS-1989606-24-7 |
| CAS-1989604-55-8 | CAS-1989605-19-7 | CAS-1989605-71-1 | CAS-1989606-26-9 |
| CAS-1989604-56-9 | CAS-1989605-20-0 | CAS-1989605-72-2 | CAS-1989606-27-0 |
| CAS-1989604-57-0 | CAS-1989605-21-1 | CAS-1989605-73-3 | CAS-1989606-28-1 |
| CAS-1989604-58-1 | CAS-1989605-22-2 | CAS-1989605-74-4 | CAS-1989606-29-2 |
| CAS-1989604-59-2 | CAS-1989605-23-3 | CAS-1989605-75-5 | CAS-1989606-30-5 |
| CAS-1989604-60-5 | CAS-1989605-24-4 | CAS-1989605-76-6 | CAS-1989606-31-6 |
| CAS-1989604-61-6 | CAS-1989605-25-5 | CAS-1989605-77-7 | CAS-1989606-32-7 |
| CAS-1989604-62-7 | CAS-1989605-26-6 | CAS-1989605-78-8 | CAS-1989606-33-8 |

(fortgesetzt)

| | | | |
|---|---|---|---|
| CAS-1989604-63-8 | CAS-1989605-27-7 | CAS-1989605-79-9 | CAS-1989606-34-9 |
| CAS-1989604-64-9 | CAS-1989605-28-8 | CAS-1989605-81-3 | CAS-1989606-35-0 |
| CAS-1989604-65-0 | CAS-1989605-29-9 | CAS-1989605-82-4 | CAS-1989606-36-1 |
| CAS-1989604-66-1 | CAS-1989605-30-2 | CAS-1989605-83-5 | CAS-1989606-37-2 |
| CAS-1989604-67-2 | CAS-1989605-31-3 | CAS-1989605-84-6 | CAS-1989606-38-3 |
| CAS-1989604-68-3 | CAS-1989605-32-4 | CAS-1989605-85-7 | CAS-1989606-39-4 |
| CAS-1989604-69-4 | CAS-1989605-33-5 | CAS-1989605-86-8 | CAS-1989606-40-7 |
| CAS-1989604-70-7 | CAS-1989605-34-6 | CAS-1989605-87-9 | CAS-1989606-41-8 |
| CAS-1989604-71-8 | CAS-1989605-35-7 | CAS-1989605-88-0 | CAS-1989606-42-9 |
| CAS-1989604-72-9 | CAS-1989605-36-8 | CAS-1989605-89-1 | CAS-1989606-43-0 |
| CAS-1989604-73-0 | CAS-1989605-37-9 | CAS-1989605-90-4 | CAS-1989606-44-1 |
| CAS-1989604-74-1 | CAS-1989605-38-0 | CAS-1989605-91-5 | CAS-1989606-45-2 |
| CAS-1989604-75-2 | CAS-1989605-39-1 | CAS-1989605-92-6 | CAS-1989606-46-3 |
| CAS-1989604-76-3 | CAS-1989605-40-4 | CAS-1989605-93-7 | CAS-1989606-48-5 |
| CAS-1989604-77-4 | CAS-1989605-41-5 | CAS-1989605-94-8 | CAS-1989606-49-6 |
| CAS-1989604-78-5 | CAS-1989605-42-6 | CAS-1989605-95-9 | CAS-1989606-53-2 |
| CAS-1989604-79-6 | CAS-1989605-43-7 | CAS-1989605-96-0 | CAS-1989606-55-4 |
| CAS-1989604-80-9 | CAS-1989605-44-8 | CAS-1989605-97-1 | CAS-1989606-56-5 |
| CAS-1989604-81-0 | CAS-1989605-45-9 | CAS-1989605-98-2 | CAS-1989606-61-2 |
| CAS-1989604-82-1 | CAS-1989605-46-0 | CAS-1989605-99-3 | CAS-1989606-62-3 |
| CAS-1989604-83-2 | CAS-1989605-47-1 | CAS-1989606-00-9 | CAS-1989606-63-4 |
| CAS-1989604-84-3 | CAS-1989605-48-2 | CAS-1989606-01-0 | CAS-1989606-67-8 |
| CAS-1989604-85-4 | CAS-1989605-49-3 | CAS-1989606-04-3 | CAS-1989606-69-0 |
| CAS-1989604-86-5 | CAS-1989605-50-6 | CAS-1989606-05-4 | CAS-1989606-70-3 |
| CAS-1989604-87-6 | CAS-1989605-51-7 | CAS-1989606-06-5 | CAS-1989606-74-7 |
| CAS-1989658-39-0 | CAS-2088184-56-7 | CAS-2088185-07-1 | CAS-2088185-66-2 |
| CAS-1989658-41-4 | CAS-2088184-57-8 | CAS-2088185-08-2 | CAS-2088185-67-3 |
| CAS-1989658-43-6 | CAS-2088184-58-9 | CAS-2088185-09-3 | CAS-2088185-68-4 |
| CAS-1989658-47-0 | CAS-2088184-59-0 | CAS-2088185-10-6 | CAS-2088185-69-5 |
| CAS-1989658-49-2 | CAS-2088184-60-3 | CAS-2088185-11-7 | CAS-2088185-70-8 |
| CAS-2088184-07-8 | CAS-2088184-61-4 | CAS-2088185-12-8 | CAS-2088185-71-9 |
| CAS-2088184-08-9 | CAS-2088184-62-5 | CAS-2088185-13-9 | CAS-2088185-72-0 |
| CAS-2088184-09-0 | CAS-2088184-63-6 | CAS-2088185-14-0 | CAS-2088185-73-1 |
| CAS-2088184-10-3 | CAS-2088184-64-7 | CAS-2088185-15-1 | CAS-2088185-74-2 |
| CAS-2088184-11-4 | CAS-2088184-65-8 | CAS-2088185-16-2 | CAS-2088185-75-3 |
| CAS-2088184-13-6 | CAS-2088184-66-9 | CAS-2088185-17-3 | CAS-2088185-76-4 |
| CAS-2088184-14-7 | CAS-2088184-67-0 | CAS-2088185-18-4 | CAS-2088185-77-5 |
| CAS-2088184-15-8 | CAS-2088184-68-1 | CAS-2088185-19-5 | CAS-2088185-78-6 |
| CAS-2088184-16-9 | CAS-2088184-69-2 | CAS-2088185-20-8 | CAS-2088185-79-7 |

(fortgesetzt)

| | | | |
|---|---|---|---|
| CAS-2088184-17-0 | CAS-2088184-70-5 | CAS-2088185-21-9 | CAS-2088185-80-0 |
| CAS-2088184-18-1 | CAS-2088184-71-6 | CAS-2088185-22-0 | CAS-2088185-81-1 |
| CAS-2088184-19-2 | CAS-2088184-72-7 | CAS-2088185-23-1 | CAS-2088185-82-2 |
| CAS-2088184-20-5 | CAS-2088184-73-8 | CAS-2088185-32-2 | CAS-2088185-83-3 |
| CAS-2088184-21-6 | CAS-2088184-74-9 | CAS-2088185-33-3 | CAS-2088185-84-4 |
| CAS-2088184-22-7 | CAS-2088184-75-0 | CAS-2088185-34-4 | CAS-2088185-85-5 |
| CAS-2088184-23-8 | CAS-2088184-76-1 | CAS-2088185-35-5 | CAS-2088185-86-6 |
| CAS-2088184-24-9 | CAS-2088184-77-2 | CAS-2088185-36-6 | CAS-2088185-87-7 |
| CAS-2088184-25-0 | CAS-2088184-78-3 | CAS-2088185-37-7 | CAS-2088185-88-8 |
| CAS-2088184-26-1 | CAS-2088184-79-4 | CAS-2088185-38-8 | CAS-2088185-89-9 |
| CAS-2088184-27-2 | CAS-2088184-80-7 | CAS-2088185-39-9 | CAS-2088185-90-2 |
| CAS-2088184-28-3 | CAS-2088184-81-8 | CAS-2088185-40-2 | CAS-2088185-91-3 |
| CAS-2088184-29-4 | CAS-2088184-82-9 | CAS-2088185-41-3 | CAS-2088185-92-4 |
| CAS-2088184-30-7 | CAS-2088184-83-0 | CAS-2088185-42-4 | CAS-2088185-93-5 |
| CAS-2088184-32-9 | CAS-2088184-84-1 | CAS-2088185-43-5 | CAS-2088185-94-6 |
| CAS-2088184-34-1 | CAS-2088184-85-2 | CAS-2088185-44-6 | CAS-2088185-95-7 |
| CAS-2088184-35-2 | CAS-2088184-86-3 | CAS-2088185-45-7 | CAS-2088185-96-8 |
| CAS-2088184-36-3 | CAS-2088184-87-4 | CAS-2088185-46-8 | CAS-2088185-97-9 |
| CAS-2088184-37-4 | CAS-2088184-88-5 | CAS-2088185-47-9 | CAS-2088185-98-0 |
| CAS-2088184-38-5 | CAS-2088184-89-6 | CAS-2088185-48-0 | CAS-2088185-99-1 |
| CAS-2088184-39-6 | CAS-2088184-90-9 | CAS-2088185-49-1 | CAS-2088186-00-7 |
| CAS-2088184-40-9 | CAS-2088184-91-0 | CAS-2088185-50-4 | CAS-2088186-01-8 |
| CAS-2088184-41-0 | CAS-2088184-92-1 | CAS-2088185-51-5 | CAS-2088186-02-9 |
| CAS-2088184-42-1 | CAS-2088184-93-2 | CAS-2088185-52-6 | CAS-2088195-88-2 |
| CAS-2088184-43-2 | CAS-2088184-94-3 | CAS-2088185-53-7 | CAS-2088195-89-3 |
| CAS-2088184-44-3 | CAS-2088184-95-4 | CAS-2088185-54-8 | CAS-2088195-90-6 |
| CAS-2088184-45-4 | CAS-2088184-96-5 | CAS-2088185-55-9 | CAS-2088195-91-7 |
| CAS-2088184-46-5 | CAS-2088184-97-6 | CAS-2088185-56-0 | CAS-861806-70-4 |
| CAS-2088184-47-6 | CAS-2088184-98-7 | CAS-2088185-57-1 | CAS-1269508-30-6 |
| CAS-2088184-48-7 | CAS-2088184-99-8 | CAS-2088185-58-2 | |
| CAS-2088184-49-8 | CAS-2088185-00-4 | CAS-2088185-59-3 | |
| CAS-2088184-50-1 | CAS-2088185-01-5 | CAS-2088185-60-6 | |
| CAS-2088184-51-2 | CAS-2088185-02-6 | CAS-2088185-61-7 | |
| CAS-2088184-52-3 | CAS-2088185-03-7 | CAS-2088185-62-8 | |
| CAS-2088184-53-4 | CAS-2088185-04-8 | CAS-2088185-63-9 | |
| CAS-2088184-54-5 | CAS-2088185-05-9 | CAS-2088185-64-0 | |
| CAS-2088184-55-6 | CAS-2088185-06-0 | CAS-2088185-65-1 | |

In der erfindungsgemäßen Zusammensetzung wird bevorzugt jede Mischung M1, M2, M3, M4, M5, M6, M7,

M8, M9, M10, M11, M12, M13, M14, M15, M16, M17, M18, M19, M20, M21, M22, M23, M24, M25, M26, M27, M28, M29, M30, M31, M32, M33, M34, M35, M36, M37, M38, M39, M40, M41, M42, M43, M44, M45, M46, M47, M48, M49, M50, M51, M52, M53, M54, M55, M56, M57, M58, M59, M60, M61, M62, M63, M64, M65, M66, M67, M68, M69, M70, M71 oder M72 mit einer Verbindung aus Tabelle 4 oder 5 kombiniert.

Die erfindungsgemäße Zusammensetzung enthaltend mindestens einen phosphoreszierenden Emitter bildet bevorzugt eine infra-rot emittierende, gelb, orange, rot, grün, blau oder ultra-violett emittierende Schicht, besonders bevorzugt eine grün emittierende Schicht.

Dabei wird unter einer gelb emittierenden Schicht eine Schicht verstanden, deren Photolumineszenzmaximum im Bereich von 540 bis 570 nm liegt. Unter einer orange emittierenden Schicht wird eine Schicht verstanden, deren Photolumineszenzmaximum im Bereich von 570 bis 600 nm liegt. Unter einer rot emittierenden Schicht wird eine Schicht verstanden, deren Photolumineszenzmaximum im Bereich von 600 bis 750 nm liegt. Unter einer grün emittierenden Schicht wird eine Schicht verstanden, deren Photolumineszenzmaximum im Bereich von 490 bis 540 nm liegt. Unter einer blau emittierenden Schicht wird eine Schicht verstanden, deren Photolumineszenzmaximum im Bereich von 440 bis 490 nm liegt. Dabei wird das Photolumineszenzmaximum der Schicht durch Messung des Photolumineszenzspektrums der Schicht mit einer Schichtdicke von 50 nm bei Raumtemperatur bestimmt, wobei die Schicht die erfindungsgemäße Zusammensetzung aufweist, also Emitter und Matrix enthält.

Die Aufnahme des Photolumineszenzspektrums der Schicht erfolgt beispielsweise mit einem handelsüblichen Photolumineszenzspektrometer.

Das Photolumineszenzspektrum des gewählten Emitters wird in der Regel in Sauerstoff-freier Lösung, 10-5 molar, gemessen, wobei die Messung bei Raumtemperatur erfolgt und jedes Lösemittel geeignet ist, in dem sich der gewählte Emitter in der genannten Konzentration löst. Besonders geeignete Lösemittel sind üblicherweise Toluol oder 2-Methyl-THF, aber auch Dichlormethan. Gemessen wird mit einem handelsüblichen Photolumineszenzspektrometer. Die Triplettenergie T1 in eV wird aus den Photolumineszenzspektren der Emitter bestimmt. Es wird zunächst das Peakmaximum Plmax. (in nm) des Photolumineszenzspektrums bestimmt. Das Peakmaximum Plmax. (in nm) wird dann in eV umgerechnet gemäß: E(T1 in eV) = 1240 / E(T1 in nm) = 1240 / Plmax. (in nm).

Bevorzugte phosphoreszierende Emitter sind demzufolge infra-rote Emitter, vorzugsweise aus Tabelle 4 oder 5, deren Triplettenergie $T_1$ bevorzugt bei ~1.9 eV bis ~1.0 eV liegt.

Bevorzugte phosphoreszierende Emitter sind demzufolge rote Emitter, vorzugsweise aus Tabelle 4 oder 5, deren Triplettenergie $T_1$ bevorzugt bei ~2.1 eV bis ~1.9 eV liegt.

Bevorzugte phosphoreszierende Emitter sind demzufolge gelbe Emitter, vorzugsweise aus Tabelle 4 oder 5, deren Triplettenergie $T_1$ bevorzugt bei ~2.3 eV bis ~2.1 eV liegt.

Bevorzugte phosphoreszierende Emitter sind demzufolge grüne Emitter, vorzugsweise aus Tabelle 4 oder 5, deren Triplettenergie $T_1$ bevorzugt bei ~2.5 eV bis ~2.3 eV liegt.

Bevorzugte phosphoreszierende Emitter sind demzufolge blaue Emitter, vorzugsweise aus Tabelle 4 oder 5, deren Triplettenergie $T_1$ bevorzugt bei ~3.1 eV bis ~2.5 eV liegt.

Bevorzugte phosphoreszierende Emitter sind demzufolge ultra-violette Emitter, vorzugsweise aus Tabelle 4 oder 5, deren Triplettenergie $T_1$ bevorzugt bei ~4.0 eV bis ~3.1 eV liegt.

Besonders bevorzugte phosphoreszierende Emitter sind demzufolge grüne Emitter, vorzugsweise aus Tabelle 4 oder 5, wie zuvor beschrieben.

Ganz besonders bevorzugte phosphoreszierende Emitter sind demzufolge grüne Emitter, vorzugsweise aus Tabelle 4 oder 5, deren Triplettenergie $T_1$ bevorzugt bei ~2.5 eV bis ~2.3 eV liegt.

Ganz besonders bevorzugt werden grüne Emitter, vorzugsweise aus Tabelle 4 oder 5, wie zuvor beschrieben, für die erfindungsgemäße Zusammensetzung bzw. erfindungsgemäße emittierende Schicht ausgewählt.

Bevorzugte fluoreszierende Emitter sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte fluoreszierende Emitter sind Indenofluorenamine bzw. -diamine, beispielsweise gemäß WO 2006/108497 oder WO 2006/122630, Benzoindenofluorenamine bzw. -diamine, beispielsweise gemäß WO 2008/006449, und Dibenzoindenofluorenamine bzw. -diamine, beispielsweise gemäß WO 2007/140847, sowie die in WO 2010/012328 offenbarten Indenofluorenderivate mit kondensierten Arylgruppen.

In einer weiteren bevorzugten Ausführungsform der Erfindung wird die erfindungsgemäße Zusammensetzung als eine Komponente von Mixed-Matrix-Systemen verwendet. Die Mixed-Matrix-Systeme umfassen bevorzugt drei oder vier verschiedene Matrixmaterialien, besonders bevorzugt drei verschiedene Matrixmaterialien (das heißt, eine weitere Matrixkomponente zusätzlich zu der erfindungsgemäßen Zusammensetzung). Besonders geeignete Matrixmaterialien, welche in Kombination mit der erfindungsgemäßen Zusammensetzung als Matrixkomponente(n) eines Mixed-Matrix-Systems verwendet werden können, sind ausgewählt aus wide-band-gap-Materialien, Elektronentransportmaterialien (ETM) und Lochtransportmaterialien (HTM).

Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten. Besonders geeignete Matrixmaterialien, welche in Kombination mit der erfindungsgemäßen Zusammensetzung als Matrixkomponente(n) eines Mixed-Matrix-Systems in phosphoreszierenden oder fluoreszierenden organischen Elektrolumineszenzvorrichtungen eingesetzt werden können, sind ausgewählt aus den unten angegebenen bevorzugten Matrixmaterialien für phosphoreszierende Emitter oder den bevorzugten Matrixmaterialien für fluoreszierende Emitter, je nachdem welche Art von Emitter eingesetzt wird. Bevorzugt wird das Mixed-Matrix-System auf einen Emitter der Tabelle 4 oder 5 optimiert.

Als weitere Hostmaterialien, bevorzugt für fluoreszierende Emitter, kommen neben der erfindungsgemäßen Zusammensetzung, wie zuvor beschrieben, umfassend besonders bevorzugt eine Mischung an Materialien ausgewählt aus M1 bis M72, verschiedene Stoffklassen in Frage. Bevorzugte weitere Hostmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Hostmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Als weitere Matrixmaterialien, bevorzugt für phosphoreszierende Emitter, kommen neben der erfindungsgemäßen Zusammensetzung, wie zuvor beschrieben, umfassend besonders bevorzugt eine Mischung an Materialien ausgewählt aus M1 bis M72, verschiedene Stoffklassen in Frage. Bevorzugte weitere Matrixmaterialien sind ausgewählt aus den Klassen der aromatischen Amine, insbesondere Triarylamine, z. B. gemäß US 2005/0069729, Carbazolderivate (z. B. CBP, N,N-Biscarbazolylbiphenyl) oder Verbindungen gemäß WO 2005/039246, US 2005/0069729, JP 2004/288381, EP 1205527 oder WO 2008/086851, verbrückten Carbazolderivate, z. B. gemäß WO 2011/088877 und WO 2011/128017, Indenocarbazolderivate, z. B. gemäß WO 2010/136109 und WO 2011/000455, Azacarbazolderivate, z. B. gemäß EP 1617710, EP 1617711, EP 1731584, JP 2005/347160, Indolocarbazolderivate, z. B. gemäß WO 2007/063754 oder WO 2008/056746, Ketone, z. B. gemäß WO 2004/093207 oder WO 2010/006680, Phosphinoxide, Sulfoxide und Sulfone, z. B. gemäß WO 2005/003253, Oligophenylene, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Aluminiumkomplexe, z. B. BAlq, Diazasilol- und Tetraazasilol-Derivate, z. B. gemäß WO 2010/054729, DiazaphospholDerivate, z. B. gemäß WO 2010/054730 und Aluminiumkomplexe, z. B. BAlQ.

Gemäß einer alternativen Ausführungsform der vorliegenden Erfindung enthält die Zusammensetzung neben den Bestandteilen bipolarer Host und neutraler Co-Host und gegebenenfalls einem phosphoreszierenden Emitter, keine weiteren Bestandteile, das heißt, funktionellen Materialien.

Ein weiterer Gegenstand der Erfindung ist demzufolge eine Zusammensetzung bestehend aus einer Verbindung der Formeln (1a) bis (1j) oder einer Verbindung ausgewählt aus 1 bis 8 und einer Verbindung der Formeln (2), (2a), (2b), (2c) oder einer Verbindung ausgewählt aus 9 bis 17.

Ein weiterer Gegenstand der Erfindung ist demzufolge eine Zusammensetzung bestehend aus einem phosphoreszierenden Emitter, einer Verbindung der Formeln (1a) bis (1j) oder einer Verbindung ausgewählt aus 1 bis 8 und einer Verbindung der Formeln (2), (2a), (2b), (2c) oder einer Verbindung ausgewählt aus 9 bis 17.

Die erfindungsgemäße Zusammensetzung, wie zuvor beschrieben oder bevorzugt beschrieben, eignet sich für die Verwendung in einer organischen elektronischen Vorrichtung. Dabei wird unter einer organischen elektronischen Vorrichtung eine Vorrichtung verstanden, welche mindestens eine Schicht enthält, die mindestens eine organische Verbindung enthält. Die Vorrichtung kann aber auch anorganische Materialien enthalten oder auch Schichten, welche vollständig aus anorganischen Materialien aufgebaut sind.

Ein weiterer Gegenstand der Erfindung ist demzufolge die Verwendung einer Zusammensetzung, wie zuvor beschrieben oder bevorzugt beschrieben, insbesondere einer Mischung ausgewählt aus M1 bis M72, in einer organischen elektronischen Vorrichtung.

Die Komponenten bzw. Bestandteile der Zusammensetzungen können dabei durch Aufdampfen oder aus Lösung prozessiert werden. Sofern die Zusammensetzungen aus Lösung aufgebracht werden, sind Formulierungen der erfindungsgemäßen Zusammensetzung enthaltend wenigstens ein weiteres Lösungsmittel erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden.

Ein weiterer Gegenstand der vorliegenden Erfindung ist daher eine Formulierung enthaltend eine erfindungsgemäße Zusammensetzung und mindestens ein Lösemittel.

Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, $\alpha$-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, Hexamethylindan oder Mischungen dieser Lösemittel.

Die Formulierung kann dabei auch mindestens eine weitere organische oder anorganische Verbindung enthalten, die ebenfalls in der elektronischen Vorrichtung eingesetzt wird, insbesondere eine emittierende Verbindung, insbesondere ein phosphoreszierender Emitter und/oder ein weiteres Matrixmaterial. Geeignete emittierende Verbindungen und weitere Matrixmaterialien wurden vorstehend bereits aufgeführt.

Gegenstand der vorliegenden Erfindung ist auch die Verwendung der erfindungsgemäßen Zusammensetzung in einer organischen elektronischen Vorrichtung, bevorzugt in einer elektronentransportierenden und/oder in einer emittierenden Schicht.

Die organische elektronische Vorrichtung ist bevorzugt gewählt aus den organischen integrierten Schaltungen (OICs), organischen Feld-Effekt-Transistoren (OFETs), organischen Dünnfilmtransistoren (OTFTs), organischen Elektrolumineszenzvorrichtungen, organischen Solarzellen (OSCs), organischen optischen Detektoren und organischen Photorezeptoren, wobei die organischen Elektrolumineszenzvorrichtungen besonders bevorzugt sind.

Ganz besonders bevorzugte organische Elektrolumineszenzvorrichtungen für die Verwendung der erfindungsgemäßen Zusammensetzung sind organische lichtemittierende Transistoren (OLETs), organische Feld-Quench-Devices (OFQDs), organische lichtemittierende elektrochemischen Zellen (OLECs, LECs, LEECs), organische Laserdioden (O-Laser) und organische lichtemittierende Dioden (OLEDs), insbesondere bevorzugt sind OLECs und OLEDs und am meisten bevorzugt sind OLEDs.

Bevorzugt wird die erfindungsgemäße Zusammensetzung, wie zuvor beschrieben oder als bevorzugt beschrieben, in einer elektronischen Vorrichtung in einer Schicht mit elektronentransportierender Funktion verwendet. Die Schicht ist bevorzugt eine Elektroneninjektions-schicht (EIL), eine Elektronentransportschicht (ETL), eine Lochblockier-schicht (HBL) und/oder eine Emissionsschicht (EML), besonders bevorzugt eine ETL, EIL und /oder eine EML. Ganz besonders bevorzugt wird die erfindungsgemäße Zusammensetzung in einer EML eingesetzt, insbesondere als Matrixmaterial.

Ein weiterer Gegenstand der vorliegenden Erfindung ist deshalb eine organische elektronische Vorrichtung, die insbesondere aus einer der vorstehend genannten elektronischen Vorrichtungen ausgewählt ist und die die erfindungsgemäße Zusammensetzung, wie zuvor beschrieben oder bevorzugt beschrieben, enthält, bevorzugt in einer Emissionsschicht (EML), in einer Elektronentransportschicht (ETL), in einer Elektroneninjektionsschicht (EIL) und/oder in einer Lochblockierschicht (HBL), ganz bevorzugt in einer EML, EIL und/oder ETL und ganz besonders bevorzugt in einer EML.

Wenn es sich um eine emittierende Schicht handelt, dann ist es insbesondere bevorzugt eine phosphoreszierende Schicht, die dadurch gekennzeichnet ist, dass sie zusätzlich zu der Zusammensetzung, wie zuvor beschrieben oder bevorzugt beschrieben, einen phosphoreszierenden Emitter enthält, insbesondere zusammen mit einem Emitter der Tabelle 4 oder 5 oder einem bevorzugten Emitter, wie zuvor beschrieben.

In einer besonders bevorzugten Ausführungsform der vorliegenden Erfindung handelt es sich daher bei der elektronischen Vorrichtung um eine organische Elektrolumineszenzvorrichtung, ganz besonders bevorzugt um eine organische lichtemittierende Diode (OLED), die die erfindungsgemäße Zusammensetzung, wie zuvor beschrieben oder bevorzugt beschrieben, zusammen mit einem phosphoreszierenden Emitter, bevorzugt mit einem Emitter der Tabelle 4 oder 5, besonders bevorzugt mit einem grünen Emitter, in der Emissionsschicht (EML) enthält.

Die erfindungsgemäße Zusammensetzung gemäß den bevorzugten Ausführungsformen enthaltend mindestens eine emittierende Verbindung enthält vorzugsweise zwischen 99,9 und 1 Vol.-%, weiter vorzugsweise zwischen

99 und 10 Vol.-%, besonders bevorzugt zwischen 98 und 60 Vol.-%, ganz besonders bevorzugt zwischen 97 und 80 Vol.-% an Matrixmaterial aus mindestens einer Verbindung der Formel (1a) oder Formel (1b) und mindestens einer Verbindung der Formel (2) gemäß den bevorzugten Ausführungsformen, bezogen auf die gesamte Zusammensetzung aus Emitter und Matrixmaterial. Entsprechend enthält die Zusammensetzung vorzugsweise zwischen 0,1 und 99 Vol.-%, weiter vorzugsweise zwischen 1 und 90 Vol.-%, besonders bevorzugt zwischen 2 und 40 Vol.-%, ganz besonders bevorzugt zwischen 3 und 20 Vol.-% des Emitters bezogen auf die gesamte Zusammensetzung aus Emitter und Matrixmaterial. Werden die Verbindungen aus Lösung verarbeitet, so werden statt der oben angegebenen Mengen in Vol.-% bevorzugt die entsprechenden Mengen in Gew.-% verwendet.

Außer Kathode, Anode und der Schicht enthaltend die erfindungsgemäße Zusammensetzung kann eine elektronische Vorrichtung noch weitere Schichten enthalten. Diese sind beispielsweise gewählt aus jeweils einer oder mehreren Lochinjektionsschichten, Lochtransportschichten, Lochblockierschichten, emittierenden Schichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen. Es sei aber darauf hingewiesen, dass nicht notwendigerweise jede dieser Schichten vorhanden sein muss.

Die Abfolge der Schichten in einer organischen Elektrolumineszenzvorrichtung ist bevorzugt die folgende: Anode / Lochinjektionsschicht / Lochtransportschicht / emittierende Schicht / Elektronentransportschicht / Elektroneninjektionsschicht / Kathode.

Diese Abfolge der Schichten ist eine bevorzugte Abfolge. Dabei soll erneut darauf hingewiesen werden, dass nicht alle der genannten Schichten vorhanden sein müssen, und/oder dass zusätzlich weitere Schichten vorhanden sein können.

Eine organische Elektrolumineszenzvorrichtung, die die erfindungsgemäße Zusammensetzung enthält, kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren. Insbesondere bevorzugt sind Dreischichtsysteme, also Systeme mit drei emittierenden Schichten, wobei die drei Schichten blaue, grüne und orange oder rote Emission zeigen (für den prinzipiellen Aufbau siehe z. B. WO 2005/011013). Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert.

Geeignete Ladungstransportmaterialien, wie sie in der Lochinjektions- bzw. Lochtransportschicht bzw. Elektronenblockierschicht oder in der Elektronentransportschicht der erfindungsgemäßen organischen Elektrolumineszenzvorrichtung verwendet werden können, sind beispielsweise die in Y. Shirota et al., Chem. Rev. 2007, 107(4), 953-1010 offenbarten Verbindungen oder andere Materialien, wie sie gemäß Stand der Technik in diesen Schichten eingesetzt werden.

Als Materialien für die Elektronentransportschicht können alle Materialien verwendet werden, wie sie gemäß Stand der Technik als Elektronentransportmaterialien in der Elektronentransportschicht verwendet werden. Insbesondere eignen sich Aluminiumkomplexe, beispielsweise $Alq_3$, Zirkoniumkomplexe, beispielsweise $Zrq_4$, Benzimidazolderviate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Weiterhin geeignete Materialien sind Derivate der oben genannten Verbindungen, wie sie in JP 2000/053957, WO 2003/060956, WO 2004/028217, WO 2004/080975 und WO 2010/072300 offenbart werden.

Als Lochtransportmaterialien sind insbesondere Materialien bevorzugt, die in einer Lochtransport-, Lochinjektions- oder Elektronenblockierschicht verwendet werden können, wie Indenofluorenamin-Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Amine (z. B. gemäß WO 2012/034627 oder der noch nicht offengelegten EP 12000929.5), Fluoren-Amine (z. B. gemäß WO 2014/015937, WO 2014/015938 und WO 2014/015935), Spiro-Dibenzopyran-Amine (z. B. gemäß WO 2013/083216) und Dihydroacridin-Derivate (z. B. WO 2012/150001).

Als Kathode elektronischer Vorrichtungen sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und

dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, $Li_2O$, $BaF_2$, MgO, NaF, CsF, $Cs_2CO_3$, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/$NiO_x$, Al/$PtO_x$) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Die organische elektronische Vorrichtung wird bei der Herstellung entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer weiteren bevorzugten Ausführungsform ist die organische elektronische Vorrichtung, die die erfindungsgemäße Zusammensetzung enthält, dadurch gekennzeichnet, dass eine oder mehrere organische Schichten enthaltend die erfindungsgemäße Zusammensetzung mit einem Sublimationsverfahren aufgetragen werden. Dabei werden die Materialien in Vakuum-Sublimationsanlagen bei einem Anfangsdruck kleiner $10^{-5}$ mbar, bevorzugt kleiner $10^{-6}$ mbar aufgedampft. Dabei ist es jedoch auch möglich, dass der Anfangsdruck noch geringer ist, beispielsweise kleiner $10^{-7}$ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation aufgetragen werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere organische Schichten enthaltend die erfindungsgemäße Zusammensetzung aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen der Komponenten der erfindungsgemäßen Zusammensetzung nötig. Hohe Löslichkeit lässt sich durch geeignete Substitution der entsprechenden Verbindungen erreichen. Das Verarbeiten aus Lösung hat den Vorteil, dass die Schicht enthaltend die erfindungsgemäße Zusammensetzung sehr einfach und kostengünstig aufgebracht werden kann. Diese Technik eignet sich insbesondere für die Massenproduktion organischer elektronischer Vorrichtungen.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können auf organische Elektrolumineszenzvorrichtungen angewandt werden.

Ein weiterer Gegenstand der Erfindung ist daher ein Verfahren zur Herstellung einer organischen elektronischen Vorrichtung enthaltend eine erfindungsgemäße Zusammensetzung, wie zuvor beschrieben oder bevorzugt beschrieben, dadurch gekennzeichnet, dass mindestens eine organische Schicht enthaltend eine erfindungsgemäße Zusammensetzung durch Gasphasenabscheidung, insbesondere mit einem Sublimationsverfahren und/oder mit einem OVPD (Organic Vapour Phase Deposition) Verfahren und/oder mit Hilfe einer Trägergassublimation, oder aus Lösung, insbesondere durch Spincoating oder mit einem Druckverfahren, aufgebracht wird.

Bei der Herstellung einer organischen elektronischen Vorrichtung mittels Gasphasenabscheidung bestehen grundsätzlich zwei Möglichkeiten, wie eine organische Schicht, die die erfindungsgemäße Zusammensetzung enthalten soll und die mehrere verschiedene Bestandteile umfassen kann, auf ein beliebiges Substrat aufgebracht bzw. aufgedampft werden kann. Zum einen können die verwendeten Materialien jeweils in einer Materialquelle vorgelegt und schließlich aus den verschiedenen Materialquellen verdampft werden ("co-evaporation"). Zum anderen können die verschiedenen Materialien vorgemischt ("premixed") und das Gemisch in einer einzigen Materialquelle vorgelegt werden, aus der es schließlich verdampft wird ("premix-evaporation"). Dadurch lässt sich auf einfache und schnelle Art und Weise das Aufdampfen einer Schicht mit gleichmäßiger Verteilung der Komponenten erreichen, ohne dass eine präzise Ansteuerung einer Vielzahl an Materialquellen notwendig ist.

Ein weiterer Gegenstand der Erfindung ist demzufolge ein Verfahren, dadurch gekennzeichnet, dass die mindestens eine Verbindung der Formel (1a) oder der Formel (1b), wie zuvor beschrieben oder als bevorzugt beschrieben,

und die mindestens eine Verbindung der Formel (2), wie zuvor beschrieben oder als bevorzugt beschrieben, nacheinander oder gleichzeitig aus mindestens zwei Materialquellen, gegebenenfalls mit weiteren Materialien, wie zuvor beschrieben oder bevorzugt beschrieben, aus der Gasphase abgeschieden werden und die organische Schicht bilden.

In einer bevorzugten Ausführungsform der vorliegenden Erfindung wird die mindestens eine organische Schicht mittels Gasphasenabscheidung aufgebracht, wobei die Bestandeile der Zusammensetzung vorgemischt und aus einer einzigen Materialquelle verdampft werden.

Ein weiterer Gegenstand der Erfindung ist demzufolge ein Verfahren, dadurch gekennzeichnet, dass die erfindungsgemäße Zusammensetzung, wie zuvor beschrieben oder bevorzugt beschrieben, als Materialquelle zur Gasphasenabscheidung genutzt wird, und gegebenenfalls mit weiteren Materialien, die organische Schicht bildet.

Ein weiterer Gegenstand der Erfindung ist ein Verfahren zur Herstellung einer organischen elektronischen Vorrichtung enthaltend eine erfindungsgemäße Zusammensetzung, wie zuvor beschrieben oder bevorzugt beschrieben, dadurch gekennzeichnet, dass die erfindungsgemäße Formulierung, wie zuvor beschrieben, verwendet wird, um die organische Schicht aufzubringen.

Die erfindungsgemäßen Zusammensetzungen bzw. die erfindungsgemäßen Vorrichtungen zeichnen sich durch folgende überraschende Vorteile gegenüber dem Stand der Technik aus:
Die Verwendung der erfindungsgemäßen Zusammensetzungen in organischen elektronischen Vorrichtungen, insbesondere in einer organischen Elektrolumineszenzvorrichtungen, und insbesondere in einer OLED oder OLEC, führt zu deutlichen Steigerungen der Lebensdauer der Vorrichtungen.

Wie im nachfolgend angegebenen Beispiel 1 ersichtlich, lassen sich durch den Einsatz von Hostmaterialien der Formel (1a) oder der Formel (1b) allein, beispielsweise der Verbindung benutzt in der OLED V1, bei mittleren Emitterkonzentrationen in der EML von 12% gute Spannungen und Effizienzen erreichen. Die Lebensdauer der Bauteile ist jedoch gering.

Eine Verbesserung der Lebensdauer zusammen mit einer verbesserten Bauteileffizienz kann durch die erfindungsgemäße Kombination der Verbindungen der Formel (1a) oder der Formel (1b), wie zuvor beschrieben, mit Verbindungen der Formel (2), wie zuvor beschrieben, erreicht werden.

Diese Verbesserung der Lebensdauer und verbesserter Bauteileffizienz kann bevorzugt durch die erfindungsgemäße Kombination der Verbindungen der Formel (1a) oder der Formel (1b), wie zuvor beschrieben, mit Verbindungen der Formel (2), wie zuvor beschrieben, bei Emitterkonzentrationen von 2 bis 15 Gew.-% in der Emissionsschicht erreicht werden.

Dieser Vorteil wird stellvertretend und repräsentativ für Verbindungen der Formel (1a) durch Verwendung der Verbindung 1 (abgekürzt CbzT2) mit dem Triphenylenderivat 9 (abgekürzt als WBG1) in den Beispielen E2 und E4 bei einer Emitterkonzentration von 12% gezeigt.

Selbst mit einer geringen Emitterkonzentration von lediglich 7% in der EML, bei der die Lebensdauer einer OLED typischerweise sinkt, sind die erzielten Lebensdauern der erfindungsgemäßen Kombinationen noch deutlich gegenüber dem Stand der Technik verbessert. Dies wird stellvertretend und repräsentativ für Verbindungen der Formel (1a) durch Verwendung der Verbindung 1 (abgekürzt CbzT2) mit dem Triphenylenderivat 9 (abgekürzt als WBG1) in den Beispielen E1 und E3 bei einer Emitterkonzentration von 7% gezeigt.

Der Unterschied zu der Offenbarung der WO 2015/192941 mit den Daten E21 liegt in der Struktur des bipolaren Hosts der Formel (1a) oder der Formel (1b). Die Kombination mit einem gelben Emitter zeigt eine hohe Lebensdauer. Dem Fachmann ist bekannt, dass die Lebensdauer einer elektrolumineszenten Vorrichtung enthaltend einen gelben Emitter in der Regel höher ist, als enthaltend einen grünen Emitter. Für den Fachmann nicht vorhersehbar, führt die Strukturänderung zu Verbindungen der Formel (1a) oder der Formel (1b), wie zuvor beschrieben oder als bevorzugt beschrieben, insbesondere zu den Verbindungen 1 bis 8, zu einer Verbesserung der Lebensdauer von elektronischen Vorrichtungen, insbesondere von OLEDs, sogar enthaltend einen grünen Emitter. Die Verbesserung wird deutlich, da die Lebensdauer und die Bauteileffizienz im Vergleich zum Stand der Technik erhöht wird.

Die erfindungsgemäßen Zusammensetzungen eignen sich sehr gut für den Einsatz in einer Emissionsschicht und zeigen verbesserte Leistungsdaten, insbesondere für die Lebensdauer, gegenüber Verbindungen aus dem Stand der Technik, wie zuvor beschrieben.

Die erfindungsgemäßen Zusammensetzungen können leicht prozessiert werden und eignen sich daher sehr gut für die Massenproduktion in der kommerziellen Anwendung.

Die erfindungsgemäßen Zusammensetzungen können vorgemischt und aus einer einzigen Materialquelle aufgedampft werden, so dass auf einfache und schnelle Art und Weise eine organische Schicht mit gleichmäßiger Verteilung der verwendeten Komponenten hergestellt werden kann.

Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden.

Alle Merkmale der vorliegenden Erfindung können in jeder Art miteinander kombiniert werden, es sei denn, dass sich bestimmte Merkmale und/oder Schritte sich gegenseitig ausschließen. Dies gilt insbesondere für bevorzugte

Merkmale der vorliegenden Erfindung. Gleichermaßen können Merkmale nicht wesentlicher Kombinationen separat verwendet werden (und nicht in Kombination).

Die Erfindung wird durch die nachfolgenden Beispiele näher erläutert

Allgemeine Methoden:

**Bestimmung von Orbitalenergien und elektronischer Zustände**

Die HOMO- und LUMO-Energien sowie das Triplettniveau und die Singulettniveaus der Materialien werden über quantenchemische Rechnungen bestimmt. Hierzu wird vorliegend das Programmpaket "Gaussian09, Revision D.01" (Gaussian Inc.) verwendet. Zur Berechnung organischer Substanzen ohne Metalle (mit Methode "org." bezeichnet) wird zuerst eine Geometrieoptimierung mit der semi-empirischen Methode AM1 (Gaussian-Eingabezeile "# AM1 opt") mit der Ladung (Charge) 0 und der Multiplizität (Multiplicity) 1 durchgeführt. Im Anschluss erfolgt auf Grundlage der optimierten Geometrie eine Energierechnung (single point) für den elektronischen Grundzustand und das Triplett-Niveau. Hierbei wird die TDDFT-Methode (time dependent density functional theory) B3PW91 mit dem Basissatz 6-31 G(d) (Gaussian-Eingabezeile "# B3PW91/6-31 G(d) td=(50-50,nstates=4)") verwendet (Ladung 0, Multiplizität 1). Für metallorganische Verbindungen (mit Methode "M-org." bezeichnet) wird die Geometrie mit der Methode Hartree-Fock und dem Basissatz LanL2MB (Gaussian-Eingabezeile "# HF/LanL2MB opt") optimiert (Ladung 0, Multiplizität 1). Die Energierechnung erfolgt, wie oben beschrieben, analog zu der der organischen Substanzen, mit dem Unterschied, dass für das Metallatom der Basissatz "LanL2DZ" und für die Liganden der Basissatz "6-31 G(d)" verwendet wird (Gaussian-Eingabezeile "#B3PW91/gen pseudo=lanl2 td=(50-50,nstates=4)"). Aus der Energierechnung erhält man das HOMO als das letzte mit zwei Elektronen besetze Orbital (Alpha occ. eigenvalues) und LUMO als das erste unbesetzte Orbital (Alpha virt. eigenvalues) in Hartree-Einheiten, wobei HEh und LEh für die HOMO Energie in Hartree-Einheiten beziehungsweise für die LUMO-Energie in Hartree-Einheiten steht. Daraus wird der anhand von Cyclovoltammetriemessungen kalibrierte HOMO- und LUMO-Wert in Elektronenvolt wie folgt bestimmt:

$$\text{HOMO (eV)} = (\text{HEh}*27.212)*0.8308-1.118;$$

$$\text{LUMO (eV)} = (\text{LEh}*27.212)*1.0658-0.5049.$$

Das Triplett-Niveau T1 eines Materials ist definiert als die relative Anregungsenergie (in eV) des Triplettzustands mit der niedrigsten Energie, der sich aus der quantenchemischen Energierechnung ergibt. Das Singulett-Niveau S1 eines Materials ist definiert als die relative Anregungsenergie (in eV) des Singulettzustands mit der zweitniedrigsten Energie, der sich aus der quantenchemischen Energierechnung ergibt.

Der energetisch niedrigste Singulettzustand wird als S0 bezeichnet. Die hierin beschriebene Methode ist unabhängig von dem verwendeten Softwarepaket und liefert immer dieselben Ergebnisse. Beispiele oft benutzter Programme für diesen Zweck sind "Gaussian09" (Gaussian Inc.) und Q-Chem 4.1 (Q-Chem, Inc.). Vorliegend wird zur Berechnung der Energien das Programmpaket "Gaussian09, Revision D.01" verwendet.

Beispiel 1: **Herstellung der OLEDs**

In den folgenden Beispielen E1 bis E4 (siehe Tabelle 6) wird der Einsatz der erfindungsgemäßen Materialkombinationen in OLEDs vorgestellt.

**Vorbehandlung für die Beispiele E1 - E4:** Glasplättchen, die mit strukturiertem ITO (Indium Zinn Oxid) der Dicke 50 nm beschichtet sind, werden vor der Beschichtung zunächst mit einem Sauerstoffplasma, gefolgt von einem Argonplasma, behandelt. Diese mit Plasma behandelten Glasplättchen bilden die Substrate, auf welche die OLEDs aufgebracht werden.

Die OLEDs haben prinzipiell folgenden Schichtaufbau: Substrat / Lochinjektionsschicht (HIL) / Lochtransportschicht (HTL) / Elektronenblockierschicht (EBL) / Emissionsschicht (EML) / optionale Lochblockier-schicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 6 zu entnehmen. Die zur Herstellung der OLEDs benötigten Materialien sind in Tabelle 7 gezeigt. Die Daten der OLEDs sind in Tabelle 8 aufgelistet. Das Beispiel V1 ist ein Vergleichsbeispiel enthaltend repräsentativ nur ein Hostmaterial der Formel (1a), die Beispiele E1 bis E4 zeigen Daten von erfindungsgemäßen OLEDs.

Alle Materialien werden in einer Vakuumkammer thermisch aufgedampft. Dabei besteht die Emissionsschicht immer aus mindestens einem Matrixmaterial (Hostmaterial, Wirtsmaterial), im Sinn der Erfindung den mindestens zwei Matrixmaterialien, und einem emittierenden Dotierstoff (Dotand, Emitter), der den Matrixmaterialien durch Coverdampfung in einem bestimmten Volumenanteil beigemischt wird. Eine Angabe wie CbzT2:WBG1:TEG1 (46%:47%:7%) bedeutet hierbei, dass das Material CbzT2 in einem Volumenanteil von 46%, WBG1 in einem Anteil von 47% und TEG1 in einem Anteil von 7% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (SE, gemessen in cd/A) und die externe Quanteneffizienz (EQE, gemessen in %) in Abhängigkeit der Leuchtdichte, berechnet aus Strom-Spannungs-Leuchtdichte-Kennlinien unter Annahme einer lambertschen Abstrahlcharakteristik sowie die Lebensdauer bestimmt. Die Elektrolumineszenzspektren werden bei einer Leuchtdichte von 1000 cd/$m^2$ bestimmt und daraus die CIE 1931 x und y Farbkoordinaten berechnet. Die Angabe U1000 in Tabelle 8 bezeichnet die Spannung, die für eine Leuchtdichte von 1000 cd/$m^2$ benötigt wird. SE1000 und EQE1000 bezeichnen die Stromeffizienz bzw. die externe Quanteneffizienz, die bei 1000cd/$m^2$ erreicht werden. Als Lebensdauer LD wird die Zeit definiert, nach der die Leuchtdichte bei Betrieb mit konstanter Stromdichte $j_0$ von der Startleuchtdichte auf einen gewissen Anteil L1 absinkt. Eine Angabe L1=80% in Tabelle 8 bedeutet, dass die in Spalte LD angegebene Lebensdauer der Zeit entspricht, nach der die Leuchtdichte auf 80% ihres Anfangswertes absinkt.

### Verwendung von erfindungsgemäßen Mischungen in OLEDs

Die erfindungsgemäßen Materialkombinationen können in der Emissionsschicht in phosphoreszierenden OLEDs eingesetzt werden. Die erfindungsgemäße Kombination der Verbindung CbzT2, entsprechend Verbindung **1,** mit WBG1 (entsprechend Verbindung **9)** wird in den Beispielen E1 bis E4 als Matrixmaterial in der Emissionsschicht eingesetzt.

Tabelle 6: Aufbau der OLEDs

| Bsp | HIL Dicke | HTL Dicke | EBL Dicke | EML Dicke | HBL Dicke | ETL Dicke | EIL Dicke |
|---|---|---|---|---|---|---|---|
| V1 | HATCN 5nm | SpMA1 215nm | SpMA2 20nm | CbzT2:TEG1 (88%:12%) 30nm | ST2 10nm | ST2:LiQ (50%: 50%) 30nm | LiQ 1nm |
| E1 | HATCN 5nm | SpMA1 215nm | SpMA2 20nm | CbzT2:WBG1:TEG1 (46%: 47%:7%) 30nm | ST2 10nm | ST2:LiQ (50%: 50%) 30nm | LiQ 1nm |
| E2 | HATCN 5nm | SpMA1 215nm | SpMA2 20nm | CbzT2:WBG1:TEG1 (22%: 66%:12%) 30nm | ST2 10nm | ST2:LiQ (50%: 50%) 30nm | LiQ 1nm |
| E3 | HATCN 5nm | SpMA1 215nm | SpMA2 20nm | CbzT2:WBG1:TEG2 (46%: 47%:7%) 30nm | ST2 10nm | ST2:LiQ (50%: 50%) 30nm | LiQ 1nm |
| E4 | HATCN 5nm | SpMA1 215nm | SpMA2 20nm | CbzT2:WBG1:TEG2 (22%: 66%:12%) 30nm | ST2 10nm | ST2:LiQ (50%: 50%) 30nm | LiQ 1nm |

Tabelle 7: Strukturformeln der Materialien für OLEDs

| | |
|---|---|
| 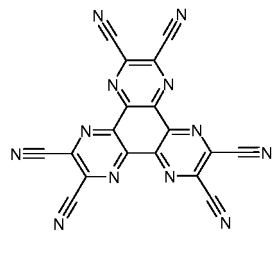 | 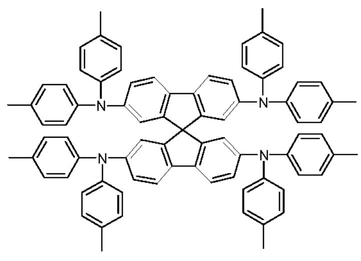 |
| HATCN | SpA1 |

(fortgesetzt)

| | |
|---|---|
| 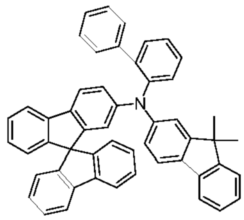 | 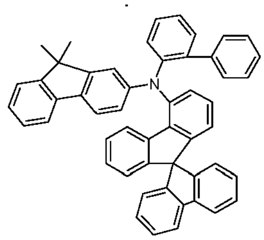 |
| SpMA1 | SpMA2 |
| 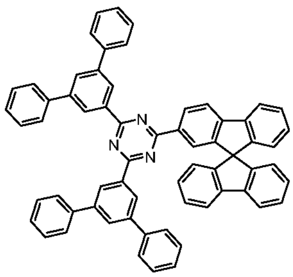 | 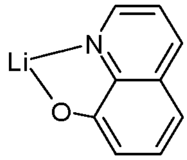 |
| ST2 | LiQ |
| 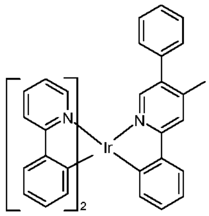 | 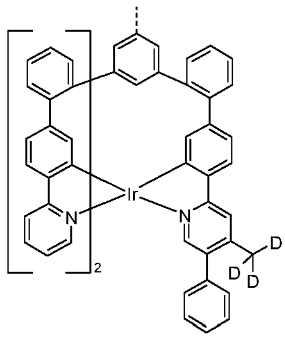 |
| TEG1 | TEG2 |
| 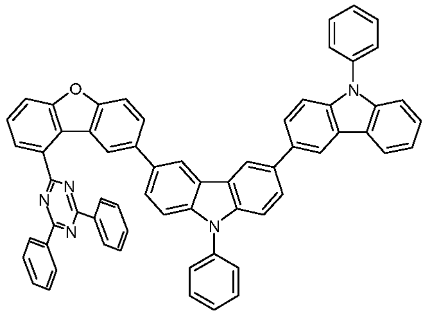 | 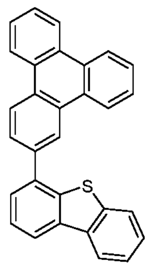 |
| CbzT2 | WBG1 |

Tabelle 8: Daten der OLEDs

| Bsp. | U1000 (V) | SE1000 (cd/A) | EQE 1000 (%) | CIE x/y bei 1000 cd/m$^2$ | $j_0$ (mA/cm$^2$) | L1 (%) | LD (h) |
|---|---|---|---|---|---|---|---|
| V1 | 3.0 | 60 | 16.4 | 0.33/0.63 | 20 | 80 | 660 |
| E1 | 3.3 | 76 | 20.6 | 0.32/0.64 | 20 | 80 | 819 |
| E2 | 3.7 | 64 | 17.4 | 0.32/0.64 | 20 | 80 | 1262 |

(fortgesetzt)

| Bsp. | U1000 (V) | SE1000 (cd/A) | EQE 1000 (%) | CIE x/y bei 1000 cd/m$^2$ | $j_0$ (mA/cm$^2$) | L1 (%) | LD (h) |
|---|---|---|---|---|---|---|---|
| E3 | 3.4 | 77 | 20.5 | 0.39/0.59 | 20 | 80 | 1576 |
| E4 | 3.8 | 76 | 19.9 | 0.38/0.60 | 20 | 80 | 3017 |

Eine ausgezeichnete Verbesserung der Lebensdauer und der Effizienz bei hohen und vor allem niedrigen Emitterkonzentration (12% oder 7%) in der EML erhält man durch die spezielle Kombination mit dem Emitter TEG1. In Kombination mit dem Emitter TEG2 (E3 und E4) lassen sich die Lebensdauern nochmals deutlich verbessern.

Die folgenden erfindungsgemäßen Mischungen zeigen ähnliches Verhalten in der elektronischen Vorrichtung:

| Carabzol-Triazin (CbzT) | Triphenylen (WBG) |
|---|---|
| 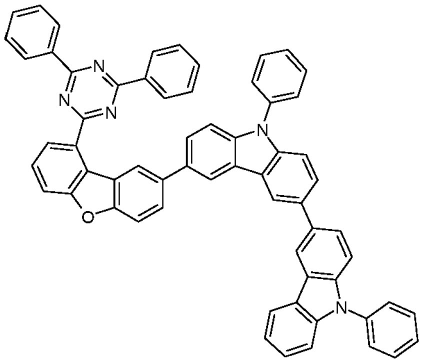 **1** | 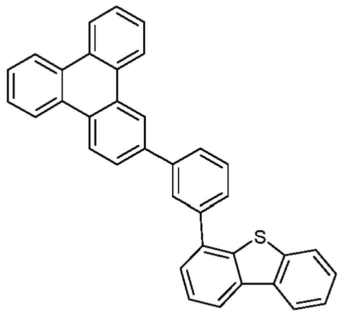 |
| 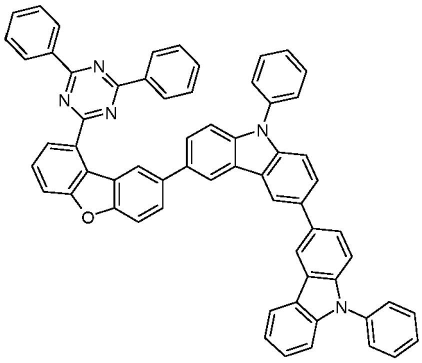 **1** | 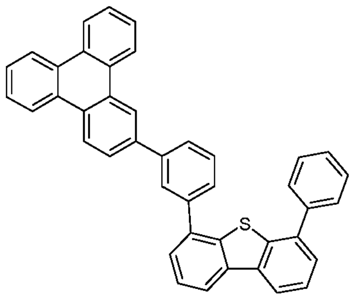 |
| 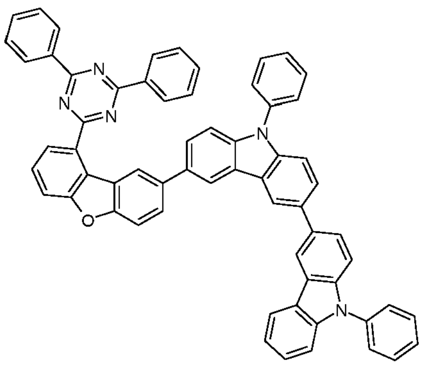 **1** | 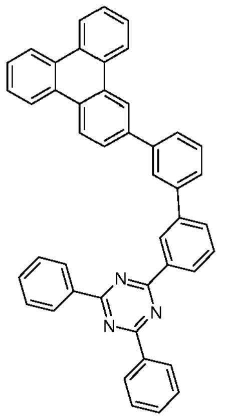 |

(fortgesetzt)

| Carabzol-Triazin (CbzT) | Triphenylen (WBG) |
|---|---|
| 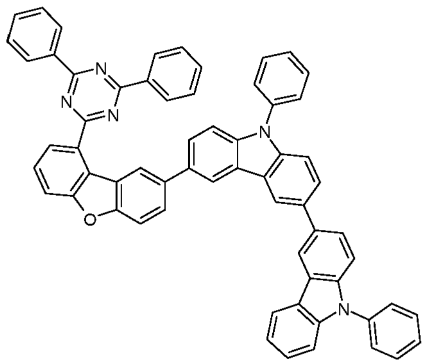 1 | 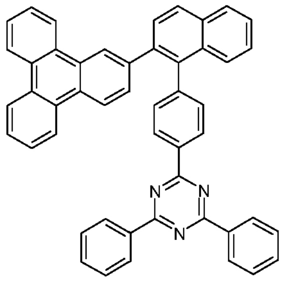 |
| 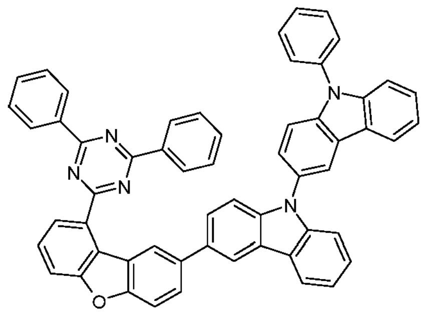 8 | 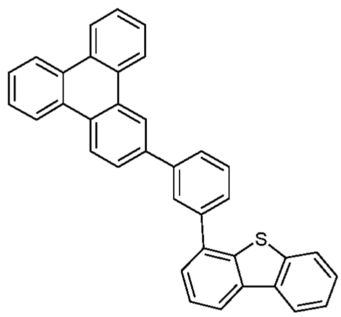 |
| 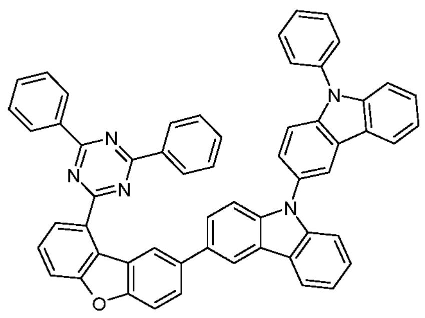 8 | 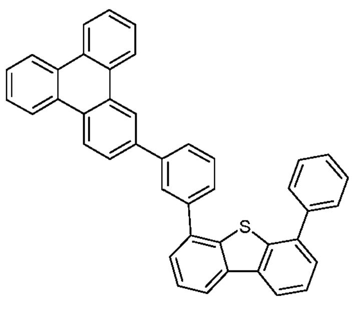 |
| 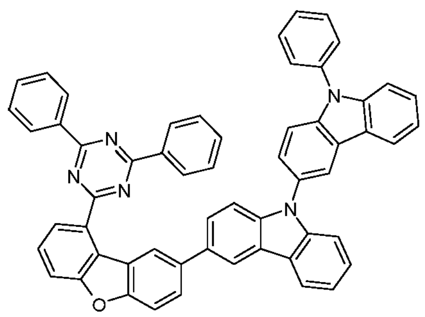 8 | 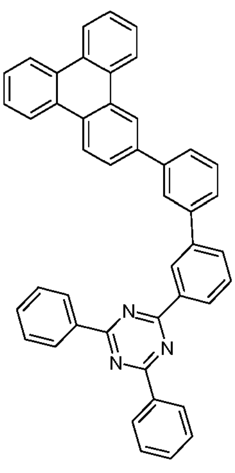 |

(fortgesetzt)

| Carabzol-Triazin (CbzT) | Triphenylen (WBG) |
|---|---|
| 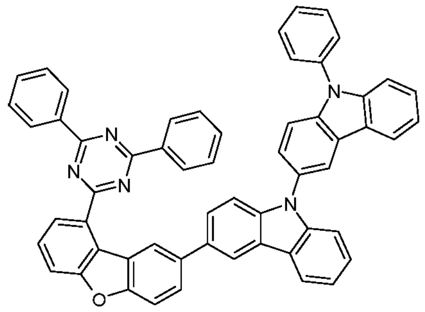 8 | 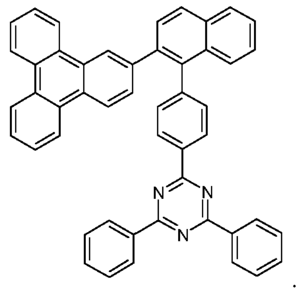 |

Beispiel 2: Synthese der Verbindung 1 (CbzT2)

**a) 6-Brom-2-fluor-2'-methoxy-biphenyl**

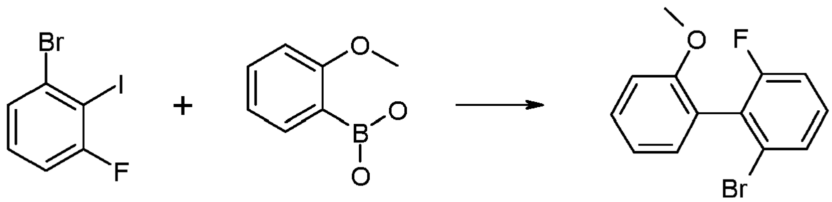

200 g (664 mmol) 1-Brom-3-fluor-2-iodbenzol, 101 g (664 mmol) 2-Methoxyphenylboronsäure und 137.5 g (997 mmol) Natriumtetraborat werden in 1000 mL THF und 600 ml Wasser gelöst und entgast. Es wird mit 9.3 g (13.3 mmol) Bis(triphenylphosphin)palladium(II)chlorid und 1 g (20 mmol) Hydraziniumhydroxid versetzt. Die Reaktionsmischung wird anschließend bei 70 °C für 48 h unter Schutzgasatmosphäre gerührt. Die abgekühlte Lösung wird mit Toluol aufgestockt, mehrmals mit Wasser gewaschen, getrocknet und eingeengt. Das Produkt wird via Säulenchromatographie an Kieselgel mit Toluol/Heptan (1:2) gereinigt. Ausbeute: 155 g (553 mmol), 83 % der Theorie.

**b) 6'-Brom-2'-fluor-biphenyl-2-ol**

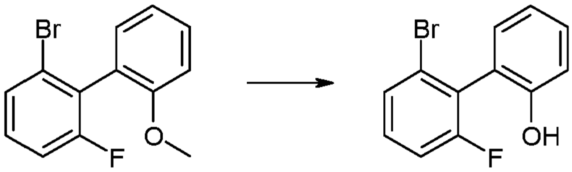

112 g (418 mmol) 6-Brom-2-fluor-2'-methoxy-biphenyl werden in 2 L Dichloromethan gelöst und auf 5 °C gekühlt. Zu dieser Lösung wird innerhalb von 90 min. 41.01 ml (431 mmol) Bortribromid zugetropft und über Nacht weiter gerührt. Das Gemisch wird im Anschluss langsam mit Wasser versetzt, die organische Phase dreimal mit Wasser gewaschen, über $Na_2SO_4$ getrocknet, einrotiert und chromatographisch gereinigt. Ausbeute: 104 g (397 mmol), 98 % der Theorie.

**c) 1-Brom-dibenzofuran**

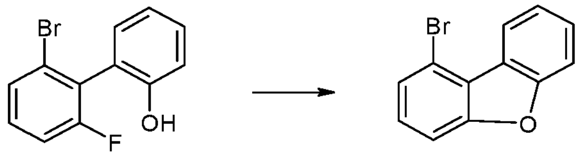

111 g (416 mmol) 6'-Brom-2'-fluor-biphenyl-2-ol werden in 2 L DMF (max. 0.003 % $H_2O$)SeccoSolv® gelöst und auf 5 °C gekühlt. Zu dieser Lösung wird portionsweise 20 g (449 mmol) Natriumhydrid (60% Suspension in Paraffinöl) zugegeben, nach beendeter Zugabe 20 min. nachgerührt und dann für 45 min. auf 100 °C erhitzt. Das Gemisch wird nach dem Abkühlen langsam mit 500 ml Ethanol versetzt, einrotiert und dann chromatographisch gereinigt. Ausbeute: 90 g (367 mmol), 88.5 % der Theorie.

**d) Dibenzofuran-1-boronsäure**

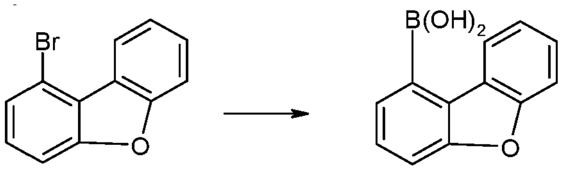

180 g (728 mmol) 1-Brom-dibenzofuran werden in 1500 mL trockenem THF gelöst und auf -78 °C gekühlt. Bei dieser Temperatur wird mit 305 mL (764 mmol / 2.5 M in Hexan) n-Butyllithium innerhalb von ca. 5 min. versetzt und anschließend für 2.5 h bei -78 °C nachgerührt. Bei dieser Temperatur wird mit 151 g (1456 mmol) Borsäuretrimethylester möglichst zügig versetzt und die Reaktion langsam auf Raumtemperatur kommen gelassen (ca. 18 h). Die Reaktionslösung wird mit Wasser gewaschen und der ausgefallene Feststoff und die organische Phase mit Toluol azeotrop getrocknet. Das Rohprodukt wird aus Toluol/Methylenchlorid bei ca. 40°C ausgerührt und abgesaugt. Ausbeute: 146 g (690 mmol), 95 % der Theorie.

**e) 2-Dibenzofuran-1-yl-4,6-diphenyl-[1,3,5]triazin**

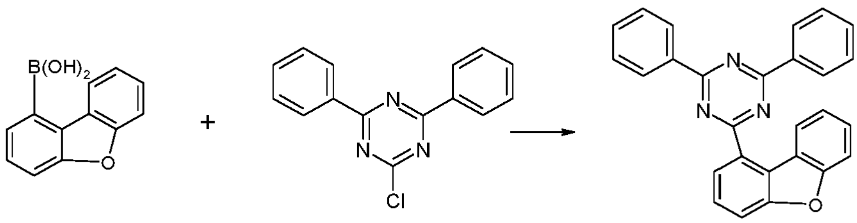

[3842-55-5]

23 g (110.0 mmol) Dibenzofuran-1-boronsäure, 29.5 g (110.0 mmol) 2-Chlor-4,6-diphenyl-1,3,5-triazin und 21 g (210.0 mmol) Natriumcarbonat werden in 500 mL Ethylenglycoldiaminether und 500 mL Wasser suspendiert. Zu dieser Suspension werden 913 mg (3.0 mmol) Tri-o-tolylphosphin und dann 112 mg (0.5 mmol) Palladium(II)acetat gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Der Rückstand wird aus Toluol und aus Dichlormethan /Heotan umkristallisiert. Die Ausbeute beträgt 37 g (94 mmol), entsprechend 87 % der Theorie.

**f) 2-(8-Brom-dibenzofuran-1-yl)-4,6-diphenyl-[1,3,5]triazin**

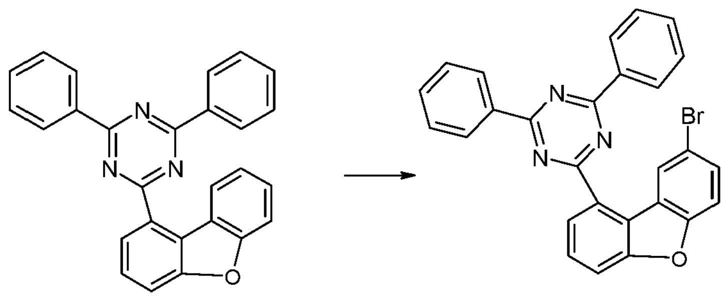

70 g (190.0 mmol) 2-Dibenzofuran-1-yl-4,6-diphenyl-[1,3,5]triazin werden in 2000 mL Essigsäure(100%) und 2000 mL Schwefelsäure (95-98%) suspendiert. Zu dieser Suspension werden portionsweise 34 g (190 mmol) NBS zugegeben und 2 h in Dunkelheit gerührt. Danach mit Wasser/Eis versetzt, der Feststoff abgetrennt und mit Ethanol nachgewaschen. Der Rückstand wird aus Toluol umkristallisiert. Die Ausbeute beträgt 80 g (167 mmol), entsprechend 87 % der Theorie.

**g) 3-[9-(4,6-Diphenyl-[1,3,5]triazin-2-yl)-dibenzofuran-2-yl]-9-phenyl-9H-carbazol** (nicht erfindungsgemäß)

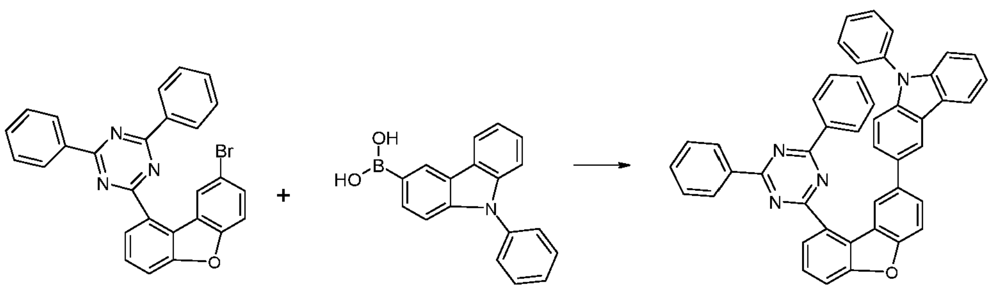

[854952-58-2] **1**

75 g (156 mmol) 2-(8-Brom-dibenzofuran-1-yl)-4,6-diphenyl-[1,3,5]-triazin, 50 g (172 mmol) N-Phenyl-carbazol-3-boronsäure [854952-58-2] und 36 g (340 mmol) Natriumcarbonat werden in 1000 mL Ethylenglycoldiaminether und 280 mL Wasser suspendiert. Zu dieser Suspension werden 1.8 g (1.5 mmol) Tetrakis-(triphenylphosphin)-palladium(0) zugegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Das Produkt wird via Säulenchromatographie an Kieselgel mit Toluol/Heptan (1:2) gereinigt und abschließend im Hochvakuum (p = 5 x $10^{-7}$ mbar) sublimiert (Reinheit 99,9%). Die Ausbeute beträgt 50 g (78mmol), entsprechend 50 % der Theorie.

Analog können folgende erfindungsgemäße Substanzen erhalten werden:

| | Edukt 1 | Edukt 2 | Produkt | Ausbeute |
|---|---|---|---|---|
| g1 | 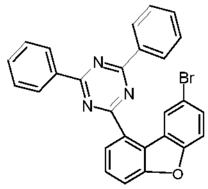 | 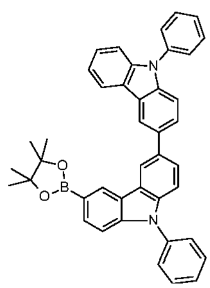 [1572537-61-1] | 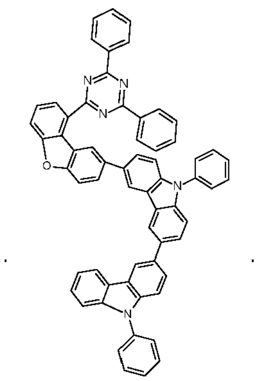 | 61% |

(fortgesetzt)

| | Edukt 1 | Edukt 2 | Produkt | Ausbeute |
|---|---|---|---|---|
| g2 | 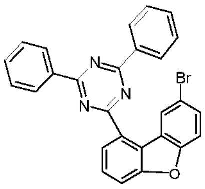 | 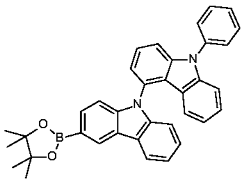 [1814934-97-8] | 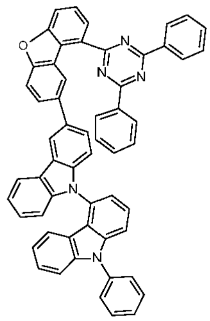 | 63% |
| g3 | 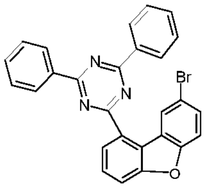 | 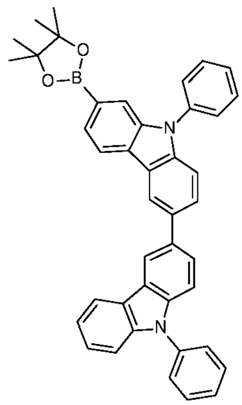 [1702359-51-0] | 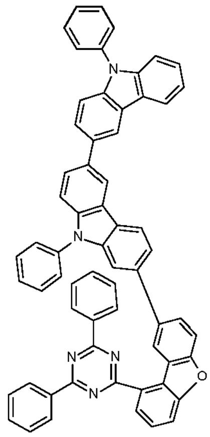 | 64% |
| g4 | 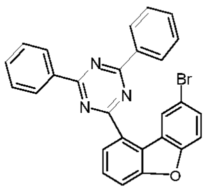 | 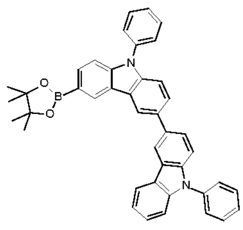 [1572537-61-1] | 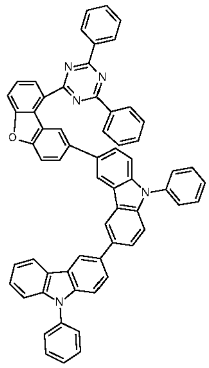 | 66% |
| g5 | 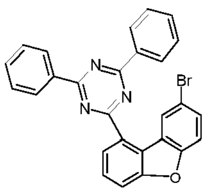 | 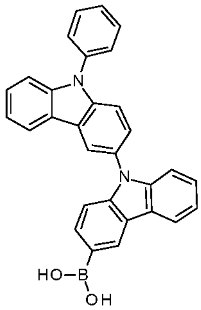 [1316311-24-6] | 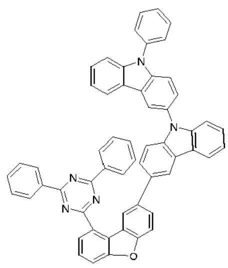 | 72% |

(fortgesetzt)

| | Edukt 1 | Edukt 2 | Produkt | Ausbeute |
|---|---|---|---|---|
| g6 | 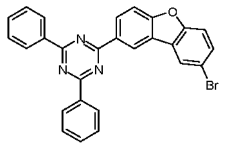 | 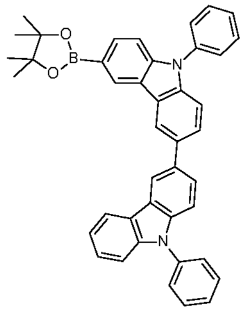 [1572537-61-1] | 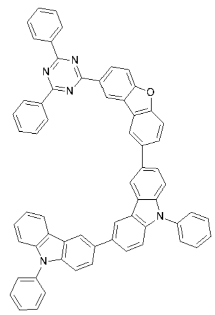 | 77% |
| g7 | 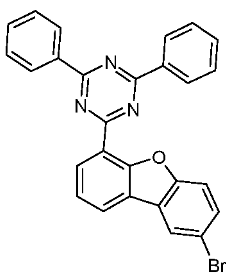 [1821221-55-9 ] | 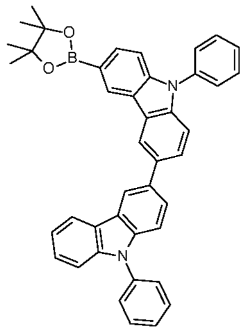 [1572537-61-1] | 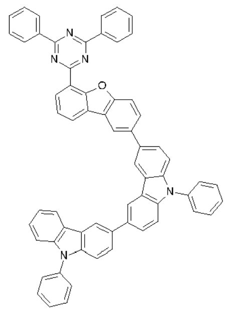 | 73% |
| g8 | 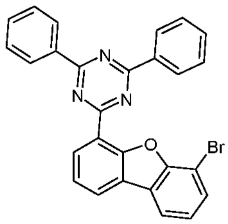 [1651196-06-3 ] | 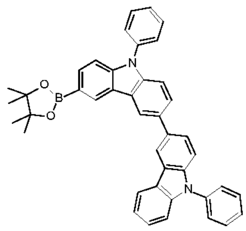 [1572537-61-1] | 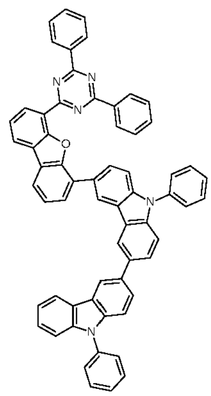 | 60% |
| g9 | 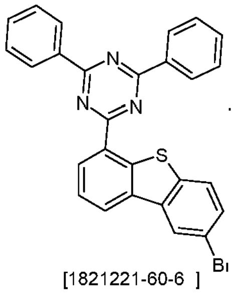 [1821221-60-6 ] | 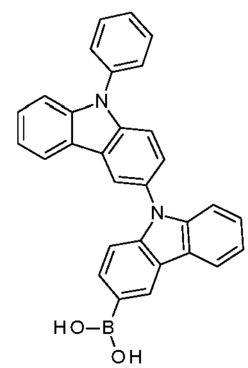 [1316311-24-6] | 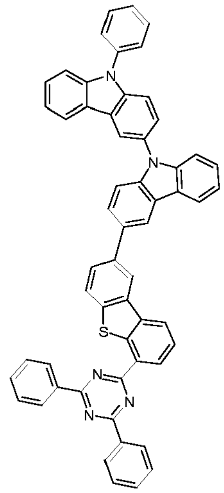 | 65% |

(fortgesetzt)

| | Edukt 1 | Edukt 2 | Produkt | Ausbeute |
|---|---|---|---|---|
| g10 | 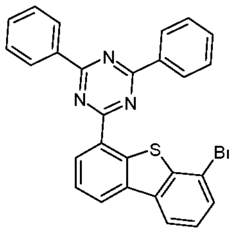 [11443049-88-4 ] | 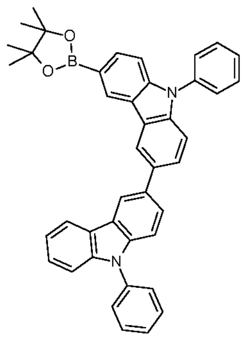 [1572537-61-1] | 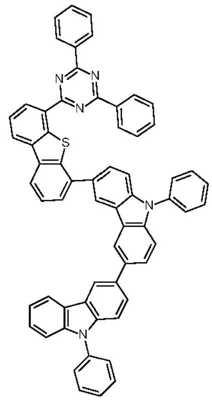 | 67% |
| g11 | 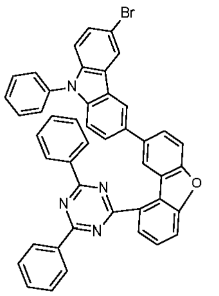 | 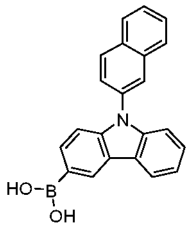 [1133057-98-3] | 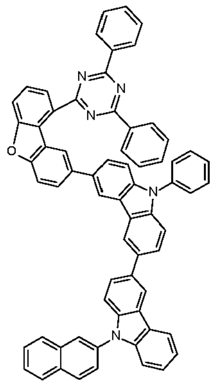 | 67% |
| g12 | 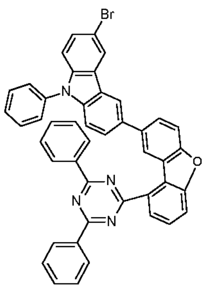 | 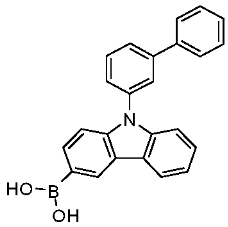 [1416814-68-0] | 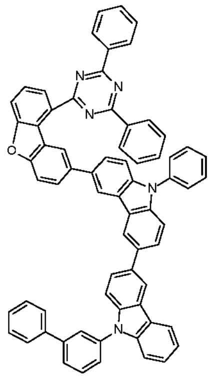 | 62% |

**h) 3-Bromo-6-[9-(4,6-diphenyl-[1,3,5]triazin-2-yl)-dibenzofuran-2-yl]-9-phenyl-9H-carbazol**

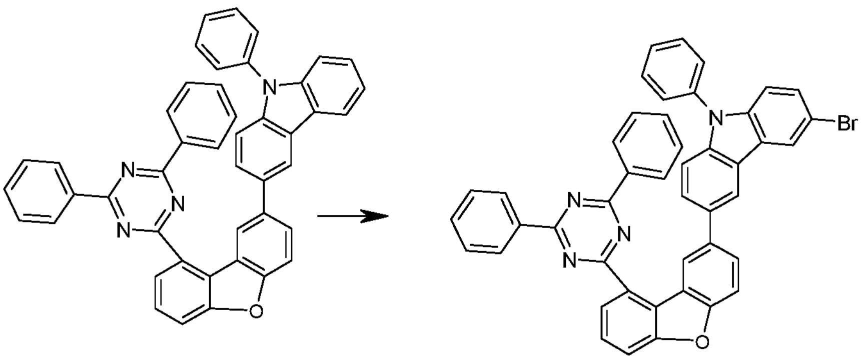

121 g (190.0 mmol) 3-[9-(4,6-Diphenyl-[1,3,5]triazin-2-yl)-dibenzofuran-2-yl]-9-phenyl-9H-carbazol werden in 2000 mL Essigsäure(100%) und 2000 mL Schwefelsäure (95-98%) suspendiert. Zu dieser Suspension werden portionsweise 34 g (190 mmol) NBS zugegeben und 2 h in Dunkelheit gerührt. Danach wird mit Wasser/Eis versetzt, der Feststoff abgetrennt und mit Ethanol nachgewaschen. Der Rückstand wird aus Toluol umkristallisiert. Die Ausbeute beträgt 103 g (143 mmol), entsprechend 76 % der Theorie.

Beispiel 3: Synthese von Verbindungen der Formel (1b)

**a) 9-Phenyl-3'-(9-{4-phenyl-6-[(E)-((Z)-1-propenyl)-buta-1,3-dienyl]-[1,3,5]triazin-2-yl}-dibenzofuran-2-yl)-9H-[3,9']bicarbazoly**

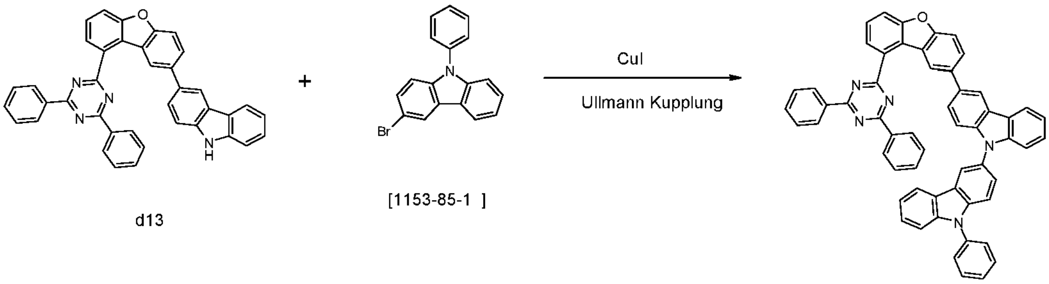


d13

[1153-85-1 ]

16,9 g (30 mmol) 3-[9-(4,6-Diphenyl-[1,3,5]triazin-2-yl)-dibenzofuran-2-yl]-9H-carbazo, 9,9 g (31 mmol, 1.1 eq) 3-Bromo-9-phenyl-9H-carbazol und 12.1 g (12 mmol, 0.36 eq) Kupfer(I)iodid werden mit 150 g (706 mmol, 4 eq) Kaliumphosphat in 1 L 1,4-Dioxan suspendiert. Anschließend wird das Reaktionsgemisch für 30 Minuten entgast und unter Schutzgas 17.6 ml (147 mmol, 0.83 eq) *trans*-Cyclohexylamin zugegeben. Der Ansatz wird 12 h unter Rückfluss erhitzt und nach beendeter Reaktion mit Dichlormethan versetzt. Der ausgefallene Feststoff wird abgesaugt, in Toluol gelöst und über Kieselgel filtriert. Nach Entfernen des Lösungsmittels im Vakuum wird der Rückstand mehrmals aus Toluol/Heptan umkristallisiert und abschließend sublimiert. Es werden 12,9 g (16 mmol, 54 %) eines farblosen Feststoffs mit einer HPLC-Reinheit >99.9% erhalten.

Analog werden folgende Verbindungen dargestellt:

| | Edukt 1 | Edukt 2 | Produkt | Ausbeute [%] |
|---|---|---|---|---|
| 1e | 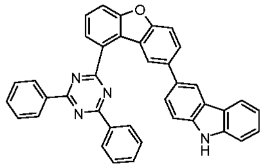 | 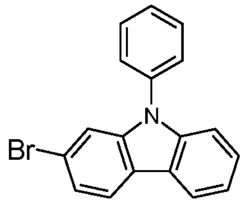 [94994-62-4 ] | 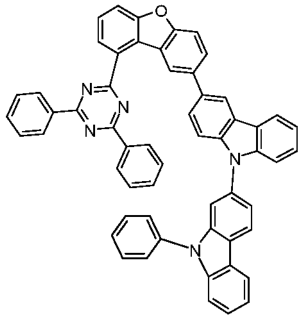 | 76 |
| 2e | 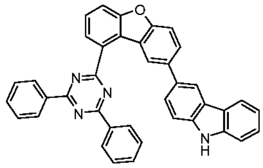 | 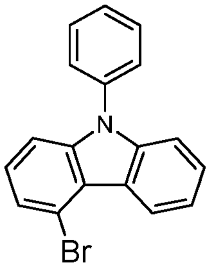 [1097884-37-1 ] | 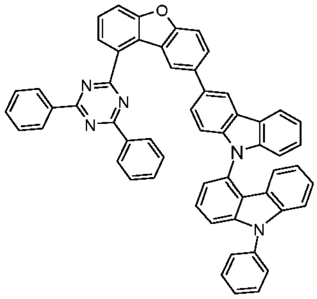 | 60 |
| 3e | 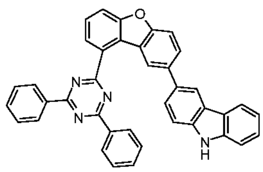 | 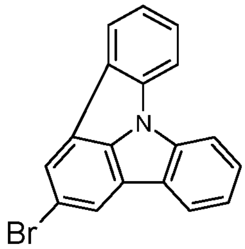 [1174032-81-5 | 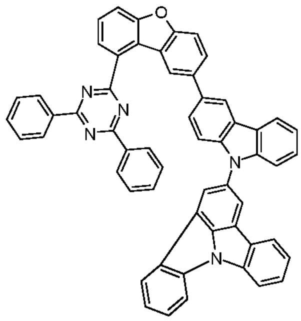 | 58 |
| 4e | 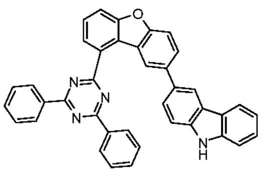 | 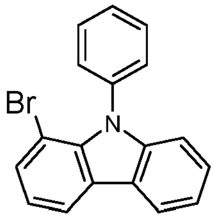 [1333002-37-1 ] | 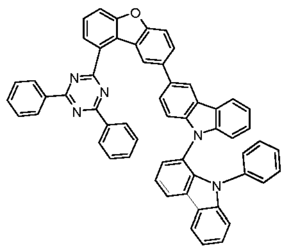 | 57 |
| 5e | 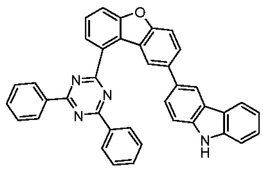 | 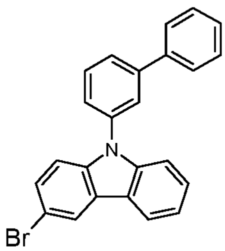 [1428551-28-3 ] | 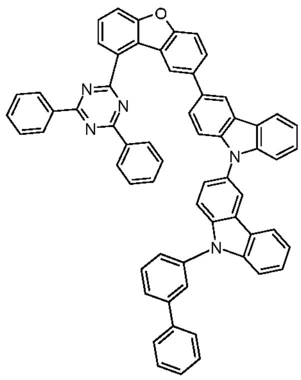 | 49 |

(fortgesetzt)

| | Edukt 1 | Edukt 2 | Produkt | Ausbeute [%] |
|---|---|---|---|---|
| 6e | 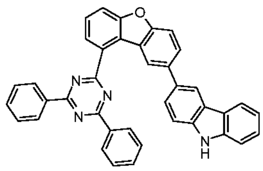 | 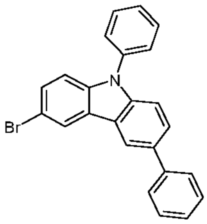 CAS 1160294-85-8 | 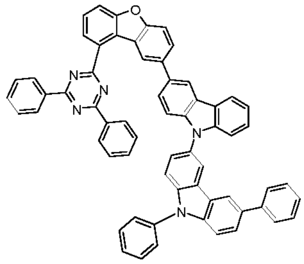 | 45 |

Beispiel 4:

Verbindung **9** ist literaturbekannt und wird analog zu WO 2009/021126, Beispiel 1 hergestellt.

Beispiel 5:

Analog zu Beispiel 4 können die Verbindungen **10** bis **17** hergestellt werden.

## Patentansprüche

**1.** Zusammensetzung umfassend mindestens eine Verbindung der Formel (1a) oder der Formel (1b) und mindestens eine Verbindung der Formel (2),

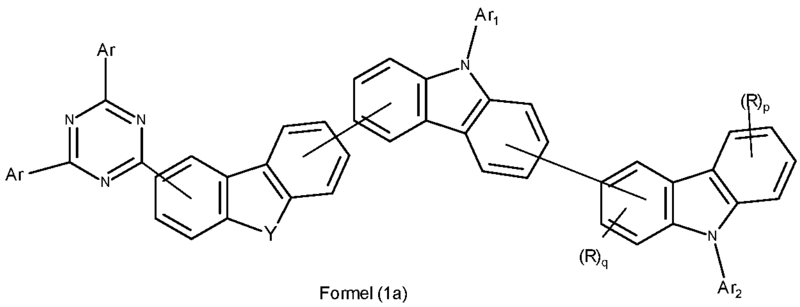

Formel (1a)

,

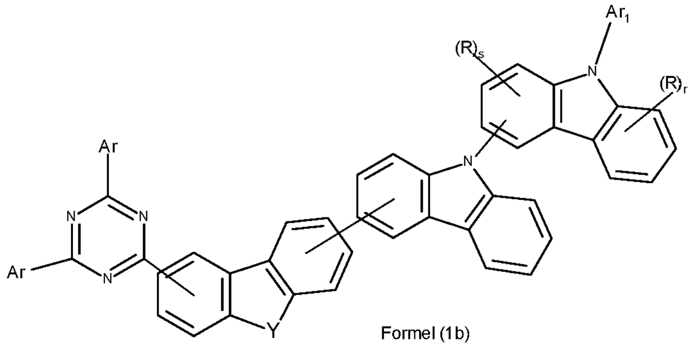

Formel (1b)

,

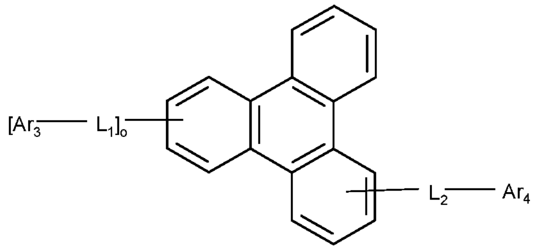

Formel (2)

,

wobei für die verwendeten Symbole und Indizes gilt:

Y ist O oder S;

Ar, $Ar_1$, $Ar_2$ sind jeweils unabhängig voneinander bei jedem Auftreten eine Aryl- oder Heteroarylgruppe mit 5 bis 40 Ringatomen, die mit einem oder mehreren Resten $R^3$ substituiert sein kann oder ein aromatisches oder heteroaromatisches Ringsystem mit 6 bis 40 Ringatomen, das mit einem oder mehreren Resten $R^3$ substituiert sein kann;

p, q sind jeweils unabhängig voneinander 0, 1, 2, 3 oder 4;

s, r sind jeweils unabhängig voneinander 0, 1, 2, 3 oder 4;

R ist bei jedem Auftreten gleich oder verschieden ausgewählt aus der Gruppe bestehend aus D, F, Cl, Br, I, CN, $NO_2$, N(Ar)2, $N(R^2)_2$, C(=O)Ar, $C(=O)R^2$, $P(=O)(Ar)_2$, $P(Ar)_2$, $B(Ar)_2$, $Si(Ar)_3$, $Si(R^2)_3$, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten $R^2$ substituiert sein kann, wobei eine oder mehrere nichtbenachbarte $CH_2$-Gruppen durch $R^2C=CR^2$, $Si(R^2)_2$, C=O, C=S, $C=NR^2$, $P(=O)(R^2)$, SO, $SO_2$, $NR^2$, O, S oder $CONR^2$ ersetzt sein können und wobei ein oder mehrere H-Atome durch D, F, Cl, Br, I, CN oder $NO_2$ ersetzt sein können, einem aromatischen oder heteroaromatischen Ringsystem mit 5 bis 40 Ringatomen, das jeweils mit einem oder mehreren Resten $R^2$ substituiert sein kann, einer Aryloxy- oder Heteroaryloxygruppe mit 5 bis 40 Ringatomen, die mit einem oder mehreren Resten $R^2$ substituiert sein kann, oder einer Aralkyl- oder Heteroaralkylgruppe mit 5 bis 40 Ringatomen, die mit einem oder mehreren Resten $R^2$ substituiert sein kann; dabei kann maximal ein Substituent R zusammen mit $Ar_1$ ein monocyclisches oder polycyclisches, aliphatisches, aromatisches oder heteroaromatisches Ringsystem bilden, das mit einem oder mehreren Resten $R^2$ substituiert sein kann;

o ist 0 oder 1;

$L_1$, $L_2$ sind bei jedem Auftreten gleich oder verschieden eine Einfachbindung oder ein aromatisches oder heteroaromatisches Ringsystem mit 5 bis 30 Ringatomen, das durch einen oder mehrere Reste $R^3$ substituiert sein kann;

$Ar_3$ und $Ar_4$ sind jeweils unabhängig voneinander ein aromatisches oder heteroaromatisches Ringsystem mit 6 bis 40 Ringatomen, das mit einem oder mehreren Resten $R^1$ substituiert sein kann;

$R^1$ ist bei jedem Auftreten gleich oder verschieden ausgewählt aus der Gruppe bestehend aus H, D, F, Cl, Br, I, CN, $NO_2$, N(Ar)2, $N(R^2)_2$, C(=O)Ar, $C(=O)R^2$, $P(=O)(Ar)_2$, $P(Ar)_2$, $B(Ar)_2$, $Si(Ar)_3$, $Si(R^2)_3$, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten $R^2$ substituiert sein kann, wobei eine oder mehrere nichtbenachbarte $CH_2$-Gruppen durch $R^2C=CR^2$, $Si(R^2)_2$, C=O, C=S, $C=NR^2$, $P(=O)(R^2)$, SO, $SO_2$, $NR^2$, O, S oder $CONR^2$ ersetzt sein können und wobei ein oder mehrere H-Atome durch D, F, Cl, Br, I, CN oder $NO_2$ ersetzt sein können, einem aromatischen oder heteroaromatischen Ringsystem mit 5 bis 40 Ringatomen, das jeweils mit einem oder mehreren Resten $R^2$ substituiert sein kann, einer Aryloxy- oder Heteroaryloxygruppe mit 5 bis 40 Ringatomen, die mit einem oder mehreren Resten $R^2$ substituiert sein kann, oder einer Aralkyl- oder Heteroaralkylgruppe mit 5 bis 40 Ringatomen, die mit einem oder mehreren Resten $R^2$ substituiert sein kann; dabei können optional zwei Substituenten $R^1$, die an dasselbe Kohlenstoffatom oder an benachbarte Kohlenstoffatome gebunden sind, ein monocyclisches oder polycyclisches, aliphatisches, aromatisches oder heteroaromatisches Ringsystem bilden, das mit einem oder mehreren Resten $R^2$ substituiert sein kann;

$R^2$ ist bei jedem Auftreten gleich oder verschieden ausgewählt aus der Gruppe bestehend aus H, D, F, Cl, Br, I, CN, $NO_2$, $N(Ar)_2$, $NH_2$, $N(R^3)_2$, C(=O)Ar, C(=O)H, $C(=O)R^3$, $P(=O)(Ar)_2$, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten $R^3$ substituiert sein kann, wobei eine oder mehrere nicht-benachbarte $CH_2$-Gruppen durch HC=CH, $R^3C=CR^3$, C=C, $Si(R^3)_2$, $Ge(R^3)_2$, $Sn(R^3)_2$, C=O, C=S, C=Se, $C=NR^3$, $P(=O)(R^3)$,

SO, $SO_2$, NH, $NR^3$, O, S, CONH oder $CONR^3$ ersetzt sein können und wobei ein oder mehrere H-Atome durch D, F, Cl, Br, I, CN oder $NO_2$ ersetzt sein können, einem aromatischen oder heteroaromatischen Ringsystem mit 5 bis 60 Ringatomen, das jeweils mit einem oder mehreren Resten $R^3$ substituiert sein kann, einer Aryloxy- oder Heteroaryloxygruppe mit 5 bis 60 Ringatomen, die mit einem oder mehreren Resten $R^3$ substituiert sein kann, oder einer Kombination dieser Systeme, wobei optional zwei oder mehr benachbarte Substituenten $R^2$ ein monocyclisches oder polycyclisches, aliphatisches, aromatisches oder heteroaromatisches Ringsystem bilden können, das mit einem oder mehreren Resten $R^3$ substituiert sein kann;

$R^3$ ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann; dabei können zwei

oder mehr benachbarte Substituenten $R^3$ miteinander ein mono- oder polycyclisches, aliphatisches Ringsystem bilden.

**2.** Zusammensetzung nach Anspruch 1, **dadurch gekennzeichnet, dass** die Verbindung der Formel (2) der Formel (2a), (2b) oder (2c) entspricht,

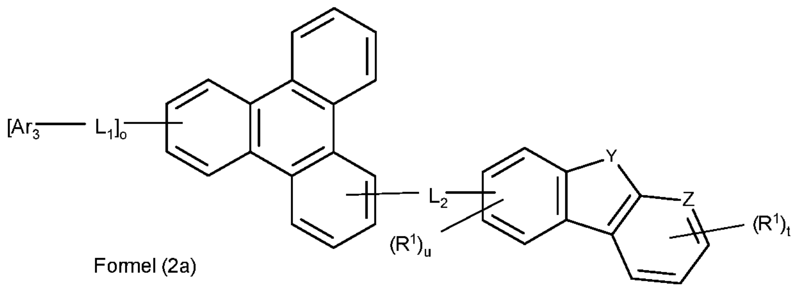

Formel (2a)

,

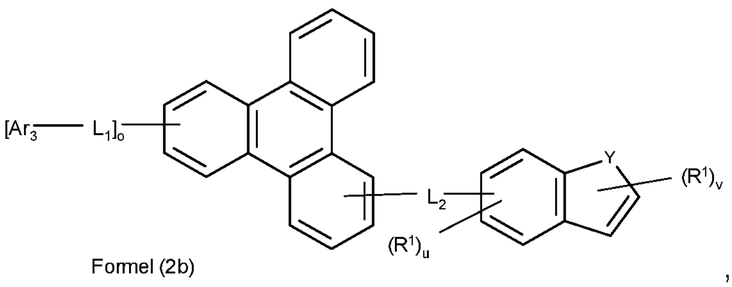

Formel (2b)

,

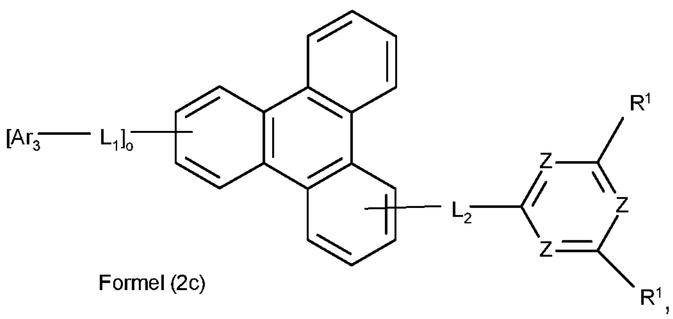

Formel (2c)

,

wobei die verwendeten Symbole und Indizes eine Bedeutung wie in Anspruch 1 haben,

Z jeweils unabhängig voneinander bei jedem Auftreten N oder $CR^1$ bedeutet, wobei $R^1$ eine in Anspruch 1 angegebene Bedeutung hat, Y in Formel (2a) oder (2b) O oder S bedeutet,

t und u jeweils unabhängig voneinander 0, 1, 2 oder 3 bedeuten,

v jeweils unabhängig voneinander 0, 1 oder 2 bedeutet.

3. Zusammensetzung nach Anspruch 1 oder 2, **dadurch gekennzeichnet, dass** die Zusammensetzung mindestens eine weitere Verbindung enthält, die ausgewählt ist aus der Gruppe bestehend aus Lochinjektionsmaterialien, Lochtransportmaterialien, Lochblockiermaterialien, wide-band-gap-Materialien, fluoreszierenden Emittern, phosphoreszierenden Emittern, Hostmaterialien, Elektronenblockiermaterialien, Elektronentransport-materialien und Elektroneninjektionsmaterialien, n-Dotanden und p-Dotanden.

4. Formulierung enthaltend eine Zusammensetzung nach einem oder mehreren der Ansprüche 1 bis 3 sowie mindestens ein Lösemittel.

5. Verwendung einer Zusammensetzung nach einem oder mehreren der Ansprüche 1 bis 3 in einer organischen elektronischen Vorrichtung.

6. Verwendung nach Anspruch 5, **dadurch gekennzeichnet, dass** die organische elektronische Vorrichtung aus der Gruppe von organischen integrierten Schaltungen (OICs), organischen Feld-Effekt-Transistoren (OFETs), organischen Dünnfilmtransistoren (OTFTs), organischen Elektrolumineszenzvorrichtungen, organischen Solarzellen (OSCs), organischen optischen Detektoren und organischen Photorezeptoren ausgewählt wird.

7. Organische elektronische Vorrichtung enthaltend mindestens eine Zusammensetzung nach einem oder mehreren der Ansprüche 1 bis 3.

8. Vorrichtung nach Anspruch 7, **dadurch gekennzeichnet, dass** sie aus der Gruppe von organischen integrierten Schaltungen (OICs), organischen Feld-Effekt-Transistoren (OFETs), organischen Dünnfilmtransistoren (OTFTs), organischen Elektrolumineszenzvorrichtungen, organischen Solarzellen (OSCs), organischen optischen Detektoren und organischen Photorezeptoren ausgewählt wird.

9. Vorrichtung nach Anspruch 7 oder 8, **dadurch gekennzeichnet, dass** es sich um eine Elektrolumineszenzvorrichtung handelt, die ausgewählt ist aus den organischen lichtemittierenden Transistoren (OLETs), organischen Feld-Quench-Devices (OFQDs), organischen lichtemittierenden elektrochemischen Zellen (OLECs, LECs, LEECs), organischen Laserdioden (O-Laser) und den organischen lichtemittierenden Dioden (OLEDs).

10. Vorrichtung nach einem oder mehreren der Ansprüche 7 bis 9, **dadurch gekennzeichnet, dass** diese die Zusammensetzung nach einem oder mehreren der Ansprüche 1 bis 3 in einer Emissionsschicht (EML), in einer Elektronentransportschicht (ETL), in einer Elektroneninjektionsschicht (EIL) und/oder in einer Lochblockierschicht (HBL) enthält.

11. Vorrichtung nach einem oder mehreren der Ansprüche 7 bis 10, **dadurch gekennzeichnet, dass** diese die Zusammensetzung nach einem oder mehreren der Ansprüche 1 bis 3 in der Emissionsschicht zusammen mit einem phosphoreszierenden Emitter enthält.

12. Verfahren zur Herstellung einer Vorrichtung nach einem oder mehreren der Ansprüche 7 bis 11, **dadurch gekennzeichnet, dass** mindestens eine organische Schicht enthaltend eine Zusammensetzung nach einem oder mehreren der Ansprüche 1 bis 3 durch Gasphasenabscheidung oder aus Lösung aufgebracht wird.

13. Verfahren nach Anspruch 12 **dadurch gekennzeichnet, dass** mindestens eine Verbindung der Formel (1a) oder der Formel (1b) und mindestens eine Verbindung der Formel (2), wie in einem der Ansprüche 1 oder 2 beschrieben, nacheinander oder gleichzeitig aus mindestens zwei Materialquellen, gegebenenfalls mit weiteren Materialien, aus der Gasphase abgeschieden werden und die organische Schicht bilden.

14. Verfahren nach Anspruch 12, **dadurch gekennzeichnet, dass** die Zusammensetzung nach einem oder mehreren der Ansprüche 1 bis 3 als Materialquelle zur Gasphasenabscheidung genutzt wird und die organische Schicht bildet.

15. Verfahren nach Anspruch 12, **dadurch gekennzeichnet, dass** die Formulierung nach Anspruch 4 verwendet wird, um die organische Schicht aufzubringen.

## Claims

1. Composition comprising at least one compound of the formula (1a) or of the formula (1b) and at least one compound of the formula (2),

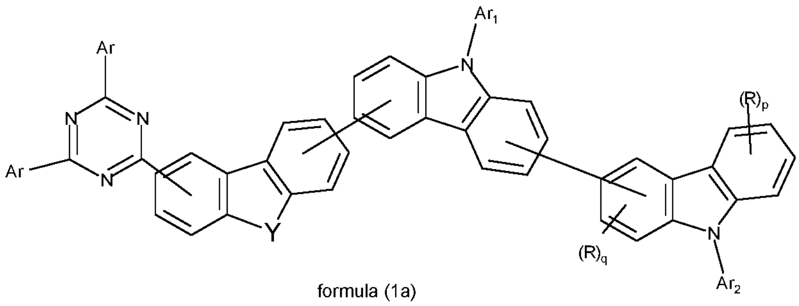

formula (1a) ,

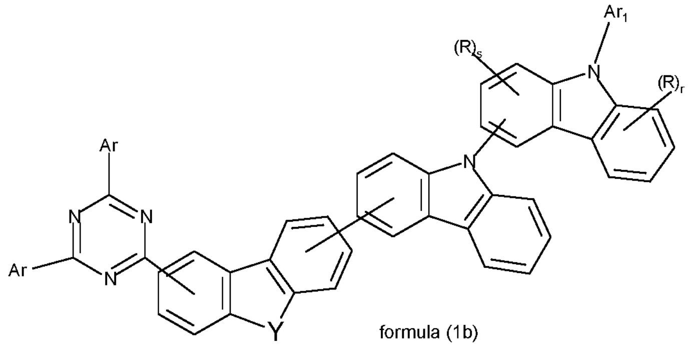

formula (1b) ,

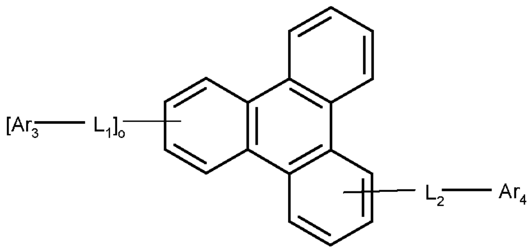

formula (2)

, where the following applies to the symbols and indices used:

Y is O or S;

Ar, $Ar_1$, $Ar_2$ are in each case, independently of one another on each occurrence, an aryl or heteroaryl group having 5 to 40 ring atoms, which may be substituted by one or more radicals $R^3$, or an aromatic or heteroaromatic ring system having 6 to 40 ring atoms, which may be substituted by one or more radicals $R^3$;

p, q are each case, independently of one another, 0, 1, 2, 3 or 4;

s, r are each case, independently of one another, 0, 1, 2, 3 or 4;

R is selected on each occurrence, identically or differently, from the group consisting of D, F, Cl, Br, I, CN, $NO_2$, $N(Ar)_2$, $N(R^2)_2$, C(=O)Ar, $C(=O)R^2$, $P(=O)(Ar)_2$, $P(Ar)_2$, $B(Ar)_2$, $Si(Ar)_3$, $Si(R^2)_3$, a straight-chain alkyl, alkoxy or thioalkyl group having 1 to 20 C atoms or a branched or cyclic alkyl, alkoxy or thioalkyl group having 3 to 20 C atoms or an alkenyl group having 2 to 20 C atoms, which may in each case be replaced by one or more radicals $R^2$, where one or more non-adjacent $CH_2$ groups may be replaced by $R^2C=CR^2$, $Si(R^2)_2$, C=O, C=S, $C=NR^2$, $P(=O)(R^2)$, SO, $SO_2$, $NR^2$, O, S or $CONR^2$ and where one or more H atoms may be replaced by D, F, Cl, Br, I, CN or $NO_2$, an aromatic or heteroaromatic ring system having 5 to 40 ring atoms, which may in each case be substituted by one or more radicals $R^2$, an aryloxy or heteroaryloxy group having 5 to 40 ring atoms, which may be substituted by one or more radicals $R^2$, or an aralkyl or heteroaralkyl group having 5 to 40 ring atoms, which may be substituted by one or more radicals $R^2$; a maximum of one substituent R may, together with $Ar_1$, form a monocyclic or polycyclic, aliphatic, aromatic or heteroaromatic ring system, which may be substituted by one or more radicals $R^2$;

o is 0 or 1;

$L_1$, $L_2$ are on each occurrence, identically or differently, a single bond or an aromatic or heteroaromatic ring system having 5 to 30 ring atoms, which may be substituted by one or more radicals $R^3$;

$Ar_3$ and $Ar_4$ are each case, independently of one another, an aromatic or heteroaromatic ring system having 6 to 40 ring atoms, which may be substituted by one or more radicals $R^1$;

$R^1$ is selected on each occurrence, identically or differently, from the group consisting of H, D, F, Cl, Br, I, CN, $NO_2$, $N(Ar)_2$, $N(R^2)_2$, C(=O)Ar, C(=O)$R^2$, P(=O)$(Ar)_2$, $P(Ar)_2$, $B(Ar)_2$, $Si(Ar)_3$, $Si(R^2)_3$, a straight-chain alkyl, alkoxy or thioalkyl group having 1 to 20 C atoms or a branched or cyclic alkyl, alkoxy or thioalkyl group having 3 to 20 C atoms or an alkenyl group having 2 to 20 C atoms, which may in each case be replaced by one or more radicals $R^2$, where one or more non-adjacent $CH_2$ groups may be replaced by $R^2C=CR^2$, $Si(R^2)_2$, C=O, C=S, C=$NR^2$, P(=O)($R^2$), SO, $SO_2$, $NR^2$, O, S or $CONR^2$ and where one or more H atoms may be replaced by D, F, Cl, Br, I, CN or $NO_2$, an aromatic or heteroaromatic ring system having 5 to 40 ring atoms, which may in each case be substituted by one or more radicals $R^2$, an aryloxy or heteroaryloxy group having 5 to 40 ring atoms, which may be substituted by one or more radicals $R^2$, or an aralkyl or heteroaralkyl group having 5 to 40 ring atoms, which may be substituted by one or more radicals $R^2$; two substituents $R^1$ that are bonded to the same carbon atom or to adjacent carbon atoms may optionally form a monocyclic or polycyclic, aliphatic, aromatic or heteroaromatic ring system, which may be substituted by one or more radicals $R^2$;

$R^2$ is selected on each occurrence, identically or differently, from the group consisting of H, D, F, Cl, Br, I, CN, $NO_2$, $N(Ar)_2$, $NH_2$, $N(R^3)_2$, C(=O)Ar, C(=O)H, C(=O)$R^3$, P(=O)$(Ar)_2$, a straight-chain alkyl, alkoxy or thioalkyl group having 1 to 40 C atoms or a branched or cyclic alkyl, alkoxy or thioalkyl group having 3 to 40 C atoms or an alkenyl or alkynyl group having 2 to 40 C atoms, which may in each case be substituted by one or more radicals $R^3$, where one or more non-adjacent $CH_2$ groups may be replaced by HC=CH, $R^3C=CR^3$, C=C, $Si(R^3)_2$, $Ge(R^3)_2$, $Sn(R^3)_2$, C=O, C=S, C=Se, C=$NR^3$, P(=O)($R^3$), SO, $SO_2$, NH, $NR^3$, O, S, CONH or $CONR^3$ and where one or more H atoms may be replaced by D, F, Cl, Br, I, CN or $NO_2$, an aromatic or heteroaromatic ring system having 5 to 60 ring atoms, which may in each case be substituted by one or more radicals $R^3$, an aryloxy or heteroaryloxy group having 5 to 60 ring atoms, which may be substituted by one or more radicals $R^3$, or a combination of these systems, where two or more adjacent substituents $R^2$ may optionally form a monocyclic or polycyclic, aliphatic, aromatic or heteroaromatic ring system, which may be substituted by one or more radicals $R^3$;

$R^3$ is selected on each occurrence, identically or differently, from the group consisting of H, D, F, CN, an aliphatic hydrocarbon radical having 1 to 20 C atoms or an aromatic or heteroaromatic ring system having 5 to 30 ring atoms, in which one or more H atoms may be replaced by D, F, Cl, Br, I or CN and which may be substituted by one or more alkyl groups, in each case having 1 to 4 carbon atoms; two or more adjacent substituents $R^3$ may form a mono- or polycyclic, aliphatic ring system with one another.

2. Composition according to Claim 1, **characterised in that** the compound of the formula (2) corresponds to the formula (2a), (2b) or (2c),

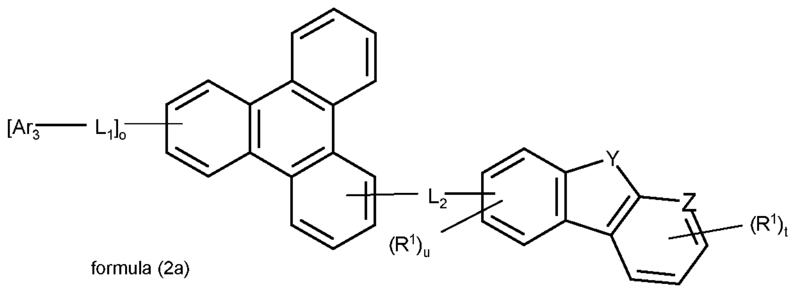

formula (2a)

,

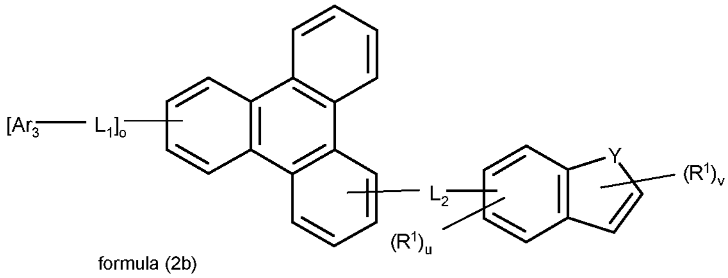

formula (2b) ,

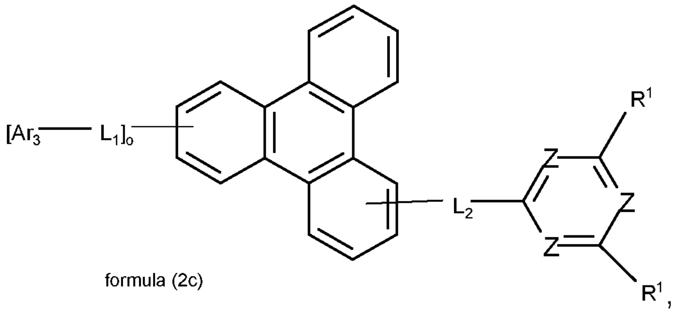

formula (2c)

where the symbols and indices used have a meaning as in Claim 1,

Z in each case, independently of one another on each occurrence, denotes N or $CR^1$, where $R^1$ has a meaning indicated in Claim 1,
Y in formula (2a) or (2b) denotes O or S,
t and u in each case, independently of one another, denote 0, 1, 2 or 3, v in each case, independently of one another, denotes 0, 1 or 2.

3. Composition according to Claim 1 or 2, **characterised in that** the composition comprises at least one further compound selected from the group consisting of hole-injection materials, hole-transport materials, hole-blocking materials, wide band-gap materials, fluorescent emitters, phosphorescent emitters, host materials, electron-blocking materials, electron-transport materials and electron-injection materials, n-dopants and p-dopants.

4. Formulation comprising a composition according to one or more of Claims 1 to 3 and at least one solvent.

5. Use of a composition according to one or more of Claims 1 to 3 in an organic electronic device.

6. Use according to Claim 5, **characterised in that** the organic electronic device is selected from the group of organic integrated circuits (OICs), organic field-effect transistors (OFETs), organic thin-film transistors (OTFTs), organic electroluminescent devices, organic solar cells (OSCs), organic optical detectors and organic photoreceptors.

7. Organic electronic device containing at least one composition according to one or more of Claims 1 to 3.

8. Device according to Claim 7, **characterised in that** it is selected from the group of organic integrated circuits (OICs), organic field-effect transistors (OFETs), organic thin-film transistors (OTFTs), organic electroluminescent devices, organic solar cells (OSCs), organic optical detectors and organic photoreceptors.

9. Device according to Claim 7 or 8, **characterised in that** it is an electroluminescent device selected from organic light-emitting transistors (OLETs), organic field-quench devices (OFQDs), organic light-emitting electrochemical cells (OLECs, LECs, LEECs), organic laser diodes (O-lasers) and organic light-emitting diodes (OLEDs).

10. Device according to one or more of Claims 7 to 9, **characterised in that** it contains the composition according to one or more of Claims 1 to 3 in an emission layer (EML), in an electron-transport layer (ETL), in an electron-injection layer (EIL) and/or in a hole-blocking layer (HBL).

11. Device according to one or more of Claims 7 to 10, **characterised in that** it contains the composition according to

one or more of Claims 1 to 3 in the emission layer together with a phosphorescent emitter.

**12.** Process for the production of a device according to one or more of Claims 7 to 11, **characterised in that** at least one organic layer comprising a composition according to one or more of Claims 1 to 3 is applied by gas-phase deposition or from solution.

**13.** Process according to Claim 12, **characterised in that** at least one compound of the formula (1a) or of the formula (1b) and at least one compound of the formula (2), as described in one of Claims 1 or 2, are deposited successively or simultaneously from at least two material sources, optionally with further materials, from the gas phase and form the organic layer.

**14.** Process according to Claim 12, **characterised in that** the composition according to one or more of Claims 1 to 3 is used as material source for the gas-phase deposition and forms the organic layer.

**15.** Process according to Claim 12, **characterised in that** the formulation according to Claim 4 is used to apply the organic layer.

## Revendications

**1.** Composition comprenant au moins un composé de formule (1a) ou de formule (1b) et au moins un composé de formule (2),

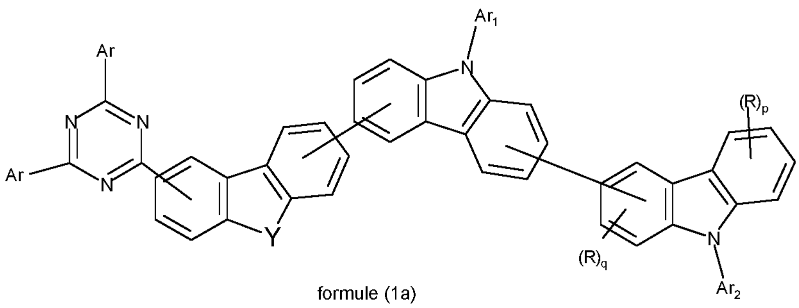

formule (1a) ,

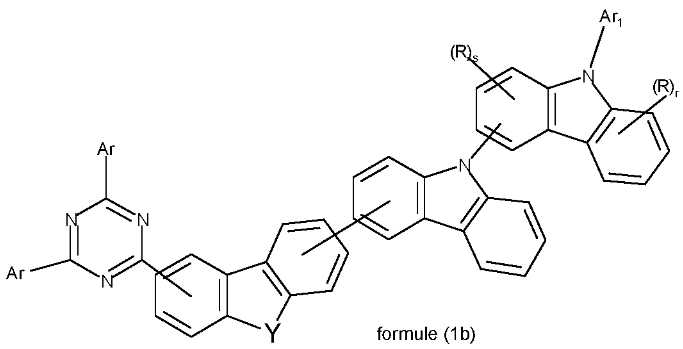

formule (1b) ,

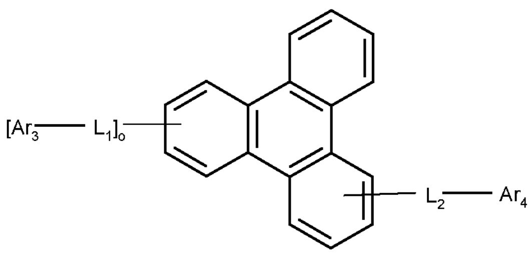

formule (2) ,

où ce qui suit s'applique aux symboles et indices utilisés :

Y est O ou S ;

Ar, $Ar_1$, $Ar_2$ sont dans chaque cas, indépendamment les uns des autres à chaque occurrence, un groupement aryle ou hétéroaryle ayant de 5 à 40 atomes de cycle, pouvant être substitué par un ou plusieurs radicaux $R^3$, ou un noyau aromatique ou hétéroaromatique ayant de 6 à 40 atomes de cycle, pouvant être substitué par un ou plusieurs radicaux $R^3$ ;

p, q valent dans chaque cas, indépendamment les uns des autres, 0, 1, 2, 3 ou 4;

s, r valent dans chaque cas, indépendamment les uns des autres, 0, 1, 2, 3 ou 4;

R est choisi à chaque occurrence, de manière identique ou différente, dans le groupe constitué par D, F, Cl, Br, I, CN, $NO_2$, $N(Ar)_2$, $N(R^2)_2$, C(=O)Ar, $C(=O)R^2$, $P(=O)(Ar)_2$, $P(Ar)_2$, $B(Ar)_2$, $Si(Ar)_3$, $Si(R^2)_3$, un groupement alkyle, alcoxy ou thioalkyle à chaîne linéaire ayant de 1 à 20 atomes de C ou un groupement alkyle, alcoxy ou thioalkyle ramifié ou cyclique ayant de 3 à 20 atomes de C ou un groupement alcényle ayant de 2 à 20 atomes de C, pouvant être dans chaque cas remplacé par un ou plusieurs radicaux $R^2$, où un ou plusieurs groupements $CH_2$ non adjacents peuvent être remplacés par $R^2C=CR^2$, $Si(R^2)_2$, C=O, C=S, $C=NR^2$, $P(=O)(R^2)$, SO, $SO_2$, $NR^2$, O, S ou $CONR^2$ et où un ou plusieurs atomes de H peuvent être remplacés par D, F, Cl, Br, I, CN ou $NO_2$, un noyau aromatique ou hétéroaromatique ayant de 5 à 40 atomes de cycle, pouvant être dans chaque cas substitué par un ou plusieurs radicaux $R^2$, un groupement aryloxy ou hétéroaryloxy ayant de 5 à 40 atomes de cycle, pouvant être substitué par un ou plusieurs radicaux $R^2$, ou un groupement aralkyle ou hétéroaralkyle ayant de 5 à 40 atomes de cycle, pouvant être substitué par un ou plusieurs radicaux $R^2$ ; un maximum d'un substituant R peut, conjointement avec $Ar_1$, former un noyau monocyclique ou polycyclique, aliphatique, aromatique ou hétéroaromatique, pouvant être substitué par un ou plusieurs radicaux $R^2$ ;

o vaut 0 ou 1 ;

$L_1$, $L_2$ sont à chaque occurrence, de manière identique ou différente, une liaison simple ou un noyau aromatique ou hétéroaromatique ayant de 5 à 30 atomes de cycle, pouvant être substitué par un ou plusieurs radicaux $R^3$ ;

$Ar_3$ et $Ar_4$ sont dans chaque cas, indépendamment l'un de l'autre, un noyau aromatique ou hétéroaromatique ayant de 6 à 40 atomes de cycle, pouvant être substitué par un ou plusieurs radicaux $R^1$ ;

$R^1$ est choisi à chaque occurrence, de manière identique ou différente, dans le groupe constitué par H, D, F, Cl, Br, I, CN, $NO_2$, $N(Ar)_2$, $N(R^2)_2$, C(=O)Ar, $C(=O)R^2$, $P(=O)(Ar)_2$, $P(Ar)_2$, $B(Ar)_2$, $Si(Ar)_3$, $Si(R^2)_3$, un groupement alkyle, alcoxy ou thioalkyle à chaîne linéaire ayant de 1 à 20 atomes de C ou un groupement alkyle, alcoxy ou thioalkyle ramifié ou cyclique ayant de 3 à 20 atomes de C ou un groupement alcényle ayant de 2 à 20 atomes de C, pouvant être dans chaque cas remplacé par un ou plusieurs radicaux $R^2$, où un ou plusieurs groupements $CH_2$ non adjacents peuvent être remplacés par $R^2C=CR^2$, $Si(R^2)_2$, C=O, C=S, $C=NR^2$, $P(=O)(R^2)$, SO, $SO_2$, $NR^2$, O, S ou $CONR^2$ et où un ou plusieurs atomes de H peuvent être remplacés par D, F, Cl, Br, I, CN ou $NO_2$, un noyau aromatique ou hétéroaromatique ayant de 5 à 40 atomes de cycle, pouvant être dans chaque cas substitué par un ou plusieurs radicaux $R^2$, un groupement aryloxy ou hétéroaryloxy ayant de 5 à 40 atomes de cycle, pouvant être substitué par un ou plusieurs radicaux $R^2$, ou un groupement aralkyle ou hétéroaralkyle ayant de 5 à 40 atomes de cycle, pouvant être substitué par un ou plusieurs radicaux $R^2$ ; deux substituants $R^1$ qui sont liés au même atome de carbone ou à des atomes de carbone adjacents peuvent éventuellement former un noyau monocyclique ou polycyclique, aliphatique, aromatique ou hétéroaromatique, pouvant être substitué par un ou plusieurs radicaux $R^2$ ;

$R^2$ est choisi à chaque occurrence, de manière identique ou différente, dans le groupe constitué par H, D, F, Cl, Br, I, CN, $NO_2$, $N(Ar)_2$, $NH_2$, $N(R^3)_2$, C(=O)Ar, C(=O)H, $C(=O)R^3$, $P(=O)(Ar)_2$, un groupement alkyle, alcoxy ou thioalkyle à chaîne linéaire ayant de 1 à 40 atomes de C ou un groupement alkyle, alcoxy ou thioalkyle ramifié ou cyclique ayant de 3 à 40 atomes de C ou un groupement alcényle ou alcynyle ayant de 2 à 40 atomes de C, pouvant être dans chaque cas substitué par un ou plusieurs radicaux $R^3$, où un ou plusieurs groupements $CH_2$ non adjacents peuvent être remplacés par HC=CH, $R^3C=CR^3$, $C\equiv C$, $Si(R^3)_2$, $Ge(R^3)_2$, $Sn(R^3)_2$, C=O, C=S, C=Se, $C=NR^3$, $P(=O)(R^3)$, SO, $SO_2$, NH, $NR^3$, O, S, CONH ou $CONR^3$ et où un ou plusieurs atomes de H peuvent être remplacés par D, F, Cl, Br, I, CN ou $NO_2$, un noyau aromatique ou hétéroaromatique ayant de 5 à 60 atomes de cycle, pouvant être dans chaque cas substitué par un ou plusieurs radicaux $R^3$, un groupement aryloxy ou hétéroaryloxy ayant de 5 à 60 atomes de cycle, pouvant être substitué par un ou plusieurs radicaux $R^3$, ou une combinaison de ces noyaux, où deux, ou plus, substituants $R^2$ adjacents peuvent éventuellement former un noyau monocyclique ou polycyclique, aliphatique, aromatique ou hétéroaromatique, pouvant être substitué par un ou plusieurs radicaux $R^3$ ;

$R^3$ est choisi à chaque occurrence, de manière identique ou différente, dans le groupe constitué par H, D, F, CN, un radical hydrocarboné aliphatique ayant de 1 à 20 atomes de C ou un noyau aromatique ou hétéroaromatique ayant de 5 à 30 atomes de cycle, où un ou plusieurs atomes de H peuvent être remplacés par D, F, Cl, Br, I ou CN et pouvant être substitué par un ou plusieurs groupements alkyle, dans chaque cas ayant de 1 à 4 atomes de carbone ; deux, ou plus, substituants $R^3$ adjacents peuvent former un noyau mono- ou polycyclique, aliphatique les uns avec les autres.

**2.** Composition selon la revendication 1, **caractérisée en ce que** le composé de formule (2) correspond à la formule (2a), (2b) ou (2c),

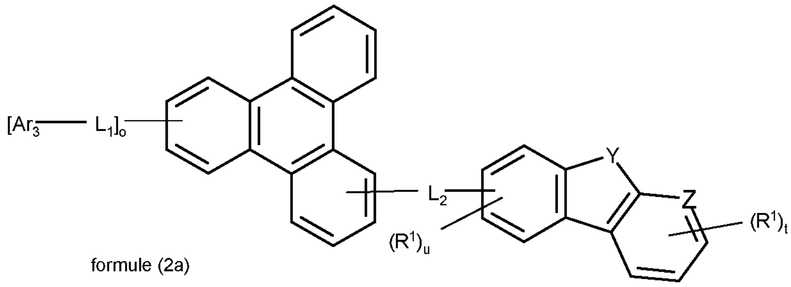

formule (2a)

,

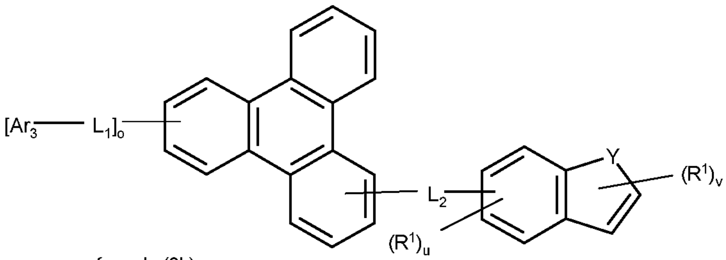

formule (2b)

,

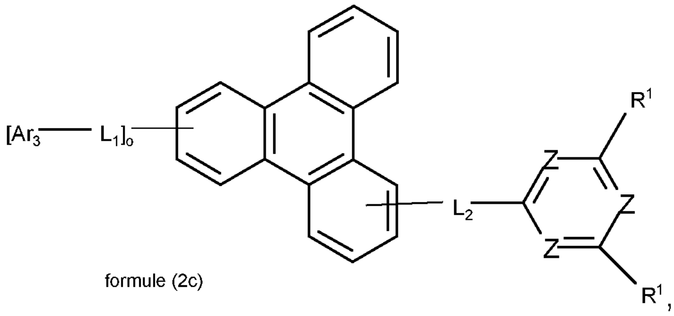

formule (2c)

où les symboles et indices utilisés revêtent une signification selon la revendication 1,

Z dans chaque cas, indépendamment les uns des autres à chaque occurrence, désigne N ou $CR^1$, où $R^1$ revêt une signification indiquée selon la revendication 1,
Y dans la formule (2a) ou (2b) désigne O ou S,
t et u dans chaque cas, indépendamment l'un de l'autre, désignent 0, 1, 2 ou 3,
v dans chaque cas, indépendamment les uns des autres, désigne 0, 1 ou 2.

**3.** Composition selon la revendication 1 ou 2, **caractérisée en ce que** la composition comprend au moins un autre composé choisi dans le groupe constitué par les matériaux d'injection de trous, les matériaux de transport de trous, les matériaux de blocage de trous, les matériaux à large bande interdite, les émetteurs fluorescents, les émetteurs phosphorescents, les matériaux hôtes, les matériaux de blocage d'électrons, les matériaux de transport d'électrons et les matériaux d'injection d'électrons, les dopants de type n et les dopants de type p.

**4.** Formulation comprenant une composition selon l'une ou plusieurs parmi les revendications 1 à 3 et au moins un solvant.

**5.** Utilisation d'une composition selon l'une ou plusieurs parmi les revendications 1 à 3, dans un dispositif électronique organique.

**6.** Utilisation selon la revendication 5, **caractérisée en ce que** le dispositif électronique organique est choisi dans le

groupe constitué par les circuits intégrés organiques (OIC), les transistors organiques à effet de champ (OFET), les transistors organiques à couche mince (OTFT), les dispositifs électroluminescents organiques, les cellules solaires organiques (OSC), les détecteurs optiques organiques et les photorécepteurs organiques.

**7.** Dispositif électronique organique contenant au moins une composition selon l'une ou plusieurs parmi les revendications 1 à 3.

**8.** Dispositif selon la revendication 7, **caractérisé en ce qu'**il est choisi dans le groupe constitué par les circuits intégrés organiques (OIC), les transistors organiques à effet de champ (OFET), les transistors organiques à couche mince (OTFT), les dispositifs électroluminescents organiques, les cellules solaires organiques (OSC), les détecteurs optiques organiques et les photorécepteurs organiques.

**9.** Dispositif selon la revendication 7 ou 8, **caractérisé en ce qu'**il s'agit d'un dispositif électroluminescent choisi parmi les transistors organiques émetteurs de lumière (OLET), les dispositifs organiques à extinction de champ (OFQD), les cellules électrochimiques organiques émettrices de lumière (OLEC, LEC, LEEC), les diodes laser organiques (O-lasers) et les diodes électroluminescentes organiques (OLED).

**10.** Dispositif selon l'une ou plusieurs parmi les revendications 7 à 9, **caractérisé en ce qu'**il contient la composition selon l'une ou plusieurs parmi les revendications 1 à 3 dans une couche d'émission (EML), dans une couche de transport d'électrons (ETL), dans une couche d'injection d'électrons (EIL) et/ou dans une couche de blocage de trous (HBL).

**11.** Dispositif selon l'une ou plusieurs parmi les revendications 7 à 10, **caractérisé en ce qu'**il contient la composition selon l'une ou plusieurs parmi les revendications 1 à 3 dans la couche d'émission conjointement avec un émetteur phosphorescent.

**12.** Procédé de production d'un dispositif selon l'une ou plusieurs parmi les revendications 7 à 11, **caractérisé en ce qu'**au moins une couche organique comprenant une composition selon l'une ou plusieurs parmi les revendications 1 à 3 est appliquée par dépôt en phase gazeuse ou à partir d'une solution.

**13.** Procédé selon la revendication 12, **caractérisé en ce qu'**au moins un composé de formule (1a) ou de formule (1b) et au moins un composé de formule (2), tels que décrits selon l'une des revendications 1 ou 2, sont déposés successivement ou simultanément à partir d'au moins deux sources de matériaux, éventuellement avec d'autres matériaux, à partir de la phase gazeuse et forment la couche organique.

**14.** Procédé selon la revendication 12, **caractérisé en ce que** la composition selon l'une ou plusieurs parmi les revendications 1 à 3 est utilisée comme source de matériau pour le dépôt en phase gazeuse et forme la couche organique.

**15.** Procédé selon la revendication 12, **caractérisé en ce que** la formulation selon la revendication 4 est utilisée pour appliquer la couche organique.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4539507 A
- US 5151629 A
- EP 0676461 A
- WO 9827136 A
- WO 2004093207 A
- WO 2010006680 A
- WO 2005003253 A
- WO 2008056746 A
- EP 0906947 A
- EP 0908787 A
- EP 0906948 A
- WO 2011116865 A
- WO 2011137951 A
- WO 2005039246 A
- US 20050069729 A
- WO 2014015931 A
- WO 2007063754 A
- WO 2010136109 A
- WO 2011000455 A
- WO 2011057706 A
- WO 2011046182 A
- WO 2009069442 A
- WO 2015014434 A
- WO 2015169412 A
- WO 2009021126 A
- US 20150340618 A
- US 6392250 B1
- US 6803720 B1
- KR 101744248
- EP 3336159 A
- WO 2015192941 A
- US 2015340618 A
- US 20060280965 A
- WO 2005086251 A2
- WO 2012175535 A1
- WO 2012175219 A1
- WO 2012168358 A1
- WO 2012031735 A1
- EP 1837926 A1
- WO 2007107306 A1
- EP 2452946 A1
- EP 2463927 A1
- WO 2009000237 A1
- US 2007145355 A1
- EP 1538684 A1
- WO 2006081780 A1
- WO 2009003455 A1
- WO 2010097433 A1
- EP 1988587 A1
- US 2010102709 A1
- EP 2180029 A1
- WO 2011131185 A1
- WO 2011134458 A1
- US 2012223296 A1
- WO 2007134873 A1
- WO 2008061517 A2
- WO 2008061518 A2
- DE 102008051737 A1
- WO 2009089821 A1
- US 2010096600 A1
- WO 2008138580 A1
- WO 2008058525 A2
- WO 2007115540 A1
- DE 102010046040 A1
- WO 2008128519 A2
- US 7294849 B
- WO 2016015815 A
- WO 0070655 A
- WO 200141512 A
- WO 200202714 A
- WO 200215645 A
- EP 1191613 A
- EP 1191612 A
- EP 1191614 A
- WO 05033244 A
- WO 05019373 A
- US 20050258742 A
- WO 2009146770 A
- WO 2010015307 A
- WO 2010031485 A
- WO 2010054731 A
- WO 2010054728 A
- WO 2010086089 A
- WO 2010099852 A
- WO 2010102709 A
- WO 2011032626 A
- WO 2011066898 A
- WO 2011157339 A
- WO 2012007086 A
- WO 2014008982 A
- WO 2014023377 A
- WO 2014094961 A
- WO 2014094960 A
- WO 2015036074 A
- WO 2015104045 A
- WO 2015117718 A
- WO 2016124304 A

- WO 2017032439 A
- WO 2006108497 A
- WO 2006122630 A
- WO 2008006449 A
- WO 2007140847 A
- WO 2010012328 A
- WO 2010108579 A
- EP 676461 A
- WO 2004081017 A
- WO 2004058911 A
- WO 2005084081 A
- WO 2005084082 A
- WO 2006048268 A
- WO 2006117052 A
- WO 2008145239 A
- JP 2004288381 A
- EP 1205527 A
- WO 2008086851 A
- WO 2011088877 A
- WO 2011128017 A
- EP 1617710 A
- EP 1617711 A
- EP 1731584 A
- JP 2005347160 A
- WO 2007137725 A
- WO 2005111172 A
- WO 2010015306 A
- EP 652273 A
- WO 2009062578 A
- WO 2010054729 A
- WO 2010054730 A
- WO 2005011013 A
- JP 2000053957 A
- WO 2003060956 A
- WO 2004028217 A
- WO 2004080975 A
- WO 2010072300 A
- WO 06122630 A
- WO 06100896 A
- EP 1661888 A
- WO 01049806 A
- US 5061569 A
- WO 9509147 A
- WO 08006449 A
- WO 07140847 A
- WO 2012034627 A
- EP 12000929
- WO 2014015937 A
- WO 2014015938 A
- WO 2014015935 A
- WO 2013083216 A
- WO 2012150001 A


**In der Beschreibung aufgeführte Nicht-Patentliteratur**


- **M. A. BALDO et al.** *Appl. Phys. Lett.,* 1999, vol. 75, 4-6
- *CHEMICAL ABSTRACTS,* 1269508-30-6
- *CHEMICAL ABSTRACTS,* 1989601-68-4
- *CHEMICAL ABSTRACTS,* 1989602-19-8
- *CHEMICAL ABSTRACTS,* 1989602-70-1
- *CHEMICAL ABSTRACTS,* 1215692-34-4
- *CHEMICAL ABSTRACTS,* 1989601-69-5
- *CHEMICAL ABSTRACTS,* 1989602-20-1
- *CHEMICAL ABSTRACTS,* 1989602-71-2
- *CHEMICAL ABSTRACTS,* 1370364-40-1
- *CHEMICAL ABSTRACTS,* 1989601-70-8
- *CHEMICAL ABSTRACTS,* 1989602-21-2
- *CHEMICAL ABSTRACTS,* 1989602-72-3
- *CHEMICAL ABSTRACTS,* 1370364-42-3
- *CHEMICAL ABSTRACTS,* 1989601-71-9
- *CHEMICAL ABSTRACTS,* 1989602-22-3
- *CHEMICAL ABSTRACTS,* 1989602-73-4
- *CHEMICAL ABSTRACTS,* 1989600-74-9
- *CHEMICAL ABSTRACTS,* 1989601-72-0
- *CHEMICAL ABSTRACTS,* 1989602-23-4
- *CHEMICAL ABSTRACTS,* 1989602-74-5
- *CHEMICAL ABSTRACTS,* 1989600-75-0
- *CHEMICAL ABSTRACTS,* 1989601-73-1
- *CHEMICAL ABSTRACTS,* 1989602-24-5
- *CHEMICAL ABSTRACTS,* 1989602-75-6
- *CHEMICAL ABSTRACTS,* 1989600-77-2
- *CHEMICAL ABSTRACTS,* 1989601-74-2
- *CHEMICAL ABSTRACTS,* 1989602-25-6
- *CHEMICAL ABSTRACTS,* 1989602-76-7
- *CHEMICAL ABSTRACTS,* 1989600-78-3
- *CHEMICAL ABSTRACTS,* 1989601-75-3
- *CHEMICAL ABSTRACTS,* 1989602-26-7
- *CHEMICAL ABSTRACTS,* 1989602-77-8
- *CHEMICAL ABSTRACTS,* 1989600-79-4
- *CHEMICAL ABSTRACTS,* 1989601-76-4
- *CHEMICAL ABSTRACTS,* 1989602-27-8
- *CHEMICAL ABSTRACTS,* 1989602-78-9
- *CHEMICAL ABSTRACTS,* 1989600-82-9
- *CHEMICAL ABSTRACTS,* 1989601-77-5
- *CHEMICAL ABSTRACTS,* 1989602-28-9
- *CHEMICAL ABSTRACTS,* 1989602-79-0
- *CHEMICAL ABSTRACTS,* 1989600-83-0
- *CHEMICAL ABSTRACTS,* 1989601-78-6
- *CHEMICAL ABSTRACTS,* 1989602-29-0
- *CHEMICAL ABSTRACTS,* 1989602-80-3
- *CHEMICAL ABSTRACTS,* 1989600-84-1
- *CHEMICAL ABSTRACTS,* 1989601-79-7
- *CHEMICAL ABSTRACTS,* 1989602-30-3
- *CHEMICAL ABSTRACTS,* 1989602-82-5
- *CHEMICAL ABSTRACTS,* 1989600-85-2
- *CHEMICAL ABSTRACTS,* 1989601-80-0
- *CHEMICAL ABSTRACTS,* 1989602-31-4
- *CHEMICAL ABSTRACTS,* 1989602-84-7
- *CHEMICAL ABSTRACTS,* 1989600-86-3
- *CHEMICAL ABSTRACTS,* 1989601-81-1

- *CHEMICAL ABSTRACTS,* 1989602-32-5
- *CHEMICAL ABSTRACTS,* 1989602-85-8
- *CHEMICAL ABSTRACTS,* 1989600-87-4
- *CHEMICAL ABSTRACTS,* 1989601-82-2
- *CHEMICAL ABSTRACTS,* 1989602-33-6
- *CHEMICAL ABSTRACTS,* 1989602-86-9
- *CHEMICAL ABSTRACTS,* 1989600-88-5
- *CHEMICAL ABSTRACTS,* 1989601-83-3
- *CHEMICAL ABSTRACTS,* 1989602-34-7
- *CHEMICAL ABSTRACTS,* 1989602-87-0
- *CHEMICAL ABSTRACTS,* 1989600-89-6
- *CHEMICAL ABSTRACTS,* 1989601-84-4
- *CHEMICAL ABSTRACTS,* 1989602-35-8
- *CHEMICAL ABSTRACTS,* 1989602-88-1
- *CHEMICAL ABSTRACTS,* 1989601-11-7
- *CHEMICAL ABSTRACTS,* 1989601-85-5
- *CHEMICAL ABSTRACTS,* 1989602-36-9
- *CHEMICAL ABSTRACTS,* 1989604-00-3
- *CHEMICAL ABSTRACTS,* 1989601-23-1
- *CHEMICAL ABSTRACTS,* 1989601-86-6
- *CHEMICAL ABSTRACTS,* 1989602-37-0
- *CHEMICAL ABSTRACTS,* 1989604-01-4
- *CHEMICAL ABSTRACTS,* 1989601-26-4
- *CHEMICAL ABSTRACTS,* 1989601-87-7
- *CHEMICAL ABSTRACTS,* 1989602-38-1
- *CHEMICAL ABSTRACTS,* 1989604-02-5
- *CHEMICAL ABSTRACTS,* 1989601-28-6
- *CHEMICAL ABSTRACTS,* 1989601-88-8
- *CHEMICAL ABSTRACTS,* 1989602-39-2
- *CHEMICAL ABSTRACTS,* 1989604-03-6
- *CHEMICAL ABSTRACTS,* 1989601-29-7
- *CHEMICAL ABSTRACTS,* 1989601-89-9
- *CHEMICAL ABSTRACTS,* 1989602-40-5
- *CHEMICAL ABSTRACTS,* 1989604-04-7
- *CHEMICAL ABSTRACTS,* 1989601-33-3
- *CHEMICAL ABSTRACTS,* 1989601-90-2
- *CHEMICAL ABSTRACTS,* 1989602-41-6
- *CHEMICAL ABSTRACTS,* 1989604-05-8
- *CHEMICAL ABSTRACTS,* 1989601-40-2
- *CHEMICAL ABSTRACTS,* 1989601-91-3
- *CHEMICAL ABSTRACTS,* 1989602-42-7
- *CHEMICAL ABSTRACTS,* 1989604-06-9
- *CHEMICAL ABSTRACTS,* 1989601-41-3
- *CHEMICAL ABSTRACTS,* 1989601-92-4
- *CHEMICAL ABSTRACTS,* 1989602-43-8
- *CHEMICAL ABSTRACTS,* 1989604-07-0
- *CHEMICAL ABSTRACTS,* 1989601-42-4
- *CHEMICAL ABSTRACTS,* 1989601-93-5
- *CHEMICAL ABSTRACTS,* 1989602-44-9
- *CHEMICAL ABSTRACTS,* 1989604-08-1
- *CHEMICAL ABSTRACTS,* 1989601-43-5
- *CHEMICAL ABSTRACTS,* 1989601-94-6
- *CHEMICAL ABSTRACTS,* 1989602-45-0
- *CHEMICAL ABSTRACTS,* 1989604-09-2
- *CHEMICAL ABSTRACTS,* 1989601-44-6
- *CHEMICAL ABSTRACTS,* 1989601-95-7
- *CHEMICAL ABSTRACTS,* 1989602-46-1
- *CHEMICAL ABSTRACTS,* 1989604-10-5
- *CHEMICAL ABSTRACTS,* 1989601-45-7
- *CHEMICAL ABSTRACTS,* 1989601-96-8
- *CHEMICAL ABSTRACTS,* 1989602-47-2
- *CHEMICAL ABSTRACTS,* 1989604-11-6
- *CHEMICAL ABSTRACTS,* 1989601-46-8
- *CHEMICAL ABSTRACTS,* 1989601-97-9
- *CHEMICAL ABSTRACTS,* 1989602-48-3
- *CHEMICAL ABSTRACTS,* 1989604-13-8
- *CHEMICAL ABSTRACTS,* 1989601-47-9
- *CHEMICAL ABSTRACTS,* 1989601-98-0
- *CHEMICAL ABSTRACTS,* 1989602-49-4
- *CHEMICAL ABSTRACTS,* 1989604-14-9
- *CHEMICAL ABSTRACTS,* 1989601-48-0
- *CHEMICAL ABSTRACTS,* 1989601-99-1
- *CHEMICAL ABSTRACTS,* 1989602-50-7
- *CHEMICAL ABSTRACTS,* 1989604-15-0
- *CHEMICAL ABSTRACTS,* 1989601-49-1
- *CHEMICAL ABSTRACTS,* 1989602-00-7
- *CHEMICAL ABSTRACTS,* 1989602-51-8
- *CHEMICAL ABSTRACTS,* 1989604-16-1
- *CHEMICAL ABSTRACTS,* 1989601-50-4
- *CHEMICAL ABSTRACTS,* 1989602-01-8
- *CHEMICAL ABSTRACTS,* 1989602-52-9
- *CHEMICAL ABSTRACTS,* 1989604-17-2
- *CHEMICAL ABSTRACTS,* 1989601-51-5
- *CHEMICAL ABSTRACTS,* 1989602-02-9
- *CHEMICAL ABSTRACTS,* 1989602-53-0
- *CHEMICAL ABSTRACTS,* 1989604-18-3
- *CHEMICAL ABSTRACTS,* 1989601-52-6
- *CHEMICAL ABSTRACTS,* 1989602-03-0
- *CHEMICAL ABSTRACTS,* 1989602-54-1
- *CHEMICAL ABSTRACTS,* 1989604-19-4
- *CHEMICAL ABSTRACTS,* 1989601-53-7
- *CHEMICAL ABSTRACTS,* 1989602-04-1
- *CHEMICAL ABSTRACTS,* 1989602-55-2
- *CHEMICAL ABSTRACTS,* 1989604-20-7
- *CHEMICAL ABSTRACTS,* 1989601-54-8
- *CHEMICAL ABSTRACTS,* 1989602-05-2
- *CHEMICAL ABSTRACTS,* 1989602-56-3
- *CHEMICAL ABSTRACTS,* 1989604-21-8
- *CHEMICAL ABSTRACTS,* 1989601-55-9
- *CHEMICAL ABSTRACTS,* 1989602-06-3
- *CHEMICAL ABSTRACTS,* 1989602-57-4
- *CHEMICAL ABSTRACTS,* 1989604-22-9
- *CHEMICAL ABSTRACTS,* 1989601-56-0
- *CHEMICAL ABSTRACTS,* 1989602-07-4
- *CHEMICAL ABSTRACTS,* 1989602-58-5
- *CHEMICAL ABSTRACTS,* 1989604-23-0
- *CHEMICAL ABSTRACTS,* 1989601-57-1
- *CHEMICAL ABSTRACTS,* 1989602-08-5
- *CHEMICAL ABSTRACTS,* 1989602-59-6
- *CHEMICAL ABSTRACTS,* 1989604-24-1
- *CHEMICAL ABSTRACTS,* 1989601-58-2
- *CHEMICAL ABSTRACTS,* 1989602-09-6
- *CHEMICAL ABSTRACTS,* 1989602-60-9
- *CHEMICAL ABSTRACTS,* 1989604-25-2
- *CHEMICAL ABSTRACTS,* 1989601-59-3
- *CHEMICAL ABSTRACTS,* 1989602-10-9

- *CHEMICAL ABSTRACTS,* 1989602-61-0
- *CHEMICAL ABSTRACTS,* 1989604-26-3
- *CHEMICAL ABSTRACTS,* 1989601-60-6
- *CHEMICAL ABSTRACTS,* 1989602-11-0
- *CHEMICAL ABSTRACTS,* 1989602-62-1
- *CHEMICAL ABSTRACTS,* 1989604-27-4
- *CHEMICAL ABSTRACTS,* 1989601-61-7
- *CHEMICAL ABSTRACTS,* 1989602-12-1
- *CHEMICAL ABSTRACTS,* 1989602-63-2
- *CHEMICAL ABSTRACTS,* 1989604-28-5
- *CHEMICAL ABSTRACTS,* 1989601-62-8
- *CHEMICAL ABSTRACTS,* 1989602-13-2
- *CHEMICAL ABSTRACTS,* 1989602-64-3
- *CHEMICAL ABSTRACTS,* 1989604-29-6
- *CHEMICAL ABSTRACTS,* 1989601-63-9
- *CHEMICAL ABSTRACTS,* 1989602-14-3
- *CHEMICAL ABSTRACTS,* 1989602-65-4
- *CHEMICAL ABSTRACTS,* 1989604-30-9
- *CHEMICAL ABSTRACTS,* 1989601-64-0
- *CHEMICAL ABSTRACTS,* 1989602-15-4
- *CHEMICAL ABSTRACTS,* 1989602-66-5
- *CHEMICAL ABSTRACTS,* 1989604-31-0
- *CHEMICAL ABSTRACTS,* 1989601-65-1
- *CHEMICAL ABSTRACTS,* 1989602-16-5
- *CHEMICAL ABSTRACTS,* 1989602-67-6
- *CHEMICAL ABSTRACTS,* 1989604-32-1
- *CHEMICAL ABSTRACTS,* 1989601-66-2
- *CHEMICAL ABSTRACTS,* 1989602-17-6
- *CHEMICAL ABSTRACTS,* 1989602-68-7
- *CHEMICAL ABSTRACTS,* 1989604-33-2
- *CHEMICAL ABSTRACTS,* 1989601-67-3
- *CHEMICAL ABSTRACTS,* 1989602-18-7
- *CHEMICAL ABSTRACTS,* 1989602-69-8
- *CHEMICAL ABSTRACTS,* 1989604-34-3
- *CHEMICAL ABSTRACTS,* 1989604-35-4
- *CHEMICAL ABSTRACTS,* 1989604-88-7
- *CHEMICAL ABSTRACTS,* 1989605-52-8
- *CHEMICAL ABSTRACTS,* 1989606-07-6
- *CHEMICAL ABSTRACTS,* 1989604-36-5
- *CHEMICAL ABSTRACTS,* 1989604-89-8
- *CHEMICAL ABSTRACTS,* 1989605-53-9
- *CHEMICAL ABSTRACTS,* 1989606-08-7
- *CHEMICAL ABSTRACTS,* 1989604-37-6
- *CHEMICAL ABSTRACTS,* 1989604-90-1
- *CHEMICAL ABSTRACTS,* 1989605-54-0
- *CHEMICAL ABSTRACTS,* 1989606-09-8
- *CHEMICAL ABSTRACTS,* 1989604-38-7
- *CHEMICAL ABSTRACTS,* 1989604-92-3
- *CHEMICAL ABSTRACTS,* 1989605-55-1
- *CHEMICAL ABSTRACTS,* 1989606-10-1
- *CHEMICAL ABSTRACTS,* 1989604-39-8
- *CHEMICAL ABSTRACTS,* 1989604-93-4
- *CHEMICAL ABSTRACTS,* 1989605-56-2
- *CHEMICAL ABSTRACTS,* 1989606-11-2
- *CHEMICAL ABSTRACTS,* 1989604-40-1
- *CHEMICAL ABSTRACTS,* 1989604-94-5
- *CHEMICAL ABSTRACTS,* 1989605-57-3
- *CHEMICAL ABSTRACTS,* 1989606-12-3
- *CHEMICAL ABSTRACTS,* 1989604-41-2
- *CHEMICAL ABSTRACTS,* 1989604-95-6
- *CHEMICAL ABSTRACTS,* 1989605-58-4
- *CHEMICAL ABSTRACTS,* 1989606-13-4
- *CHEMICAL ABSTRACTS,* 1989604-42-3
- *CHEMICAL ABSTRACTS,* 1989604-96-7
- *CHEMICAL ABSTRACTS,* 1989605-59-5
- *CHEMICAL ABSTRACTS,* 1989606-14-5
- *CHEMICAL ABSTRACTS,* 1989604-43-4
- *CHEMICAL ABSTRACTS,* 1989604-97-8
- *CHEMICAL ABSTRACTS,* 1989605-61-9
- *CHEMICAL ABSTRACTS,* 1989606-15-6
- *CHEMICAL ABSTRACTS,* 1989604-45-6
- *CHEMICAL ABSTRACTS,* 1989605-09-5
- *CHEMICAL ABSTRACTS,* 1989605-62-0
- *CHEMICAL ABSTRACTS,* 1989606-16-7
- *CHEMICAL ABSTRACTS,* 1989604-46-7
- *CHEMICAL ABSTRACTS,* 1989605-10-8
- *CHEMICAL ABSTRACTS,* 1989605-63-1
- *CHEMICAL ABSTRACTS,* 1989606-17-8
- *CHEMICAL ABSTRACTS,* 1989604-47-8
- *CHEMICAL ABSTRACTS,* 1989605-11-9
- *CHEMICAL ABSTRACTS,* 1989605-64-2
- *CHEMICAL ABSTRACTS,* 1989606-18-9
- *CHEMICAL ABSTRACTS,* 1989604-48-9
- *CHEMICAL ABSTRACTS,* 1989605-13-1
- *CHEMICAL ABSTRACTS,* 1989605-65-3
- *CHEMICAL ABSTRACTS,* 1989606-19-0
- *CHEMICAL ABSTRACTS,* 1989604-49-0
- *CHEMICAL ABSTRACTS,* 1989605-14-2
- *CHEMICAL ABSTRACTS,* 1989605-66-4
- *CHEMICAL ABSTRACTS,* 1989606-20-3
- *CHEMICAL ABSTRACTS,* 1989604-50-3
- *CHEMICAL ABSTRACTS,* 1989605-15-3
- *CHEMICAL ABSTRACTS,* 1989605-67-5
- *CHEMICAL ABSTRACTS,* 1989606-21-4
- *CHEMICAL ABSTRACTS,* 1989604-52-5
- *CHEMICAL ABSTRACTS,* 1989605-16-4
- *CHEMICAL ABSTRACTS,* 1989605-68-6
- *CHEMICAL ABSTRACTS,* 1989606-22-5
- *CHEMICAL ABSTRACTS,* 1989604-53-6
- *CHEMICAL ABSTRACTS,* 1989605-17-5
- *CHEMICAL ABSTRACTS,* 1989605-69-7
- *CHEMICAL ABSTRACTS,* 1989606-23-6
- *CHEMICAL ABSTRACTS,* 1989604-54-7
- *CHEMICAL ABSTRACTS,* 1989605-18-6
- *CHEMICAL ABSTRACTS,* 1989605-70-0
- *CHEMICAL ABSTRACTS,* 1989606-24-7
- *CHEMICAL ABSTRACTS,* 1989604-55-8
- *CHEMICAL ABSTRACTS,* 1989605-19-7
- *CHEMICAL ABSTRACTS,* 1989605-71-1
- **T. MATSUMOTO ; T. NAKADA ; J. ENDO ; K. MORI ; N. KAWAMURA ; A. YOKOI ; J. KIDO.** *Multiphoton Organic EL Device Having Charge Generation Layer*
- **Y. SHIROTA et al.** *Chem. Rev.,* 2007, vol. 107 (4), 953-1010

- **B. M. S. ARNOLD et al.** *Appl. Phys. Lett.,* 2008, vol. 92, 053301
- *CHEMICAL ABSTRACTS,* 1160294-85-8